(19) Europäisches Patentamt
European Patent Office
Office européen des brevets

(11) Veröffentlichungsnummer: **0 355 385 B1**

(12) # EUROPÄISCHE PATENTSCHRIFT

(45) Veröffentlichungstag der Patentschrift: **19.05.93**

(51) Int. Cl.⁵: **C07D 401/04**, C07D 401/14, C07D 403/04, C07D 403/14, C07D 409/14, C07D 417/14, A01N 43/653

(21) Anmeldenummer: **89113013.0**

(22) Anmeldetag: **15.07.89**

(54) **Substituierte 4-Sulfonylamino-2-azinyl-1,2,4-triazol-3-one, Verfahren sowie Zwischenprodukte zu ihrer Herstellung und ihre Verwendung als Herbizide.**

(30) Priorität: **28.07.88 DE 3825602**
**16.01.89 DE 3910184**

(43) Veröffentlichungstag der Anmeldung:
**28.02.90 Patentblatt 90/09**

(45) Bekanntmachung des Hinweises auf die Patenterteilung:
**19.05.93 Patentblatt 93/20**

(84) Benannte Vertragsstaaten:
**BE CH DE ES FR GB IT LI NL**

(56) Entgegenhaltungen:
**US-A- 3 884 910**

(73) Patentinhaber: **BAYER AG**

**W-5090 Leverkusen 1 Bayerwerk(DE)**

(72) Erfinder: **Müller, Klaus-Helmut, Dr.**
**Bockhackstrasse 55**
**W-4000 Düsseldorf 13(DE)**
Erfinder: **Kirsten, Rolf, Dr.**
**Carl-Langhans-Strasse 27**
**W-4019 Monheim(DE)**
Erfinder: **Kluth, Joachim, Dr.**
**Tannenweg 9**
**W-4018 Langenfeld(DE)**
Erfinder: **König, Klaus, Dr.**
**Zum Hahnberg 40**
**W-5068 Odenthal(DE)**
Erfinder: **Riebel, Hans-Jochem, Dr.**
**In der Beek 92**
**W-5600 Wuppertal 1(DE)**
Erfinder: **Babczinski, Peter, Dr.**
**In der Lohrenbeck 11**
**W-5600 Wuppertal 1(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**
Erfinder: **Strang, Harry, Dr.**
**Unterdorfstrasse 6A**
**W-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neuartige substituierte 4-Sulfonylamino-2-azinyl-1,2,4-triazol-3-one (nachfolgend kurz bezeichnet als "substituierte Sulfonylaminotriazolinone"). Verfahren und neue Zwischenprodukte zu ihrer Herstellung und ihre Verwendung als Herbizide.

Es ist bekannt, daß bestimmte substituierte Triazolinone, wie z.B. 4-(3-Trifluormethoxybenzylidenamino)-2,4-dihydro-3H-1,2,4-triazol-3-on, herbizid wirksam sind (vgl. US-PS 3 884 910). Die Wirkung dieser bekannten Verbindungen ist jedoch nicht in allen Belangen zufriedenstellend.

Es wurden nun die neuen substituierten Sulfonylaminotriazolinone der allgemeinen Formel (I)

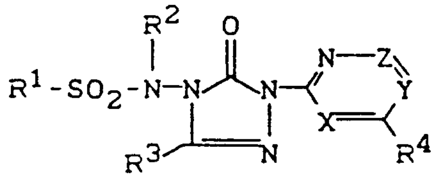

(I)

sowie Salze von Verbindungen der Formel (I) gefunden, in welcher

$R^1$ für den Rest

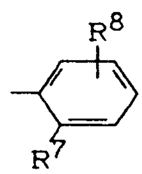

steht, worin

$R^7$ und $R^8$ gleich oder verschieden sind und für Wasserstoff, Fluor, Chlor, Brom, Iod, Cyano, Nitro, $C_1-C_6$-Alkyl (welches gegebenenfalls durch Fluor, Chlor, Brom, Cyano, Carboxy, $C_1-C_4$-Alkoxycarbonyl, $C_1-C_4$-Alkylamino-carbonyl, Di-($C_1-C_4$-alkyl)amino-carbonyl, Hydroxy, $C_1-C_4$-Alkoxy, Formyloxy, $C_1-C_4$-Alkyl-carbonyloxy, $C_1-C_4$-Alkoxy-carbonyloxy, $C_1-C_4$-Alkylamino-carbonyloxy, $C_1-C_4$-Alkylthio, $C_1-C_4$-Alkylsulfinyl, $C_1-C_4$-Alkylsulfonyl, Di-($C_1-C_4$-alkyl)-aminosulfonyl, $C_3-C_6$-Cycloalkyl oder Phenyl substituiert ist], für $C_2-C_6$-Alkenyl [welches gegebenenfalls durch Fluor, Chlor, Brom, Cyano, $C_1-C_4$-Alkoxy-carbonyl, Carboxy oder Phenyl substituiert ist], für $C_2-C_6$-Alkinyl [welches gegebenenfalls durch Fluor, Chlor, Brom, Cyano, $C_1-C_4$-Alkoxy-carbonyl, Carboxy oder Phenyl substituiert ist], für $C_1-C_4$-Alkoxy [welches gegebenenfalls durch Fluor, Chlor, Brom, Cyano, Carboxy, $C_1-C_4$-Alkoxy-carbonyl, $C_1-C_4$-Alkoxy, $C_1-C_4$-Alkylthio, $C_1-C_4$-Alkylsulfinyl oder $C_1-C_4$-Alkylsulfonyl substituiert ist], für $C_1-C_4$-Alkylthio [welches gegebenenfalls durch Fluor, Chlor, Brom, Cyano, Carboxy, $C_1-C_4$-Alkoxy-carbonyl, $C_1-C_4$-Alkylthio, $C_1-C_4$-Alkylsulfinyl oder $C_1-C_4$-Alkylsulfonyl substituiert ist], für $C_3-C_6$-Alkenyloxy [welches gegebenenfalls durch Fluor, Chlor, Brom, Cyano oder oder $C_1-C_4$-Alkoxy-carbonyl substituiert ist], für $C_2-C_6$-Alkenylthio [welches gegebenenfalls durch Fluor, Chlor, Brom, Cyano, Nitro, $C_1-C_3$-Alkylthio oder $C_1-C_4$-Alkoxy-carbonyl substituiert ist], $C_3-C_6$-Alkinyloxy, $C_3-C_6$-Alkinylthio oder für den Rest $S(O)_p-R^9$ stehen, wobei

p für die Zahlen 1 oder 2 steht und

$R^9$ für Fluor, $C_1-C_4$-Alkyl [welches gegebenenfalls durch Fluor, Chlor, Brom, Cyano oder $C_1-C_4$-Alkoxy-carbonyl substituiert ist], $C_3-C_6$-Alkenyl, $C_3-C_6$-Alkinyl, $C_1-C_4$-Alkoxy, $C_1-C_4$-Alkoxy-$C_1-C_4$-alkylamino, $C_1-C_4$-Alkylamino, Di-($C_1-C_4$-alkyl)-amino oder für den Rest $-NHOR^{10}$ steht, wobei

$R^{10}$ für $C_1-C_6$-Alkyl [welches gegebenenfalls durch Fluor, Chlor, Cyano, $C_1-C_4$-Alkoxy, $C_1-C_4$-Alkylthio, $C_1-C_4$-Alkylsulfinyl, $C_1-C_4$-Alkylsulfonyl, $C_1-C_4$-Alkyl-carbonyl, $C_1-C_4$-Alkoxy-carbonyl, $C_1-C_4$-Alkylamino-carbonyl oder Di-($C_1-C_4$-alkyl)-amino-carbonyl substituiert ist], für $C_3-C_6$-Alkenyl [welches gegebenenfalls durch Fluor, Chlor oder Brom substituiert ist], $C_3-C_6$-Alkinyl, $C_3-$

$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^7$ und/oder $R^8$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest -CO-$R^{11}$ stehen, wobei

$R^{11}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^7$ und/oder $R^8$ weiterhin für $C_1$-$C_4$-Alkylsulfonyloxy, Di-($C_1$-$C_4$-alkyl)-aminosulfonylamino oder für den Rest -CH=N-$R^{12}$ stehen, wobei

$R^{12}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

$R^1$ für den Rest

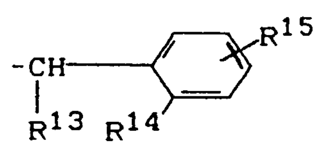

steht, worin

$R^{13}$ für Wasserstoff oder $C_1$-$C_4$-Alkyl steht,

$R^{14}$ und $R^{15}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter

$R^1$ für den Rest

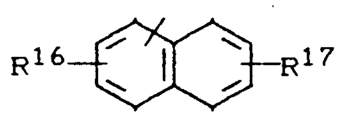

steht, worin

$R^{16}$ und $R^{17}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; worin weiter

$R^1$ für den Rest

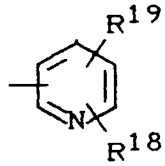

steht, worin

$R^{18}$ und $R^{19}$ gleich oder verschieden sind und für Wasserstoff, Fluor, Chlor, Brom, Nitro, Cyano, $C_1-C_4$-Alkyl [welches gegebenenfalls durch Fluor und/oder Chlor substituiert ist], $C_2-C_4$-Alkenyl [welches gegebenenfalls durch Fluor und/oder Chlor substituiert ist], $C_1-C_4$-Alkoxy [welches gegebenenfalls durch Fluor und/ oder Chlor substituiert ist], für $C_1$ $C_4$-Alkylthio, $C_1-C_4$-Alkylsulfinyl oder $C_1-C_4$-Alkylsulfonyl [welche gegebenenfalls durch Fluor und/oder Chlor substituiert sind], sowie für Di-($C_1-C_4$-alkyl)-aminosulfonyl, $C_1-C_4$-Alkoxy-carbonyl, Dimethylaminocarbonyl oder Dioxolanyl stehen; worin weiter

$R^1$ für den Rest

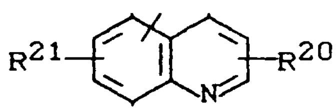

steht, worin

$R^{20}$ und $R^{21}$ gleich oder verschieden sind und für Wasserstoff, Fluor, Chlor, Brom, $C_1-C_4$-Alkyl [welches gegebenenfalls durch Fluor und/oder Brom substituiert ist], $C_1-C_4$-Alkoxy [welches gegebenenfalls durch Fluor und/oder Chlor substituiert ist], für $C_1-C_4$-Alkylthio, $C_1-C_4$-Alkylsulfinyl oder $C_1-C_4$-Alkylsulfonyl [welche gegebenenfalls durch Fluor und/oder Chlor substituiert sind], oder für Di-($C_1-C_4$-alkyl)-aminosulfonyl stehen; worin weiter

$R^1$ für den Rest

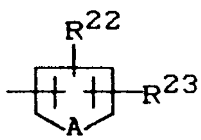

steht, worin

$R^{22}$ und $R^{23}$ gleich oder verschieden sind und für Wasserstoff, Fluor, Chlor, Brom, Cyano, Nitro, $C_1-C_4$-Alkyl [welches gegebenenfalls durch Fluor, Chlor, $C_1-C_4$-Alkoxy und/oder $C_1-C_4$-Halogenalkoxy substituiert ist], $C_1-C_4$-Alkoxy [welches gegebenenfalls durch Fluor und/oder Chlor substituiert ist], $C_1-C_4$-Alkylthio, $C_1-C_4$-Alkylsulfinyl oder $C_1-C_4$-Alkylsulfonyl [welches gegebenenfalls durch Fluor und/oder Chlor substituiert ist], Di-($C_1-C_4$-alkyl)-amino-sulfonyl oder $C_1-C_4$-Alkoxy-carbonyl stehen, und

A für Sauerstoff, Schwefel oder die Gruppierung $N-Z^1$ steht, wobei

$Z^1$ für Wasserstoff, $C_1-C_4$-Alkyl [welches gegebenenfalls durch Fluor, Chlor, Brom oder Cyano substituiert ist], $C_3-C_6$-Cycloalkyl, Benzyl, Phenyl [welches gegebenenfalls durch Fluor, Chlor, Brom oder Nitro substituiert ist], $C_1-C_4$-Alkylcarbonyl, $C_1-C_4$-Alkoxy-carbonyl oder Di-($C_1-C_4$-alkyl)-aminocarbonyl steht; worin weiter

$R^1$ für den Rest

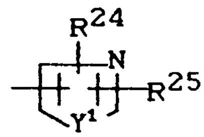

| | |
|---|---|
| | steht, worin |
| $R^{24}$ und $R^{25}$ | gleich oder verschieden sind und für Wasserstoff, $C_1-C_4$-Alkyl, Halogen, $C_1-C_4$-Alkoxycarbonyl, $C_1-C_4$-Alkoxy oder $C_1-C_4$-Halogenalkoxy stehen, |
| $Y^1$ | für Schwefel oder die Gruppierung $N-R^{26}$ steht, wobei |
| $R^{26}$ | für Wasserstoff oder $C_1-C_4$-Alkyl steht; worin weiter |
| $R^1$ | für den Rest |

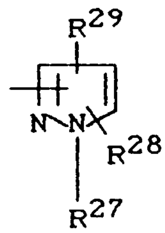

| | |
|---|---|
| | steht, worin |
| $R^{27}$ | für Wasserstoff, $C_1-C_4$-Alkyl, Benzyl, (Iso)Chinolinyl oder Phenyl steht, |
| $R^{28}$ | für Wasserstoff, Halogen, Cyano, Nitro, $C_1-C_4$-Alkyl [welches gegebenenfalls durch Fluor und/oder Chlor substituiert ist], $C_1-C_4$-Alkoxy [welches gegebenenfalls durch Fluor und/oder Chlor substituiert ist], Dioxolanyl oder $C_1-C_4$-Alkoxy-carbonyl steht und |
| $R^{29}$ | für Wasserstoff, Halogen oder $C_1-C_4$-Alkyl steht; worin weiter |
| $R^1$ | für den Rest |

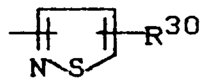

| | |
|---|---|
| | steht, worin |
| $R^{30}$ | für Wasserstoff, Halogen, $C_1-C_4$-Alkyl, $C_1-C_4$-Halogenalkyl, $C_1-C_4$-Alkoxy, $C_1-C_4$-Halogenalkoxy oder $C_1-C_4$-Alkoxy-carbonyl steht; worin weiter |
| $R^1$ | für den Rest |

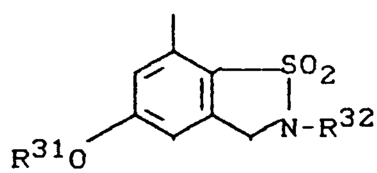

| | |
|---|---|
| | steht, worin |
| $R^{31}$ | für $C_1-C_4$-Alkyl steht und |
| $R^{32}$ | für $C_1-C_4$-Alkyl steht; worin weiter |
| $R^1$ | für den Rest |

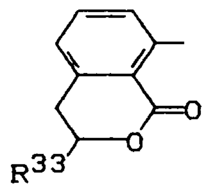

steht, worin

$R^{33}$ für Wasserstoff oder Methyl steht

in welcher weiter

$R^2$ für Wasserstoff oder die Gruppierung $-SO_2-R^1$ steht, worin

$R^1$ die oben vorzugsweise angegebene Bedeutung hat;

in welcher weiter

$R^3$ für Wasserstoff, Fluor, Chlor, Brom, Iod, Hydroxy, Mercapto, Amino oder einen gegebenenfalls durch Fluor und/oder Chlor substituierten Rest aus der Reihe $C_1-C_4$-Alkyl, $C_3-C_6$-Cycloalkyl, Benzyl, Phenyl, $C_1-C_4$-Alkoxy, $C_3-C_4$-Alkenyloxy, $C_3-C_4$-Alkinyloxy, $C_1-C_4$-Alkylthio, $C_1-C_4$-Alkylsulfinyl, $C_1-C_4$-Alkylsulfonyl, $C_3-C_4$-Alkenylthio, $C_3-C_4$-Alkinylthio, Benzyloxy, Benzylthio, $C_1-C_4$-Alkylamino und Di-($C_1-C_4$-alkyl)-amino steht,

$R^4$ für Wasserstoff, Fluor, Chlor, Brom, Hydroxy, Amino, $C_1-C_4$-Alkyl, $C_1-C_4$-Halogenalkyl, $C_1-C_2$-Alkoxy-$C_1-C_2$-alkyl, $C_1-C_4$-Alkoxy, $C_1-C_4$-Halogenalkoxy, $C_1-C_2$-Alkoxy-$C_1-C_2$-alkoxy, $C_1-C_4$-Alkylthio, $C_1-C_4$-Halogenalkylthio, $C_1-C_4$-Alkylsulfinyl, $C_1-C_4$-Alkylsulfonyl, $C_1-C_4$-Alkylamino oder Di-($C_1-C_2$-alkyl)-amino steht,

X für Stickstoff oder eine CH-Gruppierung steht,

Y für Stickstoff oder eine $CR^5$-Gruppierung steht, worin

$R^5$ für Wasserstoff, Fluor, Chlor, Brom, Cyano, Methyl, Formyl, Acetyl, Methoxycarbonyl oder Ethoxycarbonyl steht und

Z für Stickstoff oder eine $CR^6$-Gruppierung steht, worin

$R^6$ für Wasserstoff, Fluor, Chlor, Brom, $C_1-C_4$-Alkyl, $C_1-C_4$-Alkoxy, $C_1-C_4$-Halogenalkoxy, $C_1-C_4$-Alkylthio, $C_1-C_4$-Alkylsulfinyl, $C_1-C_4$-Alkylsulfonyl, $C_1-C_4$-Alkylamino, Dimethylamino oder Diethylamino steht.

Man erhält die neuen substituierten Sulfonylaminotriazolinone der allgemeinen Formel (I), wenn man

(a) substituierte Aminotriazolinone der allgemeinen Formel (II)

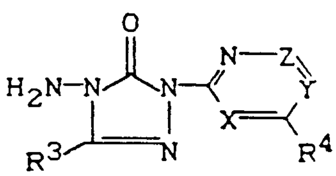

(II)

in welcher

$R^3$, $R^4$, X, Y und Z die oben angegebene Bedeutung haben,

mit Sulfonsäurehalogeniden oder Sulfonsäureanhydriden der allgemeinen Formel (III)

$R^1-SO_2-Q$ (III)

in welcher

$R^1$ die oben angegebene Bedeutung hat und

Q für Fluor, Chlor, Brom oder die Gruppierung $-O-SO_2-R^1$ steht, worin

$R^1$ die oben angegebene Bedeutung hat,

gegebenenfalls in Gegenwart eines Säureakzeptors und gegebenenfalls in Gegenwart eines Verdünnungsmittels umsetzt,

gegebenenfalls die so erhältlichen Verbindungen der Formel (I), in welcher

$R^2$ für die Gruppierung $-SO_2-R^1$ steht,

mit Desulfonylierungsmitteln,

gegebenenfalls in Gegenwart von Verdünnungsmitteln, zu Verbindungen der Formel (I), in welcher

$R^2$ für Wasserstoff steht,
umsetzt
und gegebenenfalls daraus nach üblichen Methoden Salze erzeugt; oder wenn man
(b) Sulfonylaminotriazolinone der allgemeinen Formel (IV)

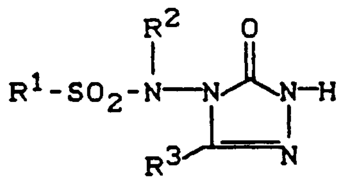

(IV)

in welcher
$R^1$, $R^2$ und $R^3$ die oben angegebenen Bedeutungen haben,
mit Azinen der allgemeinen Formel (V)

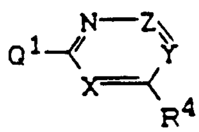

(V)

in welcher
$R^4$, X, Y und Z die oben angegebenen Bedeutungen haben und
$Q^1$ für Halogen, Benzylsulfonyl oder Alkylsulfonyl steht,
gegebenenfalls in Gegenwart eines Säureakzeptors und gegebenenfalls in Gegenwart eines Verdünnungsmittels umsetzt und gegebenenfalls aus den so erhaltenen Verbindungen der Formel (I) nach üblichen Methoden Salze erzeugt.

Die neuen substituierten Sulfonylaminotriazolinone der allgemeinen Formel (I) zeichnen sich durch starke herbizide Wirksamkeit aus. Diese Verbindungen stellen eine chemisch neuartige Klasse von Herbiziden dar. Überraschenderweise zeigen die neuen Verbindungen der Formel (I) erheblich bessere herbizide Wirkung als das strukturell ähnliche bekannte 4-(3-Trifluormethoxy-benzylidenamino)-2,4-dihydro-3H-1,2,4-triazol-3-on.

Gegenstand der Erfindung sind vorzugsweise Verbindungen der Formel (I), in welcher
$R^1$ für den Rest

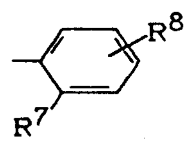

steht worin
$R^7$ für Fluor, Chlor, Brom, Methyl, Trifluormethyl, Methoxy, Difluormethoxy, Trifluormethoxy, $C_1-C_3$-Alkylthio, $C_1-C_3$-Alkylsulfinyl, $C_1-C_3$-Alkylsulfonyl, Dimethylaminosulfonyl, N-Methoxy-N-methylaminosulfonyl, Phenyl, Phenoxy oder $C_1-C_3$-Alkoxy-carbonyl steht und
$R^8$ für Wasserstoff, Fluor oder Chlor steht; worin weiter
$R^1$ für den Rest

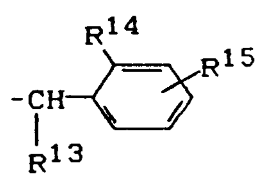

steht, worin

$R^{13}$ für Wasserstoff steht,

$R^{14}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{15}$ für Wasserstoff steht; worin weiter

$R^1$ für den Rest

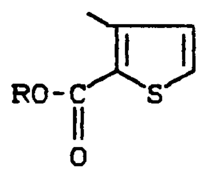

steht, worin

R für $C_1-C_4$-Alkyl steht, oder

$R^1$ für den Rest

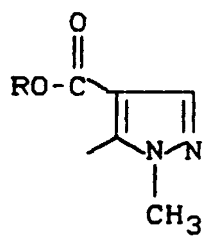

steht, worin

R für $C_1-C_4$-Alkyl steht, oder

$R^1$ für den Rest

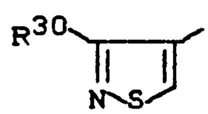

steht, worin

$R^{30}$ für Wasserstoff, Chlor, Methyl, Ethyl, Methoxycarbonyl oder Ethoxycarbonyl steht;

in welcher weiter

$R^2$ für Wasserstoff oder für die Gruppierung $-SO_2-R^1$ steht, worin

$R^1$ die oben als insbesondere bevorzugt angegebene Bedeutung hat,

$R^3$ für Wasserstoff, Methyl, Ethyl, Propyl, Isopropyl, Trifluormethyl, Cyclopropyl, Benzyl, Phenyl, t-Butyl, s-Butyl, i-Butyl, n-Butyl, Methoxy oder Methylthio steht,

$R^4$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine CH-Gruppierung steht,

Y für Stickstoff oder eine $CR^5$-Gruppierung steht, worin

$R^5$ für Wasserstoff, Fluor, Chlor oder Methyl steht, und

Z für Stickstoff oder eine $CR^6$-Gruppierung steht, worin

$R^6$ für Wasserstoff, Fluor, Chlor, Brom, Methyl, Ethyl, Methoxy, Ethoxy, Propoxy, Isopropoxy, Difluormethoxy, Methylthio, Ethylthio, Methylamino, Ethylamino, Dimethylamino oder Diethylamino steht.

Die Erfindung betrifft weiter vorzugsweise Salze von Verbindungen der Formel (I)

α) mit Protonensäuren, wie z. B. Salzsäure, Schwefelsäure, Phosphorsäure, Methansulfonsäure, Benzol- oder p-Toluolsulfonsäure, oder Naphthalin-mono- oder -di-sulfonsäuren, oder

ß) mit Basen, wie z. B. Natrium – , Kalium – oder Calcium – hydroxid, – hydrid, – amid oder – carbonat, Natrium – oder Kalium – $C_1$ – $C_4$ – alkanolaten, Ammoniak, $C_1$ – $C_4$ – Alkylaminen, Di – ($C_1$ – $C_4$ – alkyl) – aminen oder Tri – ($C_1$ – $C_4$ – alkyl) – aminen.

Verwendet man beim erfindungsgemäßen Verfahren (a) beispielsweise 4 – Amino – 5 – methyl – 2 – (4,6 – dimethoxy – s – triazin – 2 – yl) – 2,4 – dihydro – 3H – 1,2,4 – triazol – 3 – on und 2 – Fluorbenzol – sulfonsäurechlorid (mindestens zwei Moläquivalente) als Ausgangsstoffe, so kann der Reaktionsablauf beim erfindungsgemäßen Verfahren (a) durch folgendes Formelschema skizziert werden:

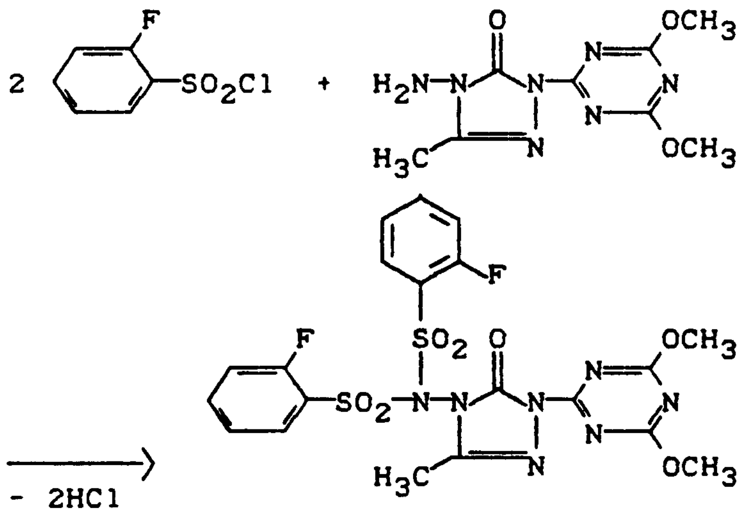


Verwendet man beim erfindungsgemäßen Verfahren (b) beispielsweise 4 – (2 – Difluormethoxy – phenylsulfonylamino) – 5 – trifluormethyl – 2,4 – dihydro – 3H – 1,2,4 – triazol – 3 – on und 4,6 – Dimethoxy – 2 – methylsulfonyl – pyrimidin als Ausgangsstoffe, so kann der Reaktionsablauf beim erfindungsgemäßen Verfahren (b) durch folgendes Formelschema skizziert werden:

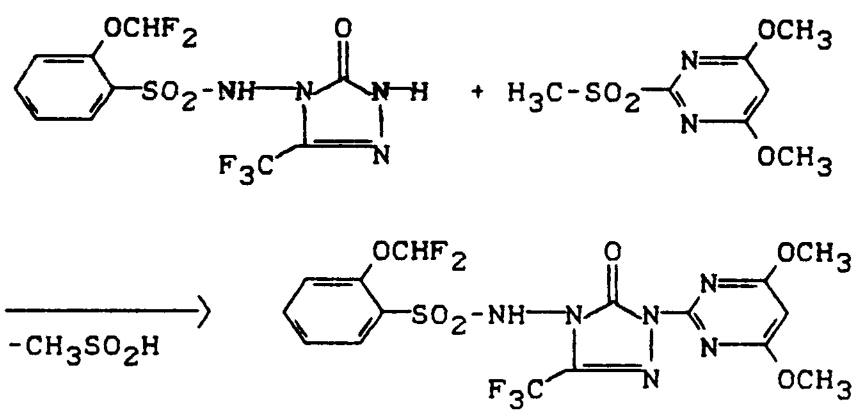


Die beim erfindungsgemäßen Verfahren (a) zur Herstellung von Verbindungen der Formel (I) als Ausgangsstoffe zu verwendenden substituierten Aminotriazolinone sind durch die Formel (II) allgemein definiert.

In Formel (II) haben $R^3$, $R^4$, X, Y und Z vorzugsweise diejenigen Bedeutungen, die bereits oben im Zusammenhang mit der Beschreibung der erfindungsgemäßen Verbindungen der Formel (I) vorzugsweise für $R^3$, $R^4$, X, Y und Z angegeben wurden.

Beispiele für die Ausgangsstoffe der Formel (II) sind in der nachstehenden Tabelle 1 aufgeführt.

Tabelle 1: Beispiele für die Ausgangsstoffe der Formel (II)

| $R^3$ | $R^4$ | X | Y | Z |
|---|---|---|---|---|
| H | $CH_3$ | N | CH | $C-CH_3$ |
| H | $CH_3$ | N | CH | $C-OCH_3$ |
| H | $CH_3$ | N | CH | $C-OC_2H_5$ |
| H | $OCH_3$ | N | CH | $C-OCH_3$ |
| H | $OCH_3$ | N | CH | C-Cl |
| H | H | N | CH | $C-CH_3$ |
| H | $CF_3$ | N | CH | $C-CH_3$ |
| H | $CF_3$ | N | CH | $C-OCH_3$ |
| H | $CF_3$ | N | CH | $C-CF_3$ |
| H | $OCH_3$ | N | CH | $C-OCHF_2$ |
| H | $CH_3$ | N | CH | $C-OCHF_2$ |
| H | $OCHF_2$ | N | CH | $C-OCHF_2$ |
| H | $CH_3$ | N | N | $C-OCH_3$ |

Tabelle 1 - Fortsetzung

| $R^3$ | $R^4$ | X | Y | Z |
|---|---|---|---|---|
| H | $OCH_3$ | N | N | C-$OCH_3$ |
| H | $OCH_3$ | N | N | C-Cl |
| H | $C_2H_5$ | N | CH | C-$OCH_3$ |
| H | $C_2H_5$ | N | N | C-$OCH_3$ |
| $CH_3$ | $CH_3$ | N | CH | C-$CH_3$ |
| $CH_3$ | $CH_3$ | N | CH | C-$OCH_3$ |
| $CH_3$ | $OCH_3$ | N | CH | C-$OCH_3$ |
| $CH_3$ | $OCH_3$ | N | CH | C-Cl |
| $CH_3$ | $OCHF_2$ | N | CH | C-$OCHF_2$ |
| $CH_3$ | H | N | CH | C-$CH_3$ |
| $CH_3$ | $CF_3$ | N | CH | C-$CH_3$ |
| $CH_3$ | $CF_3$ | N | CH | C-$OCH_3$ |
| $CH_3$ | $CF_3$ | N | CH | C-$CF_3$ |
| $CH_3$ | $OCH_3$ | N | CH | C-$OCHF_2$ |
| $CH_3$ | Cl | N | CH | C-$OCHF_2$ |
| $CH_3$ | $CH_3$ | N | CH | C-$OCHF_2$ |
| $CH_3$ | NH-$CH_3$ | N | CH | C-$OCH_3$ |
| $CH_3$ | $CH_3$ | N | N | C-$CH_3$ |
| $CH_3$ | $CH_3$ | N | N | C-$OCH_3$ |
| $CH_3$ | $OCH_3$ | N | N | C-$OCH_3$ |

Tabelle 1 - Fortsetzung

| $R^3$ | $R^4$ | X | Y | Z |
|---|---|---|---|---|
| $CH_3$ | $C_2H_5$ | N | CH | C-$OCH_3$ |
| $CH_3$ | $CH_3$ | N | N | C-$OC_2H_5$ |
| $CH_3$ | $C_2H_5$ | N | N | C-$OCH_3$ |
| $CH_3$ | $CH_3$ | N | N | C-Cl |
| $CH_3$ | $CH_3$ | CH | N | C-$CH_3$ |
| $CH_3$ | $OCH_3$ | CH | N | C-$OCH_3$ |
| $CH_3$ | $CH_3$ | N | CH | C-$SCH_3$ |
| H | $CH_3$ | N | CH | C-$N(CH_3)_2$ |
| H | $OCH_3$ | N | CH | C-$SCH_3$ |
| H | $OCH_3$ | N | N | C-$NHC_2H_5$ |
| H | $OC_2H_5$ | N | N | C-$NHCH_3$ |
| H | $CH_3$ | CH | CH | C-$CH_3$ |
| $OCH_3$ | $CH_3$ | N | CH | C-$CH_3$ |
| $OCH_3$ | $CH_3$ | N | CH | C-$OCH_3$ |
| $OCH_3$ | $OCH_3$ | N | CH | C-$OCH_3$ |
| $OCH_3$ | $OCH_3$ | N | CH | C-Cl |
| $OCH_3$ | H | N | CH | C-$CH_3$ |
| $OCH_3$ | $CF_3$ | N | CH | C-$OCH_3$ |
| $OCHF_2$ | $OCH_3$ | N | CH | C-$OCHF_2$ |
| $OCH_3$ | $CH_3$ | N | CH | C-$OCHF_2$ |
| $OCH_3$ | $CH_3$ | N | N | C-$CH_3$ |
| $OCH_3$ | $CH_3$ | N | N | C-$OCH_3$ |

Tabelle 1 - Fortsetzung

| $R^3$ | $R^4$ | X | Y | Z |
|---|---|---|---|---|
| $OCH_3$ | $OCH_3$ | N | N | $C-OCH_3$ |
| $OCH_3$ | $C_2H_5$ | N | CH | $C-OCH_3$ |
| $OCH_3$ | $C_2H_5$ | N | N | $C-OCH_3$ |
| $SCH_3$ | $CH_3$ | N | CH | $C-CH_3$ |
| $OCH_3$ | $CH_3$ | N | N | $C-OC_2H_5$ |
| $SCH_3$ | $CH_3$ | N | CH | $C-OCH_3$ |
| $SCH_3$ | $CH_3$ | N | CH | $C-OC_2H_5$ |
| $SCH_3$ | $OCH_3$ | N | CH | $C-OCH_3$ |
| $SCH_3$ | $OCH_3$ | N | CH | C-Cl |
| $SCH_3$ | H | N | CH | $C-CH_3$ |
| $SCH_3$ | $CF_3$ | N | CH | $C-CF_3$ |
| $SCH_3$ | $CF_3$ | N | CH | $C-OCH_3$ |
| $SCH_3$ | $CF_3$ | N | CH | $C-CH_3$ |
| $SCH_3$ | $OCH_3$ | N | CH | $C-OCHF_2$ |
| $SCH_3$ | $CH_3$ | N | CH | $C-OCHF_2$ |
| $SCH_3$ | $OCHF_2$ | N | CH | $C-OCHF_2$ |
| $SCH_3$ | $NHCH_3$ | N | CH | $C-OCH_3$ |
| $SCH_3$ | $CH_3$ | N | N | $C-CH_3$ |
| $SCH_3$ | $CH_3$ | N | N | $C-OCH_3$ |
| $SCH_3$ | $OCH_3$ | N | N | $C-OCH_3$ |
| $SCH_3$ | $OCH_3$ | N | N | C-Cl |

Tabelle 1 - Fortsetzung

| $R^3$ | $R^4$ | X | Y | Z |
|---|---|---|---|---|
| $SCH_3$ | $C_2H_5$ | N | CH | $C-OCH_3$ |
| $SCH_3$ | $C_2H_5$ | N | N | $C-OCH_3$ |
| $SCH_3$ | $OCH_3$ | N | N | $C-NH-C_2H_5$ |
| $SCH_3$ | $OC_2H_5$ | N | N | $C-NH-CH_3$ |
| $C_2H_5$ | $CH_3$ | N | CH | $C-CH_3$ |
| $C_2H_5$ | $CH_3$ | N | CH | $C-OCH_3$ |
| $C_2H_5$ | $CH_3$ | N | CH | $C-OC_2H_5$ |
| $C_2H_5$ | $OCH_3$ | N | CH | $C-OCH_3$ |
| $C_2H_5$ | $OCH_3$ | N | CH | C-Cl |
| $C_2H_5$ | H | N | CH | $C-CH_3$ |
| $C_2H_5$ | $CF_3$ | N | CH | $C-CH_3$ |
| $C_2H_5$ | $CF_3$ | N | CH | $C-OCH_3$ |
| $C_2H_5$ | $CF_3$ | N | CH | $C-CF_3$ |
| $C_2H_5$ | $OCH_3$ | N | CH | $C-OCHF_2$ |
| $C_2H_5$ | $CH_3$ | N | CH | $C-OCHF_2$ |
| $C_2H_5$ | $OCHF_2$ | N | CH | $C-OCHF_2$ |
| $C_2H_5$ | $NHCH_3$ | N | CH | $C-OCH_3$ |
| $C_2H_5$ | $CH_3$ | N | N | $C-CH_3$ |
| $C_2H_5$ | $CH_3$ | N | N | $C-OCH_3$ |
| $C_2H_5$ | $OCH_3$ | N | N | $C-OCH_3$ |
| $C_2H_5$ | $C_2H_5$ | N | N | $C-OCH_3$ |

Tabelle 1 - Fortsetzung

| $R^3$ | $R^4$ | X | Y | Z |
|---|---|---|---|---|
| $C_2H_5$ | $CH_3$ | N | N | $C-N(CH_3)_2$ |
| $C_2H_5$ | $OCH_3$ | N | N | $C-NHC_2H_5$ |
| $C_2H_5$ | $OC_2H_5$ | N | N | $C-NHCH_3$ |
| $C_3H_7$-n | $CH_3$ | N | CH | $C-CH_3$ |
| $C_3H_7$-n | $CH_3$ | N | CH | $C-OCH_3$ |
| $C_3H_7$-n | $CH_3$ | N | CH | $C-OC_2H_5$ |
| $C_3H_7$-n | $OCH_3$ | N | CH | $C-OCH_3$ |
| $C_3H_7$-n | $OCH_3$ | N | CH | C-Cl |
| $C_3H_7$-n | H | N | CH | $C-CH_3$ |
| $C_3H_7$-n | $CF_3$ | N | CH | $C-CH_3$ |
| $C_3H_7$-n | $CF_3$ | N | CH | $C-OCH_3$ |
| $C_3H_7$-n | $CF_3$ | N | CH | $C-CF_3$ |
| $C_3H_7$-n | $OCH_3$ | N | CH | $C-OCHF_2$ |
| $C_3H_7$-n | $CH_3$ | N | CH | $C-OCHF_2$ |
| $C_3H_7$-n | $OCHF_2$ | N | CH | $C-OCHF_2$ |
| $C_3H_7$-n | $NHCH_3$ | N | CH | $C-OCH_3$ |
| $C_3H_7$-n | $CH_3$ | N | N | $C-CH_3$ |
| $C_3H_7$-n | $CH_3$ | N | N | $C-OCH_3$ |
| $C_3H_7$-n | $OCH_3$ | N | N | $C-OCH_3$ |
| $C_3H_7$-n | $C_2H_5$ | N | N | $C-OCH_3$ |
| $C_3H_7$-n | $CH_3$ | N | N | $C-N(CH_3)_2$ |

Tabelle 1 - Fortsetzung

| $R^3$ | $R^4$ | X | Y | Z |
|---|---|---|---|---|
| $C_3H_7$-n | $OCH_3$ | N | N | C-$NHC_2H_5$ |
| $C_3H_7$-n | $OC_2H_5$ | N | N | C-$NHCH_3$ |
| $C_3H_7$-i | $CH_3$ | N | CH | C-$CH_3$ |
| $C_3H_7$-i | $CH_3$ | N | CH | C-$OCH_3$ |
| $C_3H_7$-i | $CH_3$ | N | CH | C-$OC_2H_5$ |
| $C_3H_7$-i | $OCH_3$ | N | CH | C-$OCH_3$ |
| $C_3H_7$-i | $OCH_3$ | N | CH | C-Cl |
| $C_3H_7$-i | H | N | CH | C-$CH_3$ |
| $C_3H_7$-i | $CF_3$ | N | CH | C-$CH_3$ |
| $C_3H_7$-i | $CF_3$ | N | CH | C-$OCH_3$ |
| $C_3H_7$-i | $CF_3$ | N | CH | C-$CF_3$ |
| $C_3H_7$-i | $OCH_3$ | N | CH | C-$OCHF_2$ |
| $C_3H_7$-i | $CH_3$ | N | CH | C-$OCHF_2$ |
| $C_3H_7$-i | $OCHF_2$ | N | CH | C-$OCHF_2$ |
| $C_3H_7$-i | $NHCH_3$ | N | CH | C-$OCH_3$ |
| $C_3H_7$-i | $CH_3$ | N | N | C-$CH_3$ |
| $C_3H_7$-i | $CH_3$ | N | N | C-$OCH_3$ |
| $C_3H_7$-i | $OCH_3$ | N | N | C-$OCH_3$ |
| $C_3H_7$-i | $C_2H_5$ | N | N | C-$OCH_3$ |
| $C_3H_7$-i | $CH_3$ | N | N | C-$N(CH_3)_2$ |
| $C_3H_7$-i | $OCH_3$ | N | N | C-$NHC_2H_5$ |

Tabelle 1 - Fortsetzung

| $R^3$ | $R^4$ | X | Y | Z |
|---|---|---|---|---|
| $C_3H_7$-i | $OC_2H_5$ | N | N | C-$NHCH_3$ |
| 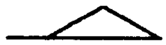 | $CH_3$ | N | CH | C-$CH_3$ |
| 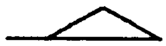 | $CH_3$ | N | CH | C-$OCH_3$ |
| 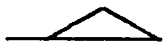 | $CH_3$ | N | CH | C-$OC_2H_5$ |
| 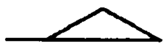 | $OCH_3$ | N | CH | C-$OCH_3$ |
| 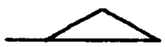 | $OCH_3$ | N | CH | C-Cl |
| 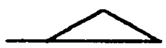 | H | N | CH | C-$CH_3$ |
| 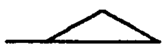 | $CF_3$ | N | CH | C-$CH_3$ |
| 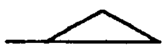 | $CF_3$ | N | CH | C-$OCH_3$ |
| 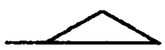 | $CF_3$ | N | CH | C-$CF_3$ |
| 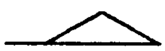 | $OCH_3$ | N | CH | C-$OCHF_2$ |
| 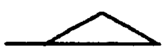 | $CH_3$ | N | CH | C-$OCHF_2$ |
| 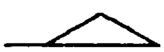 | $OCHF_2$ | N | CH | C-$OCHF_2$ |
| 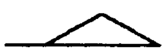 | $NHCH_3$ | N | CH | C-$OCH_3$ |
| 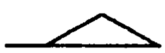 | $CH_3$ | N | N | C-$CH_3$ |
| 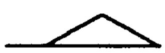 | $CH_3$ | N | N | C-$OCH_3$ |
| 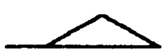 | $OCH_3$ | N | N | C-$OCH_3$ |
| 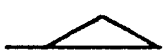 | $C_2H_5$ | N | N | C-$OCH_3$ |
| 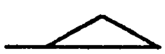 | $CH_3$ | N | N | C-$N(CH_3)_2$ |
| 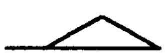 | $OCH_3$ | N | N | C-$NHC_2H_5$ |
| 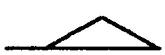 | $OC_2H_5$ | N | N | C-$NHCH_3$ |

Tabelle 1 - Fortsetzung

| $R^3$ | $R^4$ | X | Y | Z |
|---|---|---|---|---|
| $C_4H_9$-n | $CH_3$ | N | CH | C-$CH_3$ |
| $C_4H_9$-n | $CH_3$ | N | CH | C-$OCH_3$ |
| $C_4H_9$-n | $CH_3$ | N | CH | C-$OC_2H_5$ |
| $C_4H_9$-n | $OCH_3$ | N | CH | C-$OCH_3$ |
| $C_4H_9$-n | $OCH_3$ | N | CH | C-Cl |
| $C_4H_9$-n | H | N | CH | C-$CH_3$ |
| $C_4H_9$-n | $CF_3$ | N | CH | C-$CH_3$ |
| $C_4H_9$-n | $CF_3$ | N | CH | C-$OCH_3$ |
| $C_4H_9$-n | $CF_3$ | N | CH | C-$CF_3$ |
| $C_4H_9$-n | $OCH_3$ | N | CH | C-$OCHF_2$ |
| $C_4H_9$-n | $CH_3$ | N | CH | C-$OCHF_2$ |
| $C_4H_9$-n | $OCHF_2$ | N | CH | C-$OCHF_2$ |
| $C_4H_9$-n | $NHCH_3$ | N | CH | C-$OCH_3$ |
| $C_4H_9$-n | $CH_3$ | N | N | C-$CH_3$ |
| $C_4H_9$-n | $CH_3$ | N | N | C-$OCH_3$ |
| $C_4H_9$-n | $OCH_3$ | N | N | C-$OCH_3$ |
| $C_4H_9$-n | $C_2H_5$ | N | N | C-$OCH_3$ |
| $C_4H_9$-n | $CH_3$ | N | N | C-$N(CH_3)_2$ |
| $C_4H_9$-n | $OCH_3$ | N | N | C-$NHC_2H_5$ |
| $C_4H_9$-n | $OC_2H_5$ | N | N | C-$NHCH_3$ |

Tabelle 1 - Fortsetzung

| $R^3$ | $R^4$ | X | Y | Z |
|---|---|---|---|---|
| $C_4H_9$-s | $CH_3$ | N | CH | C-$CH_3$ |
| $C_4H_9$-s | $CH_3$ | N | CH | C-$OCH_3$ |
| $C_4H_9$-s | $CH_3$ | N | CH | C-$OC_2H_5$ |
| $C_4H_9$-s | $OCH_3$ | N | CH | C-$OCH_3$ |
| $C_4H_9$-s | $OCH_3$ | N | CH | C-Cl |
| $C_4H_9$-s | H | N | CH | C-$CH_3$ |
| $C_4H_9$-s | $CF_3$ | N | CH | C-$CH_3$ |
| $C_4H_9$-s | $CF_3$ | N | CH | C-$OCH_3$ |
| $C_4H_9$-s | $CF_3$ | N | CH | C-$CF_3$ |
| $C_4H_9$-s | $OCH_3$ | N | CH | C-$OCHF_2$ |
| $C_4H_9$-s | $CH_3$ | N | CH | C-$OCHF_2$ |
| $C_4H_9$-s | $OCHF_2$ | N | CH | C-$OCHF_2$ |
| $C_4H_9$-s | $NHCH_3$ | N | CH | C-$OCH_3$ |
| $C_4H_9$-s | $CH_3$ | N | N | C-$CH_3$ |
| $C_4H_9$-s | $CH_3$ | N | N | C-$OCH_3$ |
| $C_4H_9$-s | $OCH_3$ | N | N | C-$OCH_3$ |
| $C_4H_9$-s | $C_2H_5$ | N | N | C-$OCH_3$ |
| $C_4H_9$-s | $CH_3$ | N | N | C-$N(CH_3)_2$ |
| $C_4H_9$-s | $OCH_3$ | N | N | C-$NHC_2H_5$ |
| $C_4H_9$-s | $OC_2H_5$ | N | N | C-$NHCH_3$ |

Tabelle 1 - Fortsetzung

| $R^3$ | $R^4$ | X | Y | Z |
|---|---|---|---|---|
| $C_4H_9$-i | $CH_3$ | N | CH | C-$CH_3$ |
| $C_4H_9$-i | $CH_3$ | N | CH | C-$OCH_3$ |
| $C_4H_9$-i | $CH_3$ | N | CH | C-$OC_2H_5$ |
| $C_4H_9$-i | $OCH_3$ | N | CH | C-$OCH_3$ |
| $C_4H_9$-i | $OCH_3$ | N | CH | C-Cl |
| $C_4H_9$-i | H | N | CH | C-$CH_3$ |
| $C_4H_9$-i | $CF_3$ | N | CH | C-$CH_3$ |
| $C_4H_9$-i | $CF_3$ | N | CH | C-$OCH_3$ |
| $C_4H_9$-i | $CF_3$ | N | CH | C-$CF_3$ |
| $C_4H_9$-i | $OCH_3$ | N | CH | C-$OCHF_2$ |
| $C_4H_9$-i | $CH_3$ | N | CH | C-$OCHF_2$ |
| $C_4H_9$-i | $OCHF_2$ | N | CH | C-$OCHF_2$ |
| $C_4H_9$-i | $NHCH_3$ | N | CH | C-$OCH_3$ |
| $C_4H_9$-i | $CH_3$ | N | N | C-$CH_3$ |
| $C_4H_9$-i | $CH_3$ | N | N | C-$OCH_3$ |
| $C_4H_9$-i | $OCH_3$ | N | N | C-$OCH_3$ |
| $C_4H_9$-i | $C_2H_5$ | N | N | C-$OCH_3$ |
| $C_4H_9$-i | $CH_3$ | N | N | C-$N(CH_3)_2$ |
| $C_4H_9$-i | $OCH_3$ | N | N | C-$NHC_2H_5$ |
| $C_4H_9$-i | $OC_2H_5$ | N | N | C-$NHCH_3$ |

Tabelle 1 - Fortsetzung

| $R^3$ | $R^4$ | X | Y | Z |
|---|---|---|---|---|
| $C_4H_9$-t | $CH_3$ | N | CH | C-$CH_3$ |
| $C_4H_9$-t | $CH_3$ | N | CH | C-$OCH_3$ |
| $C_4H_9$-t | $CH_3$ | N | CH | C-$OC_2H_5$ |
| $C_4H_9$-t | $OCH_3$ | N | CH | C-$OCH_3$ |
| $C_4H_9$-t | $OCH_3$ | N | CH | C-Cl |
| $C_4H_9$-t | H | N | CH | C-$CH_3$ |
| $C_4H_9$-t | $CF_3$ | N | CH | C-$CH_3$ |
| $C_4H_9$-t | $CF_3$ | N | CH | C-$OCH_3$ |
| $C_4H_9$-t | $CF_3$ | N | CH | C-$CF_3$ |
| $C_4H_9$-t | $OCH_3$ | N | CH | C-$OCHF_2$ |
| $C_4H_9$-t | $CH_3$ | N | CH | C-$OCHF_2$ |
| $C_4H_9$-t | $OCHF_2$ | N | CH | C-$OCHF_2$ |
| $C_4H_9$-t | $NHCH_3$ | N | CH | C-$OCH_3$ |
| $C_4H_9$-t | $CH_3$ | N | N | C-$CH_3$ |
| $C_4H_9$-t | $CH_3$ | N | N | C-$OCH_3$ |
| $C_4H_9$-t | $OCH_3$ | N | N | C-$OCH_3$ |
| $C_4H_9$-t | $C_2H_5$ | N | N | C-$OCH_3$ |
| $C_4H_9$-t | $CH_3$ | N | N | C-$N(CH_3)_2$ |
| $C_4H_9$-t | $OCH_3$ | N | N | C-$NHC_2H_5$ |
| $C_4H_9$-t | $OC_2H_5$ | N | N | C-$NHCH_3$ |

Tabelle 1 - Fortsetzung

| $R^3$ | $R^4$ | X | Y | Z |
|---|---|---|---|---|
| $CF_3$ | $CH_3$ | N | CH | $C-CH_3$ |
| $CF_3$ | $CH_3$ | N | CH | $C-OCH_3$ |
| $CF_3$ | $CH_3$ | N | CH | $C-OC_2H_5$ |
| $CF_3$ | $OCH_3$ | N | CH | $C-OCH_3$ |
| $CF_3$ | $OCH_3$ | N | CH | C-Cl |
| $CF_3$ | H | N | CH | $C-CH_3$ |
| $CF_3$ | $CF_3$ | N | CH | $C-CH_3$ |
| $CF_3$ | $CF_3$ | N | CH | $C-OCH_3$ |
| $CF_3$ | $CF_3$ | N | CH | $C-CF_3$ |
| $CF_3$ | $OCH_3$ | N | CH | $C-OCHF_2$ |
| $CF_3$ | $CH_3$ | N | CH | $C-OCHF_2$ |
| $CF_3$ | $OCHF_2$ | N | CH | $C-OCHF_2$ |
| $CF_3$ | $NHCH_3$ | N | CH | $C-OCH_3$ |
| $CF_3$ | $CH_3$ | N | N | $C-CH_3$ |
| $CF_3$ | $CH_3$ | N | N | $C-OCH_3$ |
| $CF_3$ | $OCH_3$ | N | N | $C-OCH_3$ |
| $CF_3$ | $C_2H_5$ | N | N | $C-OCH_3$ |
| $CF_3$ | $CH_3$ | N | N | $C-N(CH_3)_2$ |
| $CF_3$ | $OCH_3$ | N | N | $C-NHC_2H_5$ |
| $CF_3$ | $OC_2H_5$ | N | N | $C-NHCH_3$ |

Tabelle 1 - Fortsetzung

| $R^3$ | $R^4$ | X | Y | Z |
|---|---|---|---|---|
| $C_6H_5$ | $CH_3$ | N | CH | $C-CH_3$ |
| $C_6H_5$ | $CH_3$ | N | CH | $C-OCH_3$ |
| $C_6H_5$ | $OCH_3$ | N | CH | $C-OCH_3$ |
| $C_6H_5$ | $OCH_3$ | N | CH | C-Cl |
| $C_6H_5$ | $CH_3$ | N | N | $C-OCH_3$ |
| $C_6H_5$ | $OCH_3$ | N | N | $C-OCH_3$ |
| $CH_2C_6H_5$ | $CH_3$ | N | CH | $C-CH_3$ |
| $CH_2C_6H_5$ | $CH_3$ | N | CH | $C-OCH_3$ |
| $CH_2C_6H_5$ | $OCH_3$ | N | CH | $C-OCH_3$ |
| $CH_2C_6H_5$ | $OCH_3$ | N | CH | C-Cl |
| $CH_2C_6H_5$ | $CH_3$ | N | N | $C-OCH_3$ |
| $CH_2C_6H_5$ | $OCH_3$ | N | N | $C-OCH_3$ |

Die bei Verfahren (a) als Ausgangsstoffe zu verwendenden substituierten Aminotriazolinone der Formel (II) sind noch nicht aus der Literatur bekannt. Man erhält die neuen Verbindungen der Formel (II), wenn man (α) Aminotriazolinone der allgemeinen Formel (VI)

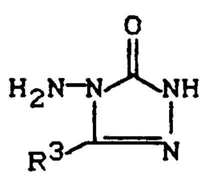

(VI)

in welcher

$R^3$ die oben angegebene Bedeutung hat,

gegebenenfalls mit Carbonylverbindungen der allgemeinen Formel (VII)

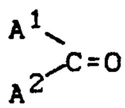

(VII)

in welcher

$A^1$ für Wasserstoff oder Alkyl steht und

$A^2$ für Alkyl oder Aryl steht,

gegebenenfalls in Gegenwart eines Katalysators, wie z.B. Schwefelsäure, Methansulfonsäure oder p-Toluolsulfonsäure, und gegebenenfalls in Gegenwart eines Verdünnungsmittels, wie z.B. Methylenchlorid, Toluol, Xylol, Methanol, Ethanol oder Isopropanol, bei Temperaturen zwischen 20 °C und 150 °C umsetzt,

gegebenenfalls die hierbei gebildeten (zum Teil neuen) Alkylidenaminotriazolinone der allgemeinen Formel (VIII)

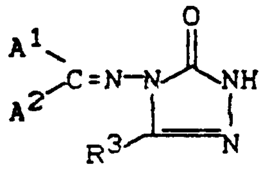

(VIII)

in welcher

$A^1$, $A^2$ und $R^3$ die oben angegebene Bedeutung haben,

isoliert und/oder quasi in situ, das heißt nach Einengen der Reaktionsmischung, mit Azinen der allgemeinen Formel (V)

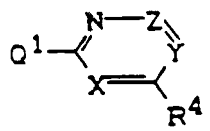

(V)

in welcher

$R^4$, X, Y und Z die oben angegebenen Bedeutungen haben und

$Q^1$ für Halogen, Benzylsulfonyl oder Alkylsulfonyl steht,

gegebenenfalls in Gegenwart eines Säureakzeptors, wie z. B. Kaliumcarbonat, und gegebenenfalls in Gegenwart eines Verdünnungsmittels, wie z. B. Acetonitril oder Dimethylformamid, bei Temperaturen zwischen 0 °C und 150 °C umsetzt

und die hierbei gebildeten (neuen) substituierten Alkylidenaminotriazolinone der allgemeinen Formel (IX),

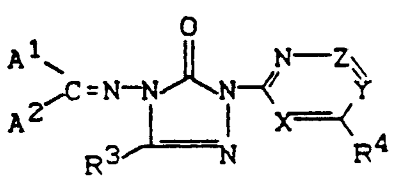

(IX)

in welcher

$A^1$, $A^2$, $R^3$, $R^4$, X, Y und Z die oben angegebene Bedeutung haben,

auf übliche Weise, beispielsweise durch Umsetzung mit Wasser, gegebenenfalls in Gegenwart eines organischen Lösungsmittels, wie z.B. Methanol, Ethanol, Isopropanol oder Dioxan, und gegebenenfalls in Gegenwart einer Säure, wie z.B. Salzsäure oder Schwefelsäure, bei Temperaturen zwischen 20 °C und 100 °C, zu den Verbindungen der Formel (II) hydrolysiert; oder wenn man

(ß) Oxadiazolone der allgemeinen Formel (X)

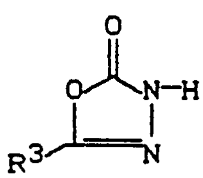

(X)

in welcher

$R^3$ die oben angegebene Bedeutung hat,

mit Azinen der allgemeinen Formel (V) – oben –
gegebenenfalls in Gegenwart eines Säureakzeptors, wie z.B. Kaliumcarbonat, und gegebenenfalls in Gegenwart eines Verdünnungsmittels, wie z.B. Acetonitril, Dioxan oder Dimethylformamid, bei Temperaturen zwischen 0 °C und 150 °C umsetzt
und die hierbei gebildeten (neuen) substituierten Oxadiazolone der allgemeinen Formel (XI)

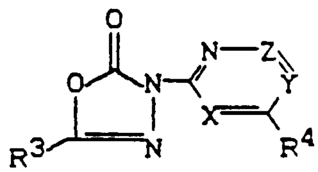

(XI)

in welcher
$R^3$, $R^4$, X, Y und Z die oben angegebenen Bedeutungen haben,
mit Hydrazin oder Hydrazinhydrat bei Temperaturen zwischen 0 °C und 150 °C umsetzt.

Manche Verbindungen der Formel (II) können ähnlich dem unter (α) beschriebenen Syntheseweg, jedoch ohne Einführung von Schutzgruppen mit Carbonylverbindungen der Formel (VII), d.h. durch direkte Umsetzung von Verbindungen der Formel (VI) mit Verbindungen der Formel (V) unter den unter (α) angegebenen Reaktionsbedingungen hergestellt werden.

Die als Zwischenprodukte benötigten Aminotriazolinone sind durch die Formel (VI) allgemein definiert. In Formel (VI) hat $R^3$ vorzugsweise diejenige Bedeutung, die bereits oben im Zusammenhang mit der Beschreibung der erfindungsgemäßen Verbindungen der Formel (I) vorzugsweise für $R^3$ angegeben wurden.

Als Beispiele für die Verbindungen der Formel (VI) seien genannt:
4-Amino-2,4-dihydro-3-H-1,2,4-triazol-3-on, 4-Amino-5-methyl-2,4-dihydro-3H-1,2,4-triazol-3-on, 4-Amino-5-methoxy-2,4-dihydro-3H-1,2,4-triazol-3-on, 4-Amino-5-methylthio-2,4-dihydro-3H-1,2,4-triazol-3-on, 4-Amino-5-ethyl-2,4-dihydro-3H-1,2,4-triazol-3-on, 4-Amino-5-propyl-2,4-dihydro-3H-1,2,4-triazol-3-on, 4-Amino-5-cyclopropyl-2,4-dihydro-3H-1,2,4-triazol-3-on, 4-Amino-5-isopropyl-2,4-dihydro-3H-1,2,4-triazol-3-on, 4-Amino-5-butyl-2,4-dihydro-3H-1,2,4-triazol-3-on, 4-Amino-5-isobutyl-2,4-dihydro-3H-1,2,4-triazol-3-on und 4-Amino-5-tert-butyl-2,4-dihydro-3H-1,2,4-triazol-3-on.

Die Aminotriazolinone der Formel (VI) sind bekannt und/oder können nach an sich bekannten Verfahren hergestellt werden (vgl. Chem. Ber. 98 (1965), 3025-3033; J.Heterocycl. Chem. 21 (1984), 1769-1774; Doga: Kim. Ser. 10 (1986), 34-39 – zitiert in chem. Abstracts 106: (1987), 138338e).

Die weiter als Zwischenprodukte benötigten Carbonylverbindungen sind durch die Formel (VII) definiert.

In Formel (VII) stehen vorzugsweise
$A^1$ für Wasserstoff oder Methyl und
$A^2$ für Methyl, Ethyl, Propyl, Isopropyl, Butyl, Isobutyl oder Phenyl.

Als Beispiele für die Verbindungen der Formel (VII) seien genannt:
Aceton, Methylethylketon, Methylpropylketon, Methylisopropylketon, Methylbutylketon, Methylisobutylketon und Benzaldehyd.

Die Zwischenprodukte der Formel (VII) sind bekannte organische Synthesechemikalien.

Die weiter als Zwischenprodukte benötigten Azine sind durch die Formel (V) allgemein definiert. In Formel (V) haben $R^4$, X, Y und Z vorzugsweise diejenigen Bedeutungen, die bereits oben im Zusammenhang mit der Beschreibung der erfindungsgemäßen Verbindungen der Formel (I) vorzugsweise für $R^4$, X, Y und Z angegeben wurden, und $Q^1$ steht vorzugsweise für Fluor, Chlor, Brom, Benzylsulfonyl oder $C_1-C_4$-Alkylsulfonyl, insbesondere für Chlor oder Methylsulfonyl.

Als Beispiele für die Verbindungen der Formel (V) seien genannt:
2-Chlor-, 2-Benzylsulfonyl- und 2-Methylsulfonyl-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidn, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluormethoxy-pyrimidin, -4-chlor-6-ethoxy-pyrimidin, -4-chlor-6-difluormethoxy-pyrimidin, -4-methoxy-5-methylpyrimidin, -4-trifluormethyl-6-difluormethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-

dimethoxy-pyrimidin, -4-methoxy-6-methylamino-pyrimidin sowie -4,6-bis-trifluormethyl-pyrimidin, ferner 2-Chlor-4,6-dimethyl-s-triazin 2-Chlor-4-methyl-6-methoxy-s-triazin, 2-Chlor-4,6-dimethoxy-s-triazin, 2,4-Dichlor-6-methoxy-s-triazin, 2-Chlor-4-ethyl-6-methoxy-s-tri-azin, 2-Chlor-4-methyl-6-ethoxy-s-triazin, 2-Chlor-4-ethoxy-6-methylamino-s-triazin, 2-Chlor-4-methoxy-6-methylamino-s-triazin, 2-Chlor-4-methoxy-6-ethylamino-s-triazin und 2-Chlor-4-ethoxy-6-ethylamino-s-triazin.

Die Azine der Formel (V) sind bekannt und/oder können nach an sich bekannten Verfahren hergestellt werden (vgl. J. Chem. Soc. 1957, 1830, 1833; J. Org. Chem. 26 (1961), 792; US-P 3 308 119 und US-P 4 711 959).

Die weiter als Zwischenprodukte zu verwendenden Oxadiazolone sind durch die Formel (X) allgemein definiert. In Formel (X) hat $R^3$ vorzugsweise diejenige Bedeutung, die bereits oben im Zusammenhang mit der Beschreibung der erfindungsgemäßen Verbindungen der Formel (I) vorzugsweise für $R^3$ angegeben wurde.

Als Beispiele für die Verbindungen der Formel (X) seien genannt:
1,3,4-Oxadiazol-2(3H)-on, 5-Methyl-1,3,4-oxadiazol-2(3H)-on, 5-Ethyl-1,3,4-oxadiazol-2-(3H)-on, 5-Propyl-1,3,4-oxadiazol-2(3H)-on, 5-Isopropyl-1,3,4-oxadiazol-2(3H)-on, 5-Butyl-1,3,4-oxadiazol-2(3H)-on, 5-Trifluormethyl-1,3,4-oxadiazol-2(3H)-on, 5-tert-Butyl-1,3,4-oxadiazol-2(3H)-on, 5-Cyclopropyl-1,3,4-oxadiazol-2(3H)-on, 5-Methyoxy-1,3,4-oxadiazol-2-(3H)-on und 5-Methylthio-1,3,4-oxadiazol-2(3H)-on.

Die Oxadiazolone der Formel (X) sind bekannt und/oder können nach an sich bekannten Verfahren hergestellt werden (vergl. Helv. Chim. Acta 55 (1972), 1174-1178).

Die beim erfindungsgemaßen Verfahren (a) zur Herstellung der neuen Verbindungen der Formel (I) weiter als Ausgangsstoffe zu verwendenden Sulfonsäurehalogenide bzw. -anhydride sind durch die Formel (III) allgemein definiert. In Formel (III) hat $R^1$ vorzugsweise diejenige Bedeutung, die oben im Rahmen der Beschreibung der erfindungsgemäßen Verbindungen der Formel (I) vorzugsweise angegeben wurde und Q steht vorzugsweise für Chlor.

Als Ausgangsstoffe der Formel (III) seien beispielsweise genannt:
Benzolsulfonsäurechlorid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-(2-Methoxy-ethoxy)-, 2-Methylsulfonyl-, 2-Isopropoxycarbonyl-, 2-Chlor-6-methyl-, 2-Brom-6-methyl-, 2-Methylthio-, 2-Trifluormethylthio-, 2-Difluormethylthio-, 2-Cyclopropyloxycarbonyl, 2-Phenoxy-, 2-Difluormethoxy-, 2-Trifluormethoxy, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonyl-benzolsulfonsäurechlorid sowie (2-Chlor-phenyl)-, (2-Cyano-phenyl)-, (2-Methoxycarbonyl-phenyl)-, (2-Trifluormethoxy-phenyl)-und (2-Difluormethoxy-phenyl)-methansulfonsäurechlorid, ferner 1-Methyl-4-methoxycarbonyl-pyrazol-5-sulfonsäurechlorid, 1-Methyl-4-ethoxycarbonyl-pyrazol-5-sulfonsäurechlorid, 1-Methyl-4-brom-pyrazol-5-sulfonsäurechlorid, 2-Methoxycarbonyl-thiophen-3-sulfonsäurechlorid, 3-Trifluormethyl-pyridin-2-sulfonsäurechlorid, 3-Dimethylaminocarbonyl-pyridin-2-sulfonsäurechlorid, 3-Dimethylaminocarbonyl-6-methyl-pyridin-2-sulfonsäurechlorid, 3-Dimethylaminocarbonyl-6-chlor-pyridin-2-sulfonsäurechlorid und 1-(Iso)Chinolinyl-4-ethoxycarbonyl-pyrazol-5-sulfonsäurechlorid.

Die Sulfonsäurehalogenide bzw. -anhydride der Formel (III) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid und Pyridin.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert.-butylat, ferner aliphatische, aromatische oder heterocyclische Amine, beispielsweise Triethylamin, Trimethylamin, Dimethylanilin, Dimethylbenzylamin, Pyridin, Picolin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen -50 °C und +50 °C, vorzugsweise bei Temperaturen zwischen -40 °C und +40 °C.

Zur Durchführung des erfindungsgemäßen Verfahrens (a) setzt man je Mol Aminotriazolinon der Formel (II) im allgemeinen zwischen 1 und 5 Mol, vorzugsweise zwischen 1 und 4 Mol, Sulfonsäurehalogenid bzw. Sulfonsäureanhydrid der Formel (III) ein. Falls doppelt sulfonylierte Verbindungen der Formel (I, $R^2$ = $-SO_2-R^1$) in einer Eintopfreaktion hergestellt werden sollen, sind je Mol Aminotriazolinon (II) mindestens 2 Mol Sulfonsäurehalogenid bzw. -anhydrid (III) einzusetzen.

Die Reaktionskomponenten können in beliebiger Reihenfolgen zusammengegeben werden.

In einer bevorzugten Ausführungsform des erfindungsgemäßen Verfahrens (a) werden die Ausgangsstoffe der Formeln (II) und (III) bei Raumtemperatur mit einem Verdünnungsmittel verrührt, und in diese Mischung wird - gegebenenfalls nach Abkühlen - der Säureakzeptor langsam eindosiert. Das Reaktionsgemisch wird dann bis zum Ende der Umsetzung gerührt.

Die Aufarbeitung kann auf übliche Weise durchgeführt werden, beispielsweise indem man - gegebenenfalls nach Einengen und/oder Verdünnen mit einem mit Wasser praktisch nicht mischbaren organischen Lösungsmittel, wie z. B. Methylenchlorid - mit Wasser wäshct, trocknet, filtriert und vom Filtrat das Lösungsmittel sorgfältig abdestilliert. Das im Rückstand verbleibende Rohprodukt kann auf übliche Weise, z. B. durch Säulenchromatographie und/oder durch Umkristallisation, weiter gereinigt werden.

Die wie oben beschrieben erhältlichen Verbindungen der Formel (I), in welcher $R^2$ für die Gruppierung $-SO_2-R^1$ steht, können durch Umsetzung mit Desulfonylierungsmitteln, gegebenenfalls in Gegenwart von Verdünnungsmitteln, zu Verbindungen der Formel (I), in welcher $R^2$ für Wasserstoff steht, umgesetzt werden.

Unter Desulfonylierungsmitteln sind in diesem Zusammenhang Stoffe zu verstehen, die aus N,N-Bis-sulfonyl-aminoverbindungen eine Sulfonylgruppierung abspalten können. Geeignete Desulfonylierungsmittel sind vor allem Alkalimetall- oder Erdalkalimetall-hydroxide, wie Natrium-, Kalium- und Calcium-hydroxid, Alkalimetall-alkoholate, wie Natrium- und Kalium-methylat und -ethylat, ferner Ammoniak, Alkylamine, wie Methylamin, Ethylamin, Propylamin und Butylamin, sowie Dialkylamine, wie Dimethylamin und Diethylamin. Vorzugsweise wird Ammoniak als Desulfonylierungsmittel eingesetzt.

Die Desulfonylierung wird vorzugsweise in Gegenwart von Verdünnungsmitteln durchgeführt. Bevorzugte Verdünnungsmittel sind neben Wasser polare organische Solventien, wie Methanol, Ethanol, Propan-ol, Isopropanol, 2-Methoxy-ethanol, 2-Ethoxy-ethanol und Dioxan.

Die Desulfonylierung wird im allgemeinen bei Temperaturen zwischen 0 °C und 150 °C, vorzugsweise bei Temperaturen zwischen 20 °C und 100 °C durchgeführt.

Die Reaktionskomponenten zur Desulfonylierung werden im allgemeinen bei Raumtemperatur vermischt und, gegebenenfalls bei erhöhter Temperatur, bis zum Ende der Umsetzung gerührt. Zur Aufarbeitung wird gegebenenfalls eingeengt, mit Wasser verdünnt und mit einer starken Säure, wie z. B. Salzsäure, angesäuert. Das dabei kristallin anfallende Produkt (I, $R^2$ = H) kann durch Absaugen isoliert werden.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Wasser, Methanol, Ethanol oder Aceton, und Zugabe einer geeigneten Säure bzw. Base. Die Salze können dann - gegebenenfalls nach längerem Rühren - durch Einengen oder Absaugen isoliert werden.

Die beim erfindungsgemäßen Verfahren (b) zur Herstellung von Verbindungen der Formel (I) als Ausgangsstoffe zu verwendenden Sulfonylaminotriazolinone sind durch die Formel (IV) allgemein definiert.

In Formel (IV) haben $R^1$, $R^2$ und $R^3$ vorzugsweise diejenigen Bedeutungen, die bereits oben im Zusammenhang mit der Beschreibung der erfindungsgemäßen Verbindungen der Formel (I) vorzugsweise für $R^1$, $R^2$ und $R^3$ angegeben wurden.

Als Beispiele für die Ausgangsstoffe der Formel (IV) seien genannt:

4-(Phenylsulfonylamino)-, 4-(2-Chlor-phenylsulfonylamino)-, 4-(2-Fluor-phenylsulfonylamino)-, 4-(2-Brom-phenylsulfonylamino)-, 4-(2-Methyl-phenylsulfonylamino)-, 4-(2-Methoxy-phenyl-

sulfonylamino)-, 4-(2-Trifluormethyl-phenylsulfonylamino)-, 4-(2-Methylthio-phenylsulfonylamino)-, 4-(2-Methylsulfonyl-phenylsulfonylamino)-, 4-(2-Isopropoxycarbonyl-phenylsulfonylamino)-, 4-(2-Chlor-6-methyl-phenylsulfonylamino)-, 4-(2-Phenyl-phenylsulfonylamino)-, 4-(2-Difluormethoxy-phenylsulfonylamino)-, 4-(2-Trifluormethoxy-phenylsulfonylamino)-, 4-(2-Dimethylaminosulfonyl-phenylsulfonylamino)-, 4-(2-Methoxycarbonyl-phenylsulfonylamino)-, 4-(2-Ethoxycarbonyl-phenylsulfonylamino)-, 4-(2-Chlor-phenylmethyl-sulfonylamino)-, 4-(2-Methoxycarbonyl-phenylmethylsulfonylamino)-, 4-(2-Ethoxycarbonyl-phenylmethylsulfonylamino)-, 4-(2-Difluormethoxy-phenylmethylsulfonylamino)-, 4-(2-Trifluormethoxy-phenylmethylsulfonylamino)-, 4-(1-Methyl-4-methoxycarbonyl-pyrazol-5-yl-sulfonylamino)-, 4-(1-Methyl-4-ethoxycarbonyl-pyrazol-5-yl-sulfonylamino)-, 4-(1-Methyl-4-brom-pyrazol-5-yl-sulfonylamino)-, 4-(2-Methoxycarbonyl-thiophen-3-yl-sulfonylamino)-, 4-(3-Trifluormethyl-pyridin-2-yl-sulfonylamino)-, 4-(3-Dimethylaminocarbonyl-pyridin-2-yl-sulfonylamino)- und 4-(1-Isochinolinyl-4-ethoxycarbonyl-pyrazol-5-yl-sulfonylamino)-2,4-dihydro-3H-1,2,4-triazol-3-on, -5-methyl-2,4-dihydro-3H-1,2,4-triazol-3-on, -5-ethyl-2,4-dihydro-3H-1,2,4-triazol-3-on, -5-propyl-2,4-dihydro-3H-1,2,4-triazol-3-on, -5-isopropyl-2,4-dihydro-3H-1,2,4-triazol-3-on, -5-trifluormethyl-2,4-dihydro-3H-1,2,4-triazol-3-on, -5-methoxy-2,4-dihydro-3H-1,2,4-triazol-3-on, -5-methylthio-2,4-dihydro-3H-1,2,4-triazol-3-on, -5-t-butyl-2,4-dihydro-3H-1,2,4-triazol-3-on, -5-benzyl-2,4-dihydro-3H-1,2,4-triazol-3-on und -5-phenyl-2,4-dihydro-3H-1,2,4-triazol-3-on.

Die als Ausgangsstoffe zu verwendenden Sulfonylaminotriazolinone der Formel (IV) sind noch nicht aus der Literatur bekannt. Man erhält die neuen Verbindungen der Formel (IV), wenn man Aminotriazolinone der allgemeinen Formel (VI)

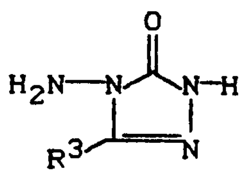

(VI)

in welcher

$R^3$ die oben angegebene Bedeutung hat,

mit Sulfonsäurehalogeniden oder Sulfonsäureanhydriden der allgemeinen Formel (III)

$R^1-SO_2-Q$ (III)

in welcher

$R^1$ und Q die oben angegebene Bedeutung haben,

gegebenenfalls in Gegenwart eines Säureakzeptors, wie z.B. Triethylamin, Pyridin oder 1,4-Diazabicyclo-[2,2,2]-octan (DABCO), und gegebenenfalls in Gegenwart eines Verdünnungsmittels, wie z.B. Methylenchlorid, Tetrahydrofuran oder Dioxan, bei Temperaturen zwischen -50 °C und +50 °C umsetzt.

Bezüglich der Ausgangsstoffe der Formeln (III) und (VI) gelten auch in diesem Zusammenhang die oben bei der Beschreibung des erfindungsgemäßen Verfahrens (a) zu diesen Stoffen gemachten Angaben.

Auch bezüglich der Azine der Formel (V), welche als Ausgangsstoffe für das erfindungsgemäße Verfahren (b) zu verwenden sind, gelten in diesem Zusammenhang die oben bei der Beschreibung des erfindungsgemäßen Verfahrens (a) gemachten Angaben.

Das erfindungsgemäße Verfahren (b) wird vorzugsweise unter Verwendung von Verdünnungsmitteln durchgeführt. Es kommen die gleichen Lösungsmittel in Betracht, die oben für Verfahren (a) als Verdünnungsmittel angegeben sind.

Verfahren (b) wird vorzugsweise in Gegenwart eines Säureakzeptors durchgeführt. Als Säureakzeptoren kommen die gleichen Säurebindemittel in Betracht, die oben für Verfahren (a) angegeben sind.

Die Reaktionstemperaturen können bei dem erfindungsgemäßen Verfahren (b) in einem größeren Bereich variiert werden. Im allgemeinen arbeitet man bei Temperaturen zwischen 0 °C und +150 °C, vorzugsweise bei Temperaturen zwischen +20 °C und +120 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethylharnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLOR-SULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 2-{4-[(6-Chlor-2-benzoxazolyl)-oxy]-phenoxy}-propansäure, deren Methyl-oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 2-[1-(Ethoxamino)-butyliden]-5-(2-ethylthiopropyl)-1,3-cyclohexadion (SETHOXYDIM); 4-Ethylamino-2-t-butylamino-6-methylthio-s-triazin (TERBUTRYNE) und 3-[[[[(4-Methoxy-6-methyl-1,3,5-triazin-2-yl)-amino]-carbonyl]-amino]-sulfonyl]-thiophen-2-carbonsäure-methylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in der Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

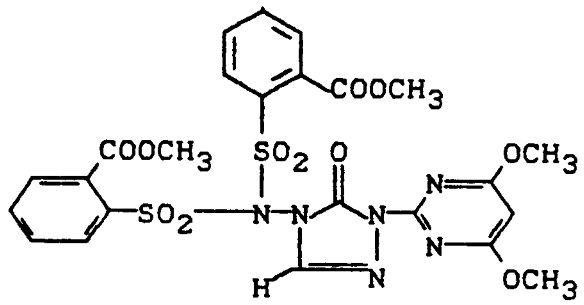

(Verfahren (a))

Eine Mischung aus 7,2 g (0,03 Mol) 4-Amino-2-(4,6-dimethoxy-pyrimidin-2-yl)-2,4-dihydro-3H-1,2,4-triazol-3-on, 21,0 g (0,09 Mol) 2-Methoxycarbonyl-benzolsulfonsäurechlorid und 50 ml Pyridin wird 12 Stunden bei 20°C gerührt. Anschließend wird im Wasserstrahlvakuum eingeengt, der Rückstand in Methylenchlorid aufgenommen und mit 2N-Salzsäure gewaschen. Nach Trocknen mit Magnesiumsulfat wird filtriert und vom Filtrat das Lösungsmittel im Wasserstrahlvakuum sorgfältig abdestilliert.

Man erhält 12,0 g (63 % der Theorie) 4-[Bis-(2-methoxycarbonyl-phenylsulfonyl)-amino]-2-(4,6-dimethoxy-pyrimidin-2-yl)-2,4-dihydro-3H-1,2,4-triazol-3-on als kristallinen Rückstand vom Schmelzpunkt 176°C.

Beispiel 2

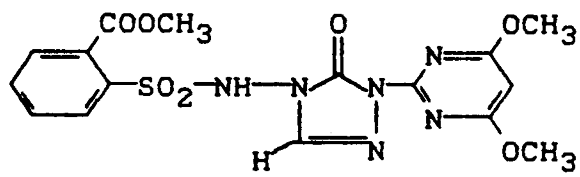

Eine Mischung aus 6,4 g (0,01 Mol) 4-[Bis-(2-methoxycarbonyl-phenylsulfonyl)-amino]-2-(4,6-dimethoxy-pyrimidin-2-yl)-2,4-dihydro-3H-1,2,4-triazol-3-on (vgl. Beispiel 1), 100 ml Methanol und 10 ml konzentriertem wässrigem Ammoniak wird 60 Minuten bei 50°C gerührt. Anschließend wird im Wasserstrahlvakuum eingeengt, der Rückstand in Wasser aufgenommen und mit 2N-Salzsäure angesäuert. Das hierbei kristallin angefallene Produkt wird durch Absaugen isoliert.

Man erhält 4,0 g (92 % der Theorie) 4-(2-Methoxycarbonyl-phenylsulfonylamino)-2-(4,6-dimethoxy-pyrimidin-2-yl)-2,4-dihydro-3H-1,2,4-triazol-3-on vom Schmelzpunkt 192°C.

Beispiel 3

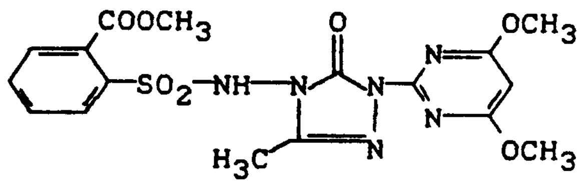

(Verfahren (a))

Eine Mischung aus 4,2 g (0,016 Mol) 4-Amino-5-methyl-2-(4,6-dimethoxy-pyrimidin-2-yl)-2,4-dihydro-3H-1,2,4-triazol-3-on, 11,7 g (0,05 Mol) 2-Methoxycarbonyl-benzolsulfonsäurechlorid und 50 ml Pyridin wird 12 Stunden bei 20 °C gerührt, dann mit Eiswasser verdünnt, mit 2N-Salzsäure angesäuert und mit Methylenchlorid geschüttelt. Die organische Phase wird abgetrennt, mit Magnesiumsulfat getrocknet und filtriert. Vom Filtrat wird das Lösungsmittel im Wasserstrahlvakuum sorgfältig abdestilliert.

Man erhält 3,8 g (50 % der Theorie) 4-(2-Methoxycarbonyl-phenylsulfonylamino)-5-methyl-2-(4,6-dimethoxy-pyrimidin-2-yl)-2,4-dihydro-3H-1,2,4-triazol-3-on als kristallinen Rückstand von Schmelzpunkt 174 °C.

Beispiel 4

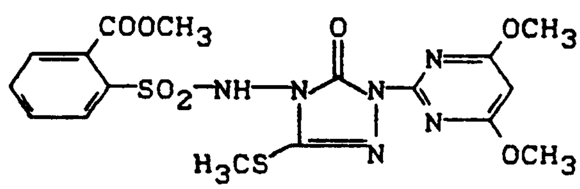

(Verfahren (a))

Eine Mischung aus 5,7 g (0,02 Mol) 4-Amino-5-methylthio-2-(4,6-dimethoxy-pyrimidin-2-yl)-2,4-dihydro-3H-1,2,4-triazol-3-on, 14,0 g (0,06 Mol) 2-Methoxycarbonyl-benzolsulfonsäurechlorid und 50 ml Pyridin wird 48 Stunden bei 20 °C gerührt. Anschließend wird im Wasserstrahlvakuum eingeengt, der Rückstand in Methylenchlorid aufgenommen, mit 2N-Salzsäure gewaschen, mit Magnesiumsulfat getrocknet und filtriert. Das Filtrat wird eingeengt und der Rückstand durch Säulenchromatographie an Kieselgel mit Methylenchlorid/Methanol (Vol. 10:1) gereinigt.

Man erhält 3,0 g (31 % der Theorie) 4-(2-Methoxycarbonyl-phenylsulfonylamino)-5-methylthio-2-(4,6-dimethoxy-pyrimidin-2-yl)-2,4-dihydro-3H-1,2,4-triazol-3-on vom Schmelzpunkt 179 °C.

Analog zu den Beispielen 1 bis 4 und entsprechend der allgemeinen Beschreibung des erfindungsgemäßen Verfahrens können die in der nachstehenden Tabelle 2 aufgeführten Verbindungen der Formel (I) hergestellt werden.

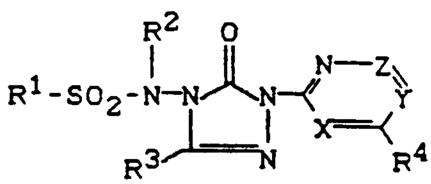 (I)

Tabelle 2 - Beispiele für die Verbindungen der Formel (I)

| Bsp.-Nr. | $R^1$ | $R^2$ | $R^3$ | $R^4$ | X | Y | Z | Schmelzpunkt (°C) |
|---|---|---|---|---|---|---|---|---|
| 5 | 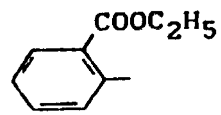 | H | H | $SO_2CH_3$ | N | CH | $C-OCH_3$ | 191 |
| 6 | 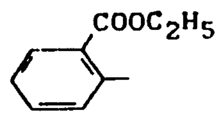 | H | $CH_3$ | $SO_2CH_3$ | N | CH | $C-OCH_3$ | 140 |
| 7 | 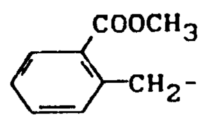 | H | $CH_3$ | $OCH_3$ | N | CH | $C-OCH_3$ | 178 |
| 8 | 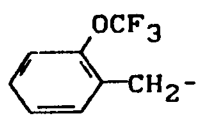 | H | $CH_3$ | $OCH_3$ | N | CH | $C-OCH_3$ | 186 |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 9 | 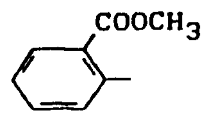 | 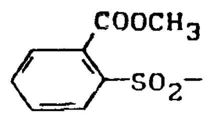 | H | $CH_3$ | N | CH | $C-CH_3$ | |
| 10 | 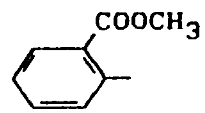 | H | H | $CH_3$ | N | CH | $C-CH_3$ | |
| 11 | 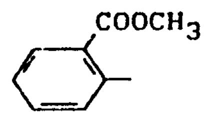 | Na | H | $CH_3$ | N | CH | $C-CH_3$ | |
| 12 | 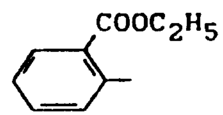 | 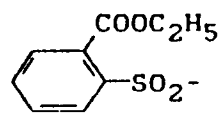 | H | $CH_3$ | N | CH | $C-CH_3$ | |
| 13 | 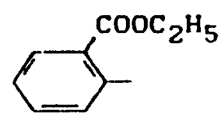 | H | H | $CH_3$ | N | CH | $C-CH_3$ | |
| 14 | 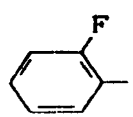 | 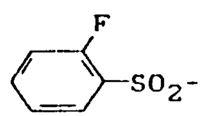 | H | $CH_3$ | N | CH | $C-CH_3$ | |

**Tabelle 2** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 15 | 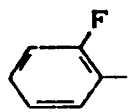 | H | H | $CH_3$ | N | CH | $C-CH_3$ | |
| 16 | 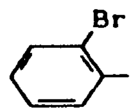 | 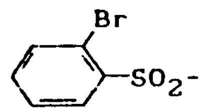 | H | $CH_3$ | N | CH | $C-CH_3$ | |
| 17 | 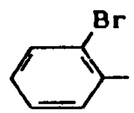 | H | H | $CH_3$ | N | CH | $C-CH_3$ | |
| 18 | 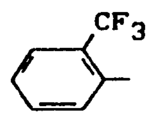 | 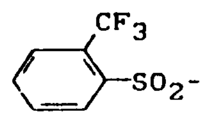 | H | $CH_3$ | N | CH | $C-CH_3$ | |
| 19 | 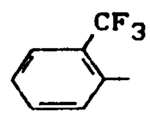 | H | H | $CH_3$ | N | CH | $C-CH_3$ | |
| 20 | 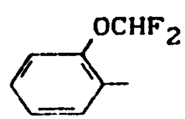 | 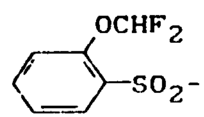 | H | $CH_3$ | N | CH | $C-CH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 21 | 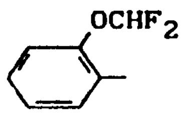 | 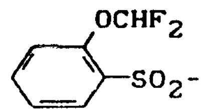 | H | $CH_3$ | N | N | $C-CH_3$ | |
| 22 | 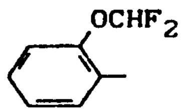 | H | H | $CH_3$ | N | CH | $C-CH_3$ | |
| 23 | 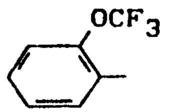 | 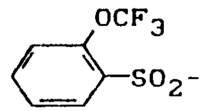 | H | $CH_3$ | N | CH | $C-CH_3$ | |
| 24 | 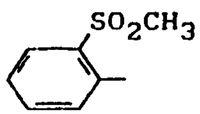 | 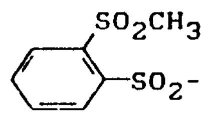 | H | $CH_3$ | N | CH | $C-CH_3$ | |
| 25 | 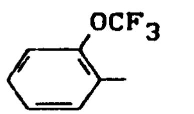 | H | H | $CH_3$ | N | CH | $C-CH_3$ | |
| 26 | 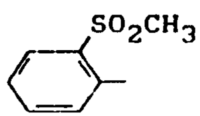 | H | H | $CH_3$ | N | CH | $C-CH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 27 | 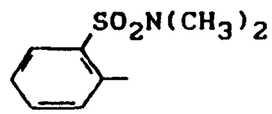 | H | H | $CH_3$ | N | CH | $C-CH_3$ | |
| 28 | 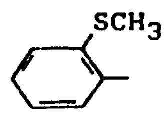 | H | H | $CH_3$ | N | CH | $C-CH_3$ | |
| 29 | 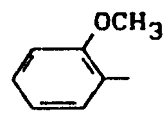 | H | H | $CH_3$ | N | CH | $C-CH_3$ | |
| 30 | 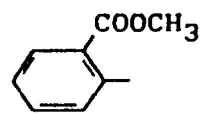 | 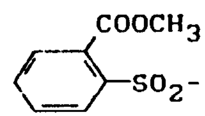 | H | $CH_3$ | N | CH | $C-OCH_3$ | |
| 31 | 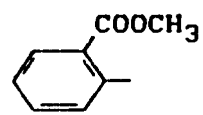 | H | H | $CH_3$ | N | CH | $C-OCH_3$ | |
| 32 | 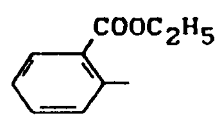 | 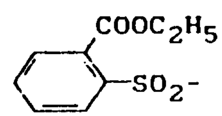 | H | $CH_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 33 | 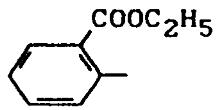 | H | H | $CH_3$ | N | CH | $C-OCH_3$ | |
| 34 | 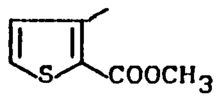 | 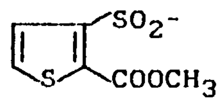 | H | $CH_3$ | N | CH | $C-OCH_3$ | |
| 35 | 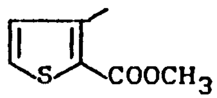 | H | H | $CH_3$ | N | CH | $C-OCH_3$ | |
| 36 | 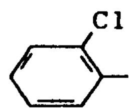 | H | H | $CH_3$ | N | CH | $C-OCH_3$ | 181 |
| 37 | 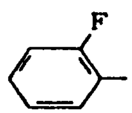 | H | H | $CH_3$ | N | CH | $C-OCH_3$ | |
| 38 | 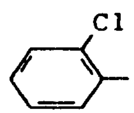 | 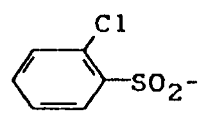 | H | $CH_3$ | N | CH | $C-OCH_3$ | |

<u>Tabelle 2</u> - Fortsetzung

| Bsp.-Nr. | $R^1$ | $R^2$ | $R^3$ | $R^4$ | X | Y | Z | Schmelzpunkt(°C) |
|---|---|---|---|---|---|---|---|---|
| 39 | 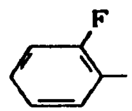 | 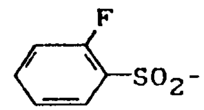 | H | $CH_3$ | N | CH | $C-OCH_3$ | |
| 40 | 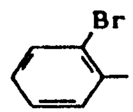 | 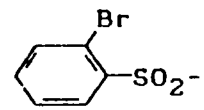 | H | $CH_3$ | N | CH | $C-OCH_3$ | |
| 41 | 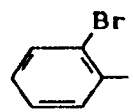 | H | H | $CH_3$ | N | CH | $C-OCH_3$ | |
| 42 | 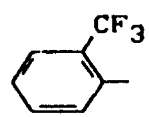 | 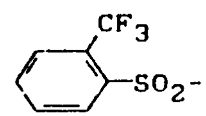 | H | $CH_3$ | N | CH | $C-OCH_3$ | |
| 43 | 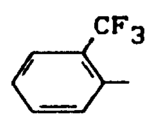 | H | H | $CH_3$ | N | CH | $C-OCH_3$ | |
| 44 | 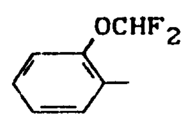 | 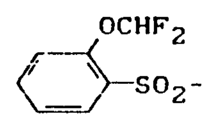 | H | $CH_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 45 | 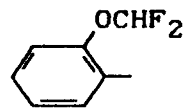 | H | H | $CH_3$ | N | CH | $C-OCH_3$ | |
| 46 | 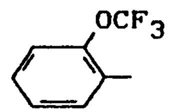 | 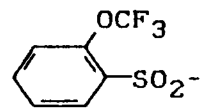 | H | $CH_3$ | N | CH | $C-OCH_3$ | |
| 47 | 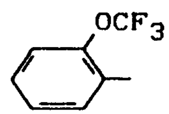 | H | H | $CH_3$ | N | CH | $C-OCH_3$ | 219 |
| 48 | 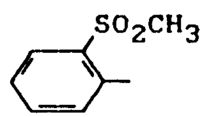 | H | H | $CH_3$ | N | CH | $C-OCH_3$ | |
| 49 | 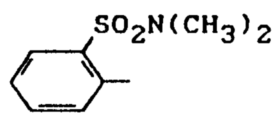 | H | H | $CH_3$ | N | CH | $C-OCH_3$ | |
| 50 | 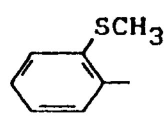 | H | H | $CH_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 51 | 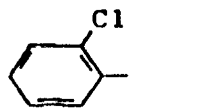 | 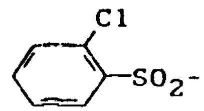 | H | $CH_3$ | N | CH | $C-CH_3$ | |
| 52 | 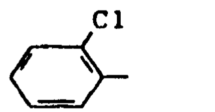 | H | H | $CH_3$ | N | CH | $C-CH_3$ | |
| 53 | 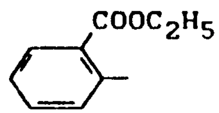 | 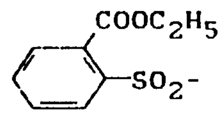 | H | $OCH_3$ | N | CH | $C-OCH_3$ | |
| 54 | 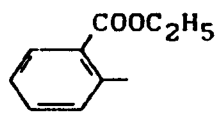 | H | H | $OCH_3$ | N | CH | $C-OCH_3$ | |
| 55 | 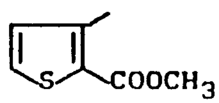 | 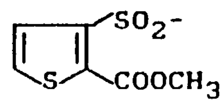 | H | $OCH_3$ | N | CH | $C-OCH_3$ | |
| 56 | 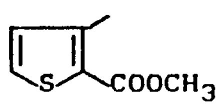 | H | H | $OCH_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 57 | 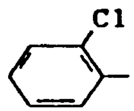 | 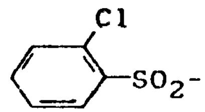 | H | $OCH_3$ | N | CH | $C-OCH_3$ | |
| 58 | 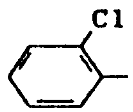 | H | H | $OCH_3$ | N | CH | $C-OCH_3$ | |
| 59 | 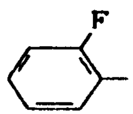 | 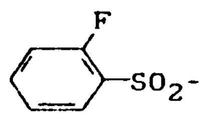 | H | $OCH_3$ | N | CH | $C-OCH_3$ | |
| 60 | 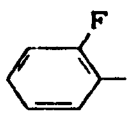 | H | H | $OCH_3$ | N | CH | $C-OCH_3$ | |
| 61 | 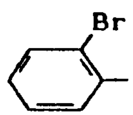 | 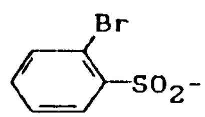 | H | $OCH_3$ | N | CH | $C-OCH_3$ | |
| 62 | 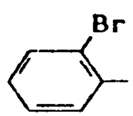 | H | H | $OCH_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelz-punkt(°C) |
|---|---|---|---|---|---|---|---|---|
| 63 | 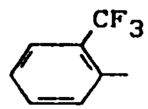 | 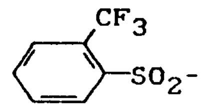 | H | $OCH_3$ | N | CH | $C-OCH_3$ | |
| 64 | 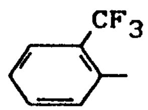 | H | H | $OCH_3$ | N | CH | $C-OCH_3$ | |
| 65 | 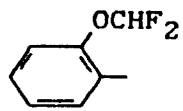 | 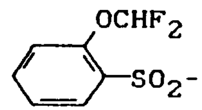 | H | $OCH_3$ | N | CH | $C-OCH_3$ | |
| 66 | 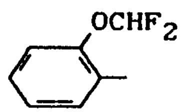 | H | H | $OCH_3$ | N | CH | $C-OCH_3$ | |
| 67 | 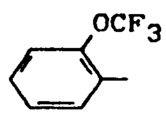 | 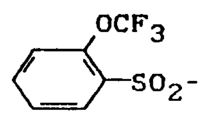 | H | $OCH_3$ | N | CH | $C-OCH_3$ | |
| 68 | 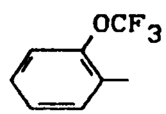 | H | H | $OCH_3$ | N | CH | $C-OCH_3$ | 208 |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 69 | 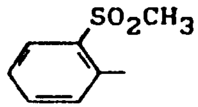 | H | H | $OCH_3$ | N | CH | $C-OCH_3$ | |
| 70 | 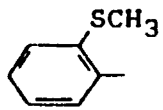 | H | H | $OCH_3$ | N | CH | $C-OCH_3$ | |
| 71 | 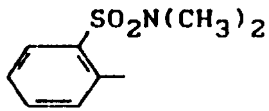 | H | H | $OCH_3$ | N | CH | $C-OCH_3$ | |
| 72 | 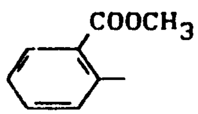 | 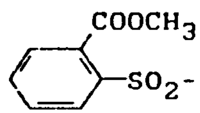 | H | $CF_3$ | N | CH | $C-OCH_3$ | |
| 73 | 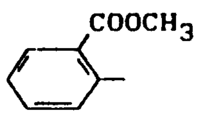 | 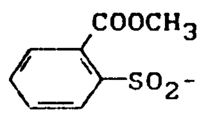 | H | $OCH_3$ | N | CH | C-Cl | |
| 74 | 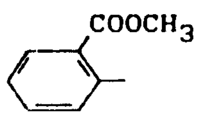 | H | H | $CF_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelzpunkt(°C) |
|---|---|---|---|---|---|---|---|---|
| 75 | 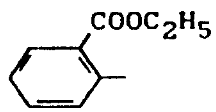 | 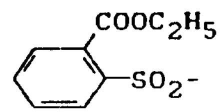 | H | $CF_3$ | N | CH | $C-OCH_3$ | |
| 76 | 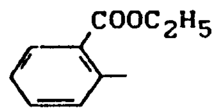 | H | H | $CF_3$ | N | CH | $C-OCH_3$ | |
| 77 | 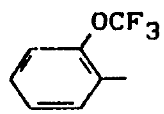 | H | H | $CF_3$ | N | CH | $C-OCH_3$ | |
| 78 | 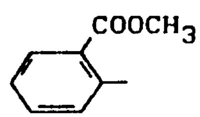 | H | H | $OCH_3$ | N | CH | C-Cl | |
| 79 | 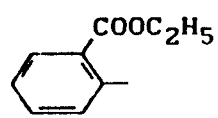 | 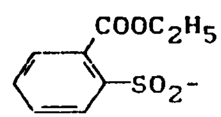 | H | $OCH_3$ | N | CH | C-Cl | |
| 80 | 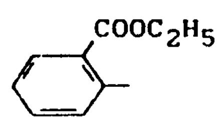 | H | H | $OCH_3$ | N | CH | C-Cl | |

**Tabelle 2** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 81 | 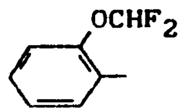 | H | H | $OCH_3$ | N | CH | C-Cl | |
| 82 | 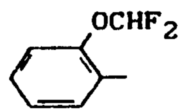 | 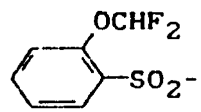 | H | $OCH_3$ | N | CH | C-Cl | |
| 83 | 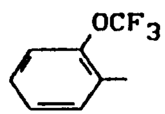 | 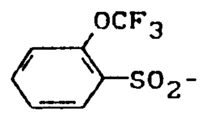 | H | $OCH_3$ | N | CH | C-Cl | |
| 84 | 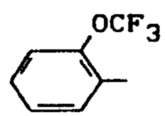 | H | H | $OCH_3$ | N | CH | C-Cl | |
| 85 | 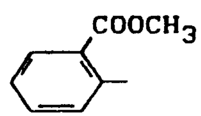 | 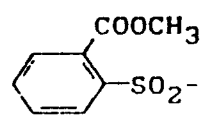 | H | $OCH_3$ | N | CH | C-$OCHF_2$ | |
| 86 | 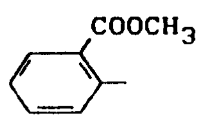 | H | H | $OCH_3$ | N | CH | C-$OCHF_2$ | |

Tabelle 2 - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 87 | 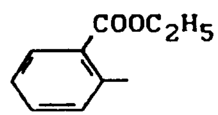 | 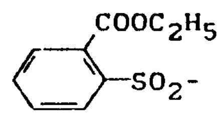 | H | $OCH_3$ | N | CH | $C-OCHF_2$ | |
| 88 | 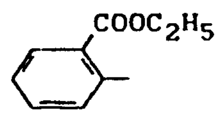 | H | H | $OCH_3$ | N | CH | $C-OCHF_2$ | |
| 89 | 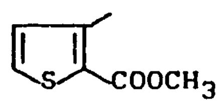 | 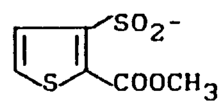 | H | $OCH_3$ | N | CH | $C-OCHF_2$ | |
| 90 | 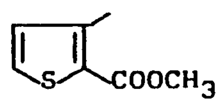 | H | H | $OCH_3$ | N | CH | $C-OCHF_2$ | |
| 91 | 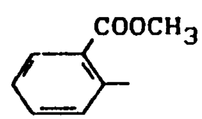 | 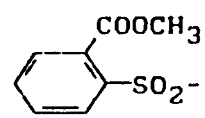 | H | $OCHF_2$ | N | CH | $C-OCHF_2$ | 198 |
| 92 | 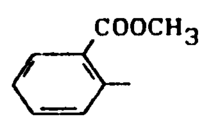 | H | H | $OCHF_2$ | N | CH | $C-OCHF_2$ | 180 |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 93 | 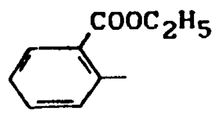 | 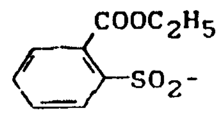 | H | $OCHF_2$ | N | CH | $C-OCHF_2$ | |
| 94 | 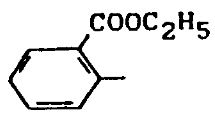 | H | H | $OCHF_2$ | N | CH | $C-OCHF_2$ | |
| 95 | 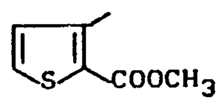 | 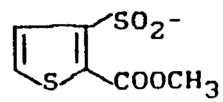 | H | $OCHF_2$ | N | CH | $C-OCHF_2$ | |
| 96 | 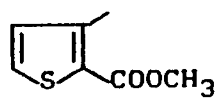 | H | H | $OCHF_2$ | N | CH | $C-OCHF_2$ | |
| 97 | 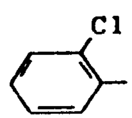 | 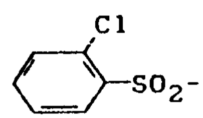 | H | $OCHF_2$ | N | CH | $C-OCHF_2$ | |
| 98 | 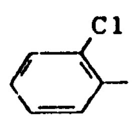 | H | H | $OCHF_2$ | N | CH | $C-OCHF_2$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 99 | 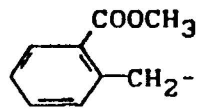 | 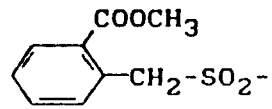 | H | $OCH_3$ | N | CH | $C-OCH_3$ | |
| 100 | 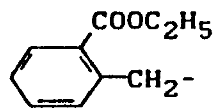 | H | H | $OCH_3$ | N | CH | $C-OCH_3$ | |
| 101 | 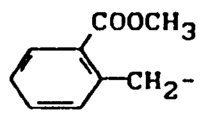 | 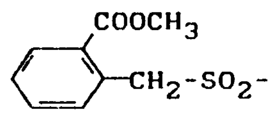 | H | $OCH_3$ | N | CH | C-Cl | |
| 102 | 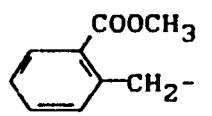 | H | H | $OCH_3$ | N | CH | C-Cl | |
| 103 | 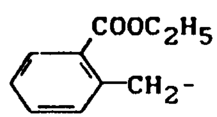 | 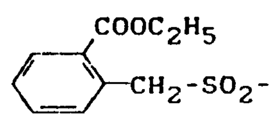 | H | $OCH_3$ | N | CH | $C-OCH_3$ | |
| 104 | 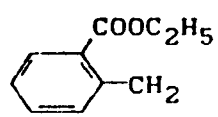 | H | H | $OCH_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 105 | 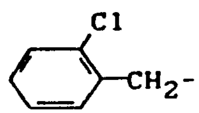 | 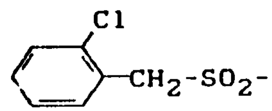 | H | $OCH_3$ | N | CH | $C-OCH_3$ | |
| 106 | 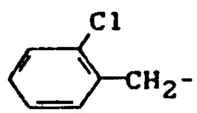 | H | H | $OCH_3$ | N | CH | $C-OCH_3$ | |
| 107 | 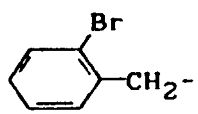 | 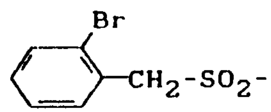 | H | $OCH_3$ | N | CH | $C-OCH_3$ | |
| 108 | 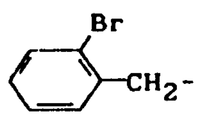 | 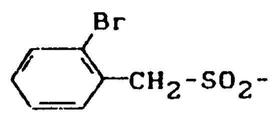 | H | $OCH_3$ | N | CH | $C-OCHF_2$ | |
| 109 | 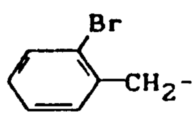 | H | H | $OCH_3$ | N | CH | $C-OCH_3$ | |
| 110 | 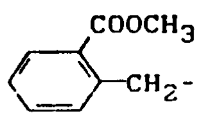 | 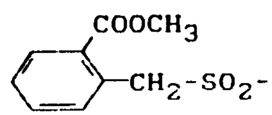 | H | $OCH_3$ | N | CH | $C-OCHF_2$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 111 | 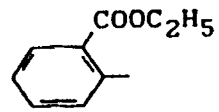 | H | H | $CH_3$ | N | N | $C-CH_3$ | |
| 112 | 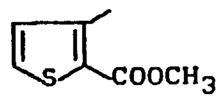 | 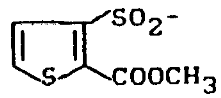 | H | $CH_3$ | N | N | $C-CH_3$ | |
| 113 | 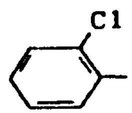 | 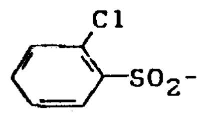 | H | $CH_3$ | N | N | $C-CH_3$ | |
| 114 | 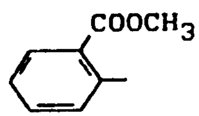 | 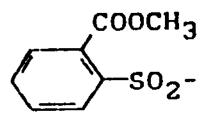 | H | $CH_3$ | N | N | $C-OCH_3$ | |
| 115 | 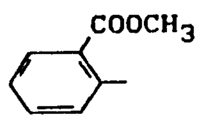 | H | H | $CH_3$ | N | N | $C-OCH_3$ | |
| 116 | 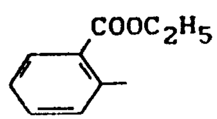 | 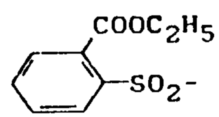 | H | $CH_3$ | N | N | $C-OCH_3$ | |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 117 | 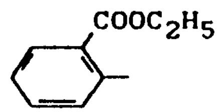 | H | H | $CH_3$ | N | N | $C-OCH_3$ | |
| 118 | 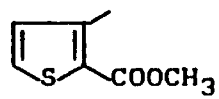 | 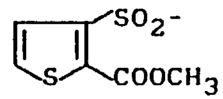 | H | $CH_3$ | N | N | $C-OCH_3$ | |
| 119 | 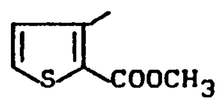 | H | H | $CH_3$ | N | N | $C-OCH_3$ | |
| 120 | 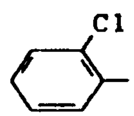 | Na | H | $CH_3$ | N | N | $C-OCH_3$ | |
| 121 | 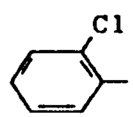 | 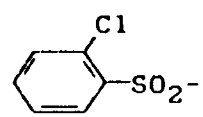 | H | $CH_3$ | N | N | $C-OCH_3$ | |
| 122 | 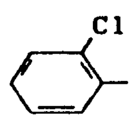 | H | H | $CH_3$ | N | N | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 123 | 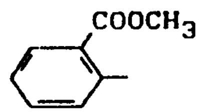 | 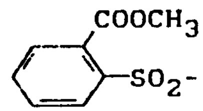 | H | $OCH_3$ | N | N | $C-OCH_3$ | |
| 124 | 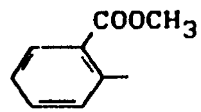 | H | H | $OCH_3$ | N | N | $C-OCH_3$ | |
| 125 | 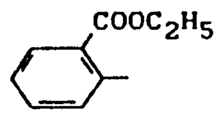 | 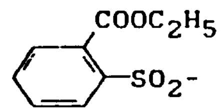 | H | $OCH_3$ | N | N | $C-OCH_3$ | |
| 126 | 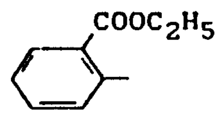 | H | H | $OCH_3$ | N | N | $C-OCH_3$ | |
| 127 | 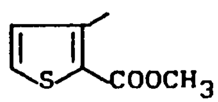 | 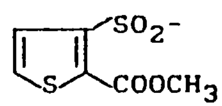 | H | $OCH_3$ | N | N | $C-OCH_3$ | |
| 128 | 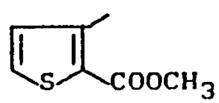 | H | H | $OCH_3$ | N | N | $C-OCH_3$ | |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 129 | 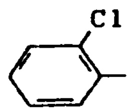 | 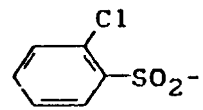 | H | $OCH_3$ | N | N | $C-OCH_3$ | |
| 130 | 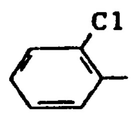 | H | H | $OCH_3$ | N | N | $C-OCH_3$ | |
| 131 | 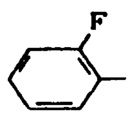 | 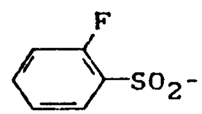 | H | $OCH_3$ | N | N | $C-OCH_3$ | |
| 132 | 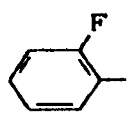 | H | H | $OCH_3$ | N | N | $C-OCH_3$ | |
| 133 | 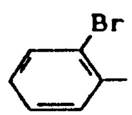 | 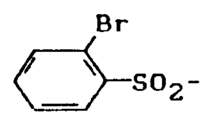 | H | $OCH_3$ | N | N | $C-OCH_3$ | |
| 134 | 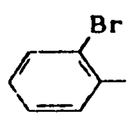 | H | H | $OCH_3$ | N | N | $C-OCH_3$ | |

<u>Tabelle 2</u> - Fortsetzung

| Bsp.-Nr. | $R^1$ | $R^2$ | $R^3$ | $R^4$ | X | Y | Z | Schmelzpunkt(°C) |
|---|---|---|---|---|---|---|---|---|
| 135 | 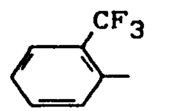 | 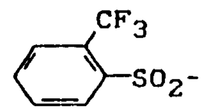 | H | $OCH_3$ | N | N | C-$OCH_3$ | |
| 136 | 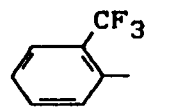 | H | H | $OCH_3$ | N | N | C-$OCH_3$ | |
| 137 | 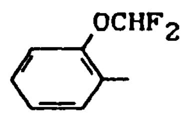 | 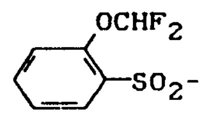 | H | $OCH_3$ | N | N | C-$OCH_3$ | |
| 138 | 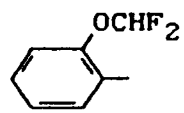 | H | H | $OCH_3$ | N | N | C-$OCH_3$ | |
| 139 | 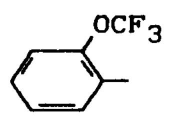 | 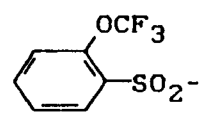 | H | $OCH_3$ | N | N | C-$OCH_3$ | |
| 140 | 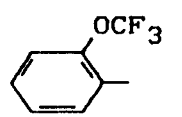 | H | H | $OCH_3$ | N | N | C-$OCH_3$ | 201 |

<u>Tabelle 2</u> - Fortsetzung

| Bsp.-Nr. | $R^1$ | $R^2$ | $R^3$ | $R^4$ | X | Y | Z | Schmelzpunkt(°C) |
|---|---|---|---|---|---|---|---|---|
| 141 | 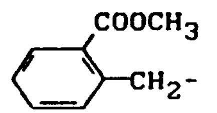 | 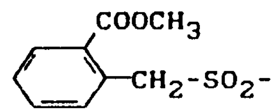 | H | $OCHF_2$ | N | CH | $C-OCHF_2$ | |
| 142 | 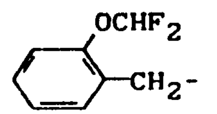 | 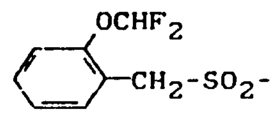 | H | $OCH_3$ | N | CH | $C-OCH_3$ | |
| 143 | 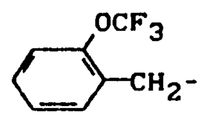 | 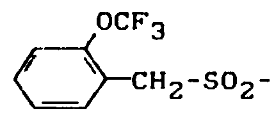 | H | $OCH_3$ | N | CH | $C-OCH_3$ | |
| 144 | 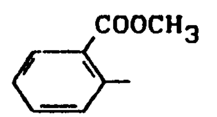 | 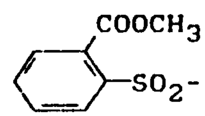 | H | $CH_3$ | N | N | $C-CH_3$ | |
| 145 | 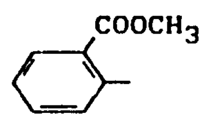 | H | H | $CH_3$ | N | N | $C-CH_3$ | |
| 146 | 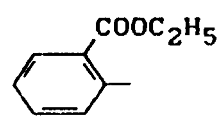 | 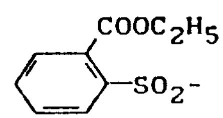 | H | $CH_3$ | N | N | $C-CH_3$ | |

**Tabelle 2** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 147 | 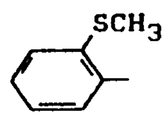 | 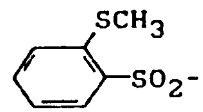 | H | $OCH_3$ | N | N | $C-OCH_3$ | |
| 148 | 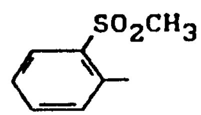 | 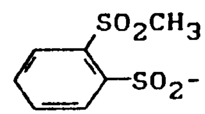 | H | $OCH_3$ | N | N | $C-OCH_3$ | |
| 149 | 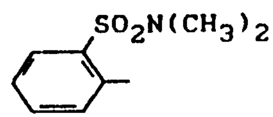 | 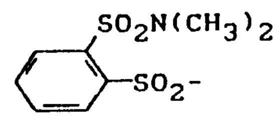 | H | $OCH_3$ | N | N | $C-OCH_3$ | |
| 150 | 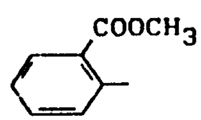 | 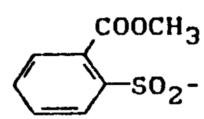 | H | $OCH_3$ | N | N | C-Cl | |
| 151 | 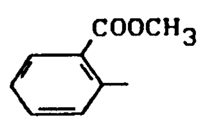 | 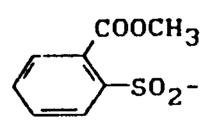 | H | $C_2H_5$ | N | N | $C-OCH_3$ | |
| 152 | 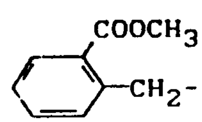 | 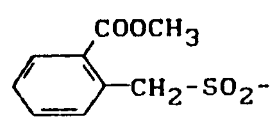 | H | $OCH_3$ | N | N | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 153 | 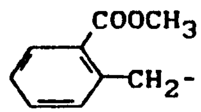 | H | H | $OCH_3$ | N | N | $C-OCH_3$ | |
| 154 | 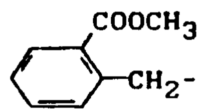 | H | H | $OCH_3$ | N | N | $C-CH_3$ | |
| 155 | 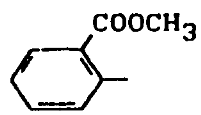 | 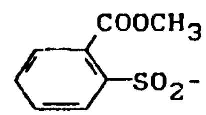 | H | $OCH_3$ | N | N | $C-NHC_2H_5$ | |
| 156 | 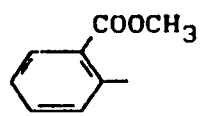 | 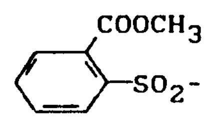 | H | $OC_2H_5$ | N | N | $C-NHCH_3$ | |
| 157 | 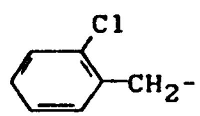 | 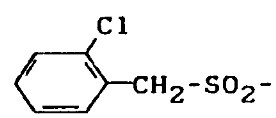 | H | $OCH_3$ | N | N | $C-OCH_3$ | |
| 158 | 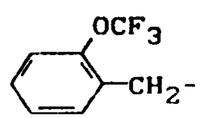 | 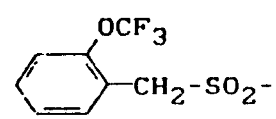 | H | $OCH_3$ | N | N | $C-OCH_3$ | |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 159 | 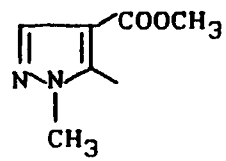 | 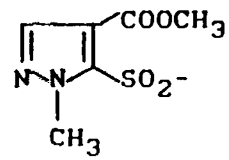 | H | $OCH_3$ | N | CH | $C-OCH_3$ | |
| 160 | 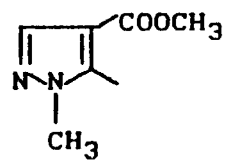 | H | H | $OCH_3$ | N | CH | $C-OCH_3$ | |
| 161 | 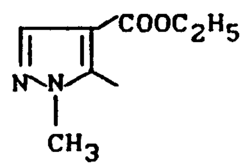 | 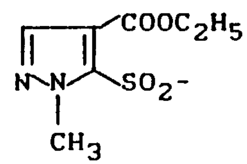 | H | $OCH_3$ | N | CH | $C-OCH_3$ | |
| 162 | 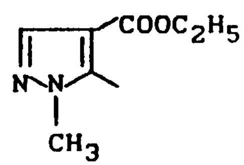 | H | H | $OCH_3$ | N | CH | $C-OCH_3$ | |
| 163 | 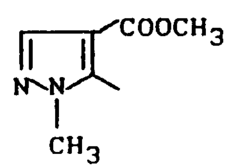 | 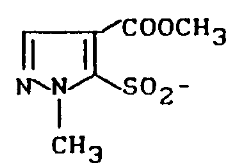 | H | $OCH_3$ | N | N | $C-OCH_3$ | |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 164 | 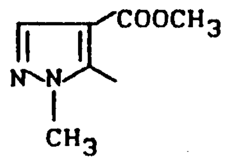 | H | H | $OCH_3$ | N | N | $C-OCH_3$ | |
| 165 | 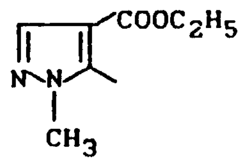 | 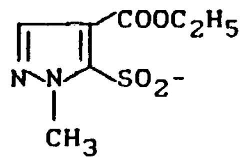 | H | $OCH_3$ | N | N | $C-OCH_3$ | |
| 166 | 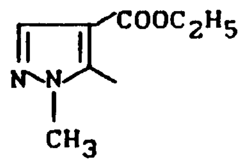 | H | H | $OCH_3$ | N | N | $C-OCH_3$ | |
| 167 | 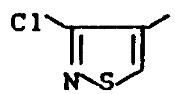 | 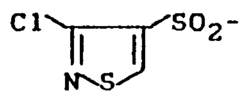 | H | $OCH_3$ | N | N | $C-OCH_3$ | |
| 168 | 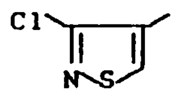 | 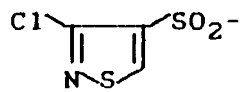 | H | $CH_3$ | N | CH | $C-CH_3$ | |
| 169 | 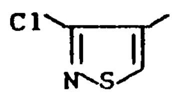 | H | H | $CH_3$ | N | CH | $C-CH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 170 | 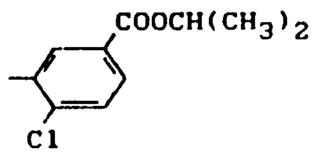 | H | H | $OCH_3$ | N | N | $C-OCH_3$ | |
| 171 | 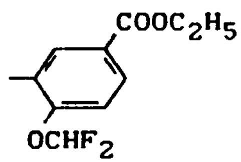 | H | H | $OCH_3$ | N | CH | $C-OCH_3$ | |
| 172 | 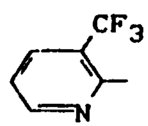 | H | H | $OCH_3$ | N | CH | $C-OCH_3$ | |
| 173 | 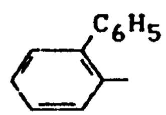 | H | H | $OCH_3$ | N | CH | $C-OCH_3$ | |
| 174 | 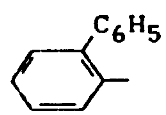 | H | H | $OCH_3$ | N | N | $C-OCH_3$ | |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 175 | 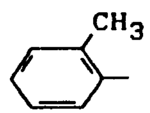 | H | H | $OCH_3$ | N | N | $C-OCH_3$ | |
| 176 | 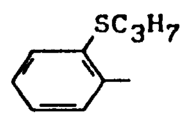 | H | H | $OCH_3$ | N | N | $C-OCH_3$ | |
| 177 | 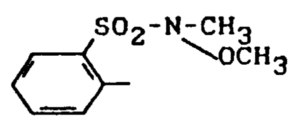 | H | H | $OCH_3$ | N | N | $C-OCH_3$ | |
| 178 | 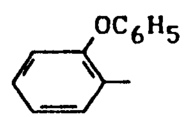 | H | H | $OCH_3$ | N | N | $C-OCH_3$ | |
| 179 | 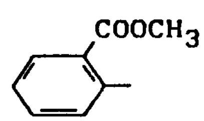 | H | $CH_3$ | $CH_3$ | N | CH | $C-CH_3$ | 233 |
| 180 | 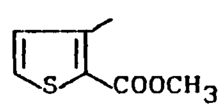 | H | $CH_3$ | $CH_3$ | N | CH | $C-CH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 181 | 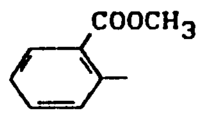 | H | $SCH_3$ | $CH_3$ | N | CH | $C-CH_3$ | |
| 182 | 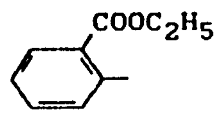 | H | $CH_3$ | $CH_3$ | N | CH | $C-CH_3$ | |
| 183 | 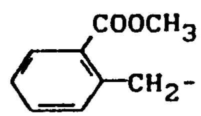 | H | $SCH_3$ | $OCH_3$ | N | CH | $C-OCH_3$ | |
| 184 | 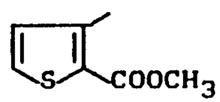 | H | $CH_3$ | $OCH_3$ | N | N | $C-OCH_3$ | |
| 185 | 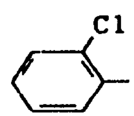 | H | $CH_3$ | $CH_3$ | N | N | $C-OCH_3$ | |
| 186 | 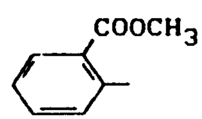 | H | $CH_3$ | $CH_3$ | N | N | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 187 | 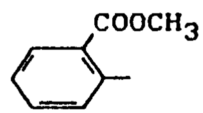 | H | $CH_3$ | $OCH_3$ | N | N | $C-OCH_3$ | 202 |
| 188 | 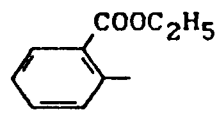 | H | $CH_3$ | $OCH_3$ | N | CH | C-Cl | |
| 189 | 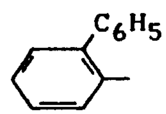 | H | $CH_3$ | $OCH_3$ | N | CH | $C-OCH_3$ | |
| 190 | 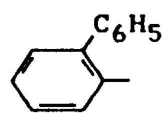 | H | $CH_3$ | $OCH_3$ | N | N | $C-OCH_3$ | |
| 191 | 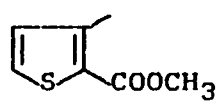 | H | $CH_3$ | $CH_3$ | N | N | $C-OCH_3$ | |
| 192 | 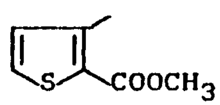 | 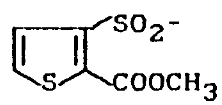 | H | $CH_3$ | N | CH | $C-CH_3$ | |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 193 | 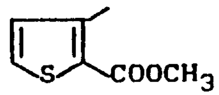 | H | H | $CH_3$ | N | CH | $C-CH_3$ | |
| 194 | 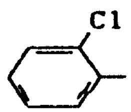 | H | $SCH_3$ | $CH_3$ | N | N | $C-OCH_3$ | |
| 195 | 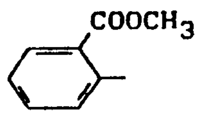 | H | $SCH_3$ | $CH_3$ | N | N | $C-OCH_3$ | |
| 196 | 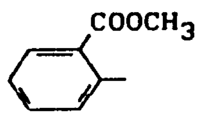 | H | $SCH_3$ | $OCH_3$ | N | N | $C-OCH_3$ | |
| 197 | 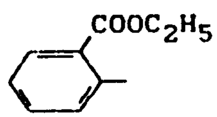 | H | H | $SO_2CH_3$ | CH | N | $C-OCH_3$ | |
| 198 | 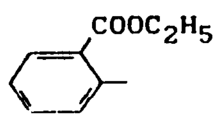 | H | $CH_3$ | $SO_2CH_3$ | CH | N | $C-OCH_3$ | |

**Tabelle 2** - Fortsetzung

| Bsp.-Nr. | $R^1$ | $R^2$ | $R^3$ | $R^4$ | X | Y | Z | Schmelz-punkt(°C) |
|---|---|---|---|---|---|---|---|---|
| 199 | 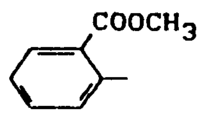 | 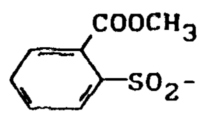 | $CH_3$ | $OCHF_2$ | CH | N | $C-OCHF_2$ | 200 |
| 200 | 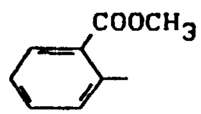 | 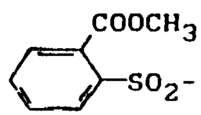 | $C_2H_5$ | $CH_3$ | N | N | $C-OCH_3$ | |
| 201 | 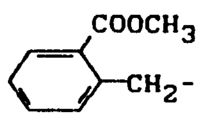 | H | $CH(CH_3)_2$ | $OCH_3$ | N | CH | $C-OCH_3$ | |
| 202 | 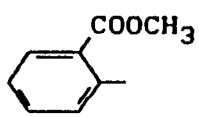 | H | $CH_2CH(CH_3)_2$ | $OCH_3$ | N | N | $C-OCH_3$ | |
| 203 | 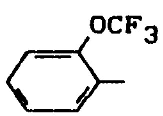 | H | $C(CH_3)_3$ | $CH_3$ | N | CH | $C-OCH_3$ | |
| 204 | 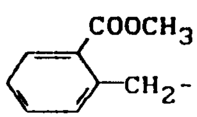 | H | $C(CH_3)_3$ | $OCH_3$ | N | CH | $C-OCH_3$ | |

**Tabelle 2** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 205 | 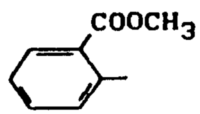 | H | $CF_3$ | $CH_3$ | N | N | $C-OCH_3$ | |
| 206 | 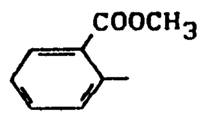 | H | $CH_3$ | $OCHF_2$ | N | CH | $C-OCHF_2$ | 118 |
| 207 | 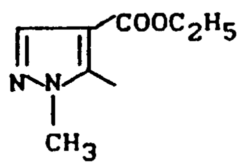 | H | $CH_3$ | $OCH_3$ | N | CH | $C-OCH_3$ | 198 |
| 208 | 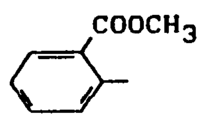 | H | $C_2H_5$ | $OCH_3$ | N | CH | $C-OCH_3$ | 174 |
| 209 | 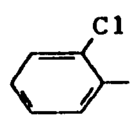 | 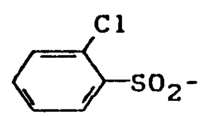 | $CH_3$ | $OCH_3$ | N | CH | $C-OCH_3$ | 204 |
| 210 | 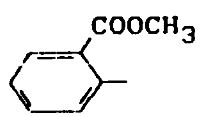 | H | $C_3H_7$ | $OCH_3$ | N | CH | $C-OCH_3$ | 179 |

<u>Tabelle 2</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 211 | 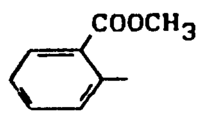 | 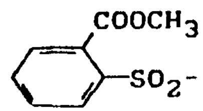 | $CH(CH_3)_2$ | $OCH_3$ | N | CH | $C-OCH_3$ | 184 |
| 212 | 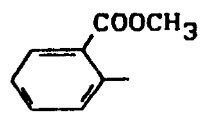 | 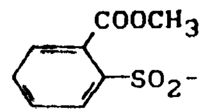 | $CH_3$ | $CH_3$ | N | CH | $C-OCH_3$ | |
| 213 | 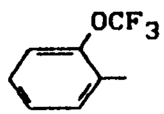 | 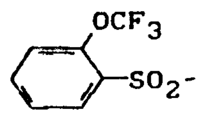 | $CH_3$ | $CH_3$ | N | CH | $C-OCH_3$ | |
| 214 | 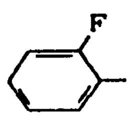 | 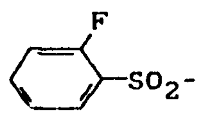 | $CH_3$ | $CH_3$ | N | CH | $C-OCH_3$ | |
| 215 | 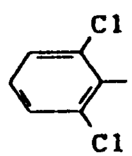 | 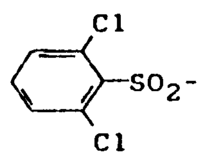 | $CH_3$ | $CH_3$ | N | CH | $C-OCH_3$ | |
| 216 | 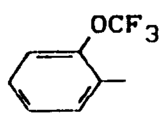 | H | $CH_3$ | $CH_3$ | N | CH | $C-OCH_3$ | |

**Tabelle 2** - Fortsetzung

| Bsp.-Nr. | $R^1$ | $R^2$ | $R^3$ | $R^4$ | X | Y | Z | Schmelz-punkt(°C) |
|---|---|---|---|---|---|---|---|---|
| 217 | 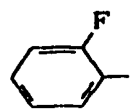 | H | $CH_3$ | $CH_3$ | N | CH | $C-OCH_3$ | 232 |
| 218 | 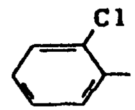 | H | $CH_3$ | $CH_3$ | N | CH | $C-OCH_3$ | 247 |
| 219 | 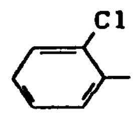 | 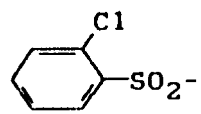 | $CH_3$ | $CH_3$ | N | CH | $C-OCH_3$ | 203 |
| 220 | 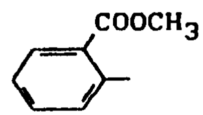 | H | $CH_3$ | $CH_3$ | N | CH | $C-OCH_3$ | |
| 221 | 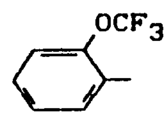 | H | $CH_3$ | $CH_3$ | N | CH | $C-OCH_3$ | 248 |
| 222 | 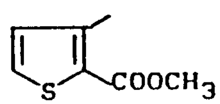 | 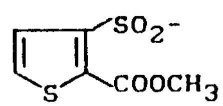 | $CH_3$ | $CH_3$ | N | CH | $C-CH_3$ | 206 |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 223 | 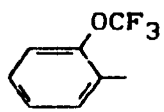 | H | $CH_3$ | $CH_3$ | N | CH | $C-CH_3$ | 308 |
| 224 | 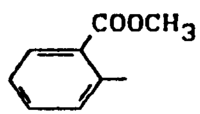 | 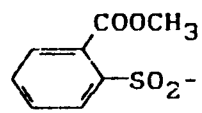 | $CH_3$ | $CH_3$ | N | CH | $C-CH_3$ | 195 |
| 225 | 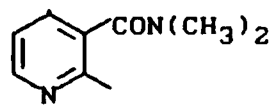 | H | H | $OCH_3$ | N | CH | $C-OCH_3$ | |
| 226 | 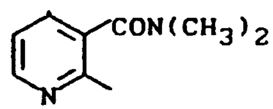 | H | $CH_3$ | $OCH_3$ | N | CH | $C-OCH_3$ | |
| 227 | 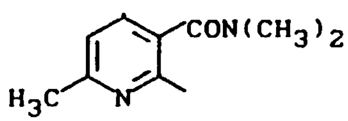 | H | $CH_3$ | $OCH_3$ | N | CH | $C-OCH_3$ | |

Tabelle 2 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 228 | 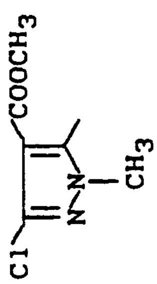 | H | $SCH_3$ | $OCH_3$ | N | CH | $C-OCH_3$ | |
| 229 | 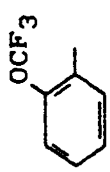 | H | $CH_3$ | $OCH_3$ | N | N | $C-OCH_3$ | 179 |
| 230 | 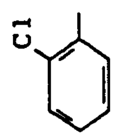 | H | $CH_3$ | $OCH_3$ | N | N | $C-OCH_3$ | (amorph) |
| 231 | 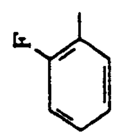 | H | $CH_3$ | $OCH_3$ | N | N | $C-OCH_3$ | 87 |

Nachstehend ist die Herstellung der in Tabelle 2 als Beispiel Nr. 221 aufgeführten Verbindung gemäß Verfahren (b) beispielhaft beschrieben:

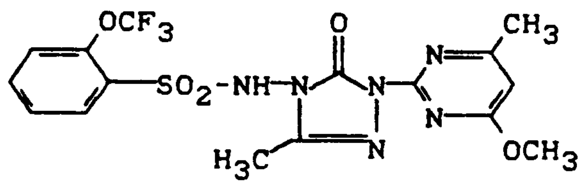

(Verfahren (b))

Eine Mischung aus 23,1 g (0,070 Mol) 4 – (2 – Trifluormethoxy – phenylsulfonylamino) – 5 – methyl – 2,4 – dihydro – 3H – 1,2,4 – triazol – 3 – on, 14,5 g (0,072 Mol) 4 – Methoxy – 6 – methyl – 2 – methylsulfonyl – pyrimidin, 50 g Kaliumcarbonat und 300 ml Dioxan wird 6 Stunden unter Rückfluß zum Sieden erhitzt. Nach Erkalten wird filtriert, der Filterrückstand mit Wasser gewaschen, dann in Methylenchlorid und 5%iger Salzsäure aufgenommen und geschüttelt. Die organische Phase wird mit Natriumsulfat getrocknet und filtriert. Nach Einengen des Filtrats wird der Rückstand durch Verreiben mit Ethanol zur Kristallisation gebracht und das kristalline Produkt wird durch Absaugen isoliert.

Man erhält 6,0 g (21 % der Theorie) 4 – (2 – Trifluormethoxy – phenylsulfonylamino) – 5 – methyl – 2 – (4 – methoxy – 6 – methylpyrimidin – 2 – yl) – 2,4 – dihydro – 3H – 1,2,4 – triazol – 3 – on vom Schmelzpunkt 248 ° C.

Ausgangsstoffe der Formel (II)

Beispiel (II – 1)

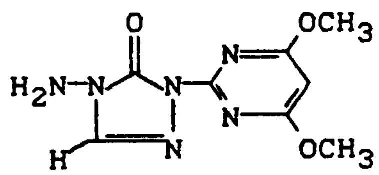

Eine Mischung aus 7,0 g (0,05 Mol) 4 – (1 – Methyl – ethylidenamino) – 2,4 – dihydro – 3H – 1,2,4 – triazol – 3 – on, 21 g (0,15 Mol) Kaliumcarbonat, 11,0 g (0,05 Mol) 4,6 – Dimethoxy – 2 – methylsulfonyl – pyrimidin und 100 ml Acetonitril wird 3 Stunden unter Rückfluß zum Sieden erhitzt. Nach dem Erkalten wird filtriert, das Filtrat eingeengt, der Rückstand in 100 ml Ethanol/Wasser (Vol. 1:1) aufgenommen und nach Zugabe von 1 ml konzentrierter Salzsäure 3 Stunden bei 60 ° C gerührt. Nach Einengen wird der Rückstand mit Ethanol verrieben und das kristallin angefallene Produkt durch Absaugen isoliert.

Man erhält 9,7 g (82 % der Theorie) 4 – Amino – 2 – (4,6 – dimethoxy – pyrimidin – 2 – yl) – 2,4 – dihydro – 3H – 1,2,4 – triazol – 3 – on vom Schmelzpunkt 238 ° C.

Beispiel (II – 2)

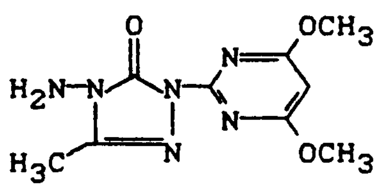

Eine Mischung aus 4,0 g (0,02 Mol) 4 – (1,3 – Dimethylbutylidenamino) – 5 – methyl – 2,4 – dihydro – 3H – 1,2,4 – triazol – 3 – on, 8,3 g (0,06 Mol) Kaliumcarbonat, 4,4 g (0,02 Mol) 4,6 – Dimethoxy – 2 – methylsulfonyl – pyrimidin und 100 ml Acetonitril wird 3 Stunden unter Rückfluß zum Sieden erhitzt. Nach

dem Erkalten wird filtriert, das Filtrat eingeengt, der Rückstand in 100 ml Ethanol/Wasser (Vol. 1:1) aufgenommen und nach Zugabe von 2 Tropfen konzentrierter Salzsäure 60 Minuten bei 60 ° C gerührt. Nach Einengen wird der Rückstand mit gesättigter Natriumhydrogencarbonat-Lösung verrührt und das kristalline Produkt durch Absaugen isoliert.

Man erhält 2,6 g (52 % der Theorie) 4-Amino-5-methyl-2-(4,6-dimethoxy-pyrimidin-2-yl)-2,4-dihydro-3H-1,2,4-triazol-3-on vom Schmelzpunkt 219 ° C.

Beispiel (II-3)

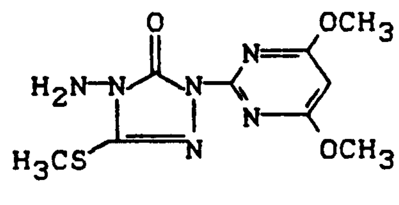

Eine Mischung aus 3,0 g (0,02 Mol) 4-Amino-5-methylthio-2,4-dihydro-3H-1,2,4-triazol-3-on und 200 ml Aceton wird nach Zugabe von 0,1 g p-Toluolsulfonsäure 4 Stunden unter Rückfluß zum Sieden erhitzt. Nach Einengen wird der Rückstand in 100 ml Acetonitril aufgenommen und nach Zugabe von 8,3 g (0,06 Mol) Kaliumcarbonat und 4,4 g (0,02 Mol) 4,6-Dimethoxy-2-methylsulfonyl-pyrimidin wird das Reaktionsgemisch 12 Stunden unter Rückfluß erhitzt. Nach Erkalten wird filtriert, das Filtrat eingeengt, der Rückstand in 100 ml Ethanol/Wasser (Vol. 1:1) aufgenommen und nach Zugabe von 2 ml konzentrierter Salzsäure 3 Stunden bei 60 ° C gerüht. nach Einengen wird der Rückstand mit gesättigter Natriumhydrogencarbonat-Lösung verrührt und das kristalline Produkt durch Absaugen isoliert.

Man erhält 4,9 g (86 % der Theorie) 4-Amino-5-methylthio-2-(4,6-dimethoxy-pyrimidin-2-yl)-2,4-dihydro-3H-1,2,4-triazol-3-on vom Schmelzpunkt 198 ° C.

Beispiel (II-4)

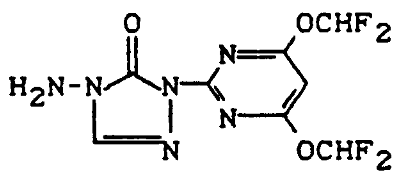

Eine Mischung aus 3,0 g (0,03 Mol) 4-Amino-2,4-dihydro-3H-1,2,4-triazol-3-on, 8,7 g (0,03 Mol) 2-Methansulfonyl-4,6-bis-difluormethoxy-pyrimidin und 12,4 g (0,09 Mol) Kaliumcarbonat in 100 ml Dioxan wird ca. 4 Stunden bei 60 ° C unter Rühren erhitzt. Es wird im Vakuum eingeengt, mit Wasser verrührt, der unlösliche Niederschlag abgesaugt und mit sehr verdünnter Salzsäure und Wasser neutral gewaschen. Nach dem Trocknen verbleiben 7,0 g (0,0226 Mol = 75 % der Theorie) 4-Amino-2-(4,6-bis-difluormethoxy)-pyrimidin-2-yl-2,4-dihydro-3H-1,2,4-triazol-3-on vom Schmelzpunkt 195 ° C.

Analog zu den Beispielen (II-1) bis (II-4) können die in der nachstehenden Tabelle 3 aufgeführten Verbindungen der Formel (II) hergestellt werden.

<u>Tabelle 3:</u> Herstellungsbeispiele für die Ausgangsstoffe der Formel (II)

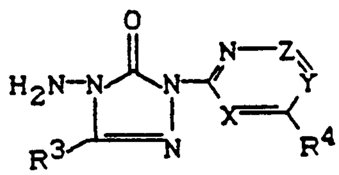

(II)

<u>T a b e l l e 3</u>

| Bsp.-Nr. | $R^3$ | $R^4$ | X | Y | Z | Schmelzpunkt (°C) |
|---|---|---|---|---|---|---|
| II-5 | $C(CH_3)_3$ | $OCH_3$ | N | CH | $C-OCH_3$ | 205 |
| II-6 | $CF_3$ | $OCH_3$ | N | CH | $C-OCH_3$ | 213 |
| II-7 | $C_2H_5$ | $OCH_3$ | N | CH | $C-OCH_3$ | 167 |
| II-8 | $C_3H_7$ | $OCH_3$ | N | CH | $C-OCH_3$ | 183 |
| II-9 | $CH(CH_3)_2$ | $OCH_3$ | N | CH | $C-OCH_3$ | 206 |
| II-10 | $CH_3$ | $CH_3$ | N | CH | $C-CH_3$ | 310 |
| II-11 | H | $OCH_3$ | N | CH | $C-NH-CH_3$ | |
| II-12 | H | $OCH_3$ | N | N | $C-OCH_3$ | |
| II-13 | $CH_3$ | $OCH_3$ | N | N | $C-CH_3$ | |
| II-14 | $CH_3$ | Cl | N | CH | $C-OCH_3$ | |
| II-15 | $CH_2$- 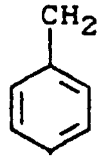 | $OCH_3$ | N | CH | $C-OCH_3$ | |
| II-16 | 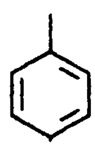 | $OCH_3$ | N | CH | $C-OCH_3$ | |
| II-17 | $CH_3$ | $OCHF_2$ | N | CH | $C-OCHF_2$ | 255 |

Ausgangsstoffe der Formel (IV)

Beispiel (IV-1)

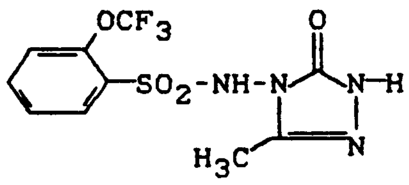

10,0 g (0,088 Mol) 4-Amino-5-methyl-2,4-dihydro-3H-1,2,4-triazol-3-on werden in 50 ml trockenem Pyridin gelöst, auf -10°C abgekühlt und mit 32,7 g (0,12 Mol) 2-Trifluormethoxy-benzolsulfonsäurechlorid versetzt. Man läßt das Reaktionsgemisch auf Raumtemperatur (ca. 20°C) kommen und rührt es ca. 12 Stunden. Nach Einengen wird der Rückstand in Methylenchlorid aufgenommen und mit 1N-Salzsäure und dann mit Wasser geschüttelt. Die organische Phase wird mit Natriumsulfat getrocknet und filtriert. Vom Filtrat wird das Lösungsmittel im Wasserstrahlvakuum sorgfältig abdestilliert.

Man erhält 23,1 g (80 % der Theorie) 4-(2-Trifluormethoxy-phenylsulfonylamino)-5-methyl-2,4-dihydro-3H-1,2,4-triazol-3-on als öligen Rückstand.

$^1$H-NMR*): (DMSO, δ, ppm): 2,10 (s, $CH_3$), 7,55, 7,83, 7,93 (m, 4H), ca. 11,5 (m, 2xNH).

Analog können die in der nachstehenden Tabelle 4 aufgeführten Verbindungen der Formel (IV) hergestellt werden.

*) Die $^1$H-HMR-Spektren wurden in Dimethylsulfoxid (DMSO) mit Tetramethylsilan (TMS) als innerem Standard aufgenommen. Angegeben ist die chemische Verschiebung als δ-Wert in ppm.)

Tabelle 4: Herstellungsbeispiele für die Ausgangsstoffe der Formel (IV)

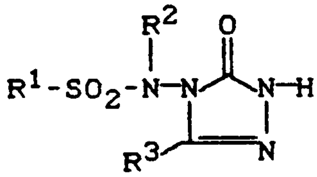 (IV)

| Bsp.-Nr. | $R^1$ | $R^2$ | $R^3$ | physikalische Daten |
|---|---|---|---|---|
| IV-2 | 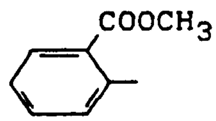 | H | $CH_3$ | NMR: 2,04[a)] |
| IV-3 | 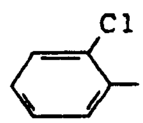 | H | $CH_3$ | NMR: 2,04[a)] |
| IV-4 | 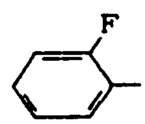 | H | $CH_3$ | NMR: 2,11[a)] |
| IV-5 | 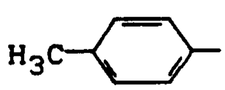 | 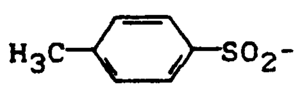 | $CH_3$ | Fp: 180-190° C |
| IV-6 | 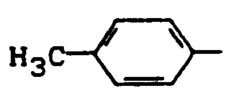 | H | $CH_3$ | Fp. 206-207° C |

a) $^1$H-NMR (DMSO, δ, ppm) für $R^3$ = $CH_3$

Ausgangsstoffe der Formel (XI)

Beispiel (XI - 1)

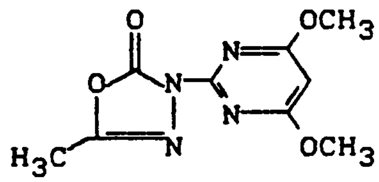

Eine Mischung aus 4,0 g (0,04 Mol) 5-Methyl-1,3,4-oxadiazol-2(3H)-on, 8,8 g (0,047 Mol) 4,6-Dimethoxy-2-methylsulfonyl-pyrimidin, 16,9 g (0,12 Mol) Kaliumcarbonat und 100 ml Acetonitril wird 4 Stunden bei 60 °C gerührt. Nach Erkalten wird im Wasserstrahlvakuum eingeengt, der Rückstand mit Wasser verrührt und das kristalline Produkt durch Absaugen isoliert.

Man erhält 4,8 g (50 % der Theorie) 3-(4,6-Dimethoxypyrimidin-2-yl)-5-methyl-1,3,4-oxadiazol-2(3H)-on vom Schmelzpunkt 158 °C.

Anwendungsbeispiele

In den folgenden Anwendungsbeispielen wird die nachstehend aufgeführte Verbindung als Vergleichssubstanz herangezogen:

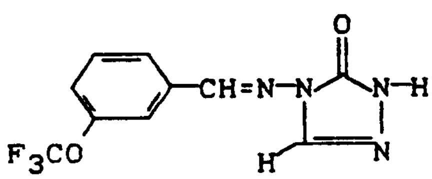

(A)

4-(3-Trifluormethoxy-benzylidenamino)-2,4-dihydro-3H-1,2,4-triazol-3-on
(bekannt aus US-PS 3 884 910).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen: (1), (2), (3), (4), (92), (208) und (210).

Beispiel B

Pre – emergence – Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoff – zubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispiele: (1), (2), (3), (4), (208) und (210).

**Patentansprüche**
**Patentansprüche für folgende Vertragsstaaten : BE, CH, DE, FR, GB, IT, LI, NL**

**1.** Substituierte Sulfonylaminotriazolinone der allgemeinen Formel (I)

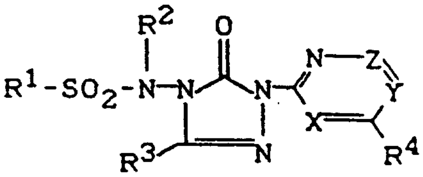

(I)

in welcher

$R^1$ für den Rest

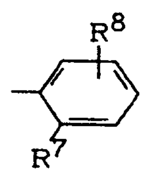

steht, worin

$R^7$ und $R^8$ gleich oder verschieden sind und für Wasserstoff, Fluor, Chlor, Brom, Iod, Cyano, Nitro, $C_1-C_6$ – Alkyl (welches gegebenenfalls durch Fluor, Chlor, Brom, Cyano, Carboxy, $C_1-C_4$ – Alkoxycarbonyl, $C_1-C_4$ – Alkylamino – carbonyl, Di – ($C_1-C_4$ – alkyl)amino – carbonyl, Hydroxy, $C_1-C_4$ – Alkoxy, Formyloxy, $C_1-C_4$ – Alkyl – carbonyloxy, $C_1-C_4$ – Alkoxy – carbonyloxy, $C_1-C_4$ – Alkylamino – carbonyloxy, $C_1-C_4$ – Alkylthio, $C_1-C_4$ – Alkylsulfinyl, $C_1-C_4$ – Alkylsulfonyl, Di – ($C_1-C_4$ – alkyl) – aminosulfonyl, $C_3-C_6$ – Cycloalkyl oder Phenyl substituiert ist], für $C_2-C_6$ – Alkenyl [welches gegebenenfalls durch Fluor, Chlor, Brom, Cyano, $C_1-C_4$ – Alkoxy – carbonyl, Carboxy oder Phenyl substituiert ist], für $C_2-C_6$ – Alkinyl [welches gegebenenfalls durch Fluor, Chlor, Brom, Cyano, $C_1-C_4$ – Alkoxy – carbonyl, Carboxy oder Phenyl substituiert ist], für $C_1-C_4$ – Alkoxy [welches gegebenenfalls durch Fluor, Chlor, Brom, Cyano, Carboxy, $C_1-C_4$ – Alkoxy – carbonyl, $C_1-C_4$ – Alkoxy, $C_1-C_4$ – Alkylthio, $C_1-C_4$ – Alkylsulfinyl oder $C_1-C_4$ – Alkylsulfonyl substituiert ist], für $C_1-C_4$ – Alkylthio [welches ge – gebenenfalls durch Fluor, Chlor, Brom, Cyano, Carboxy, $C_1-C_4$ – Alkoxy –

carbonyl, $C_1-C_4$-Alkylthio, $C_1-C_4$-Alkylsulfinyl oder $C_1-C_4$-Alkylsulfonyl substituiert ist], für $C_3-C_6$-Alkenyloxy [welches gegebenenfalls durch Fluor, Chlor, Brom, Cyano oder $C_1-C_4$-Alkoxy-carbonyl substituiert ist], für $C_2-C_6$-Alkenylthio [welches gegebenenfalls durch Fluor, Chlor, Brom, Cyano, Nitro, $C_1-C_3$-Alkylthio oder $C_1-C_4$-Alkoxy-carbonyl substituiert ist], $C_3-C_6$-Alkinyloxy, $C_3-C_6$-Alkinylthio oder für den Rest $-S(O)_p-R^9$ stehen, wobei

p für die Zahlen 1 oder 2 seht und

$R^9$ für Fluor, $C_1-C_4$-Alkyl [welches gegebenenfalls durch Fluor, Chlor, Brom, Cyano oder $C_1-C_4$-Alkoxy-carbonyl substituiert ist], $C_3-C_6$-Alkenyl, $C_3-C_6$-Alkinyl, $C_1-C_4$-Alkoxy, $C_1-C_4$-Alkoxy-$C_1-C_4$-alkylamino, $C_1-C_4$-Alkylamino, Di-($C_1-C_4$-alkyl)-amino oder fur den Rest $-NHOR^{10}$ steht, wobei

$R^{10}$ für $C_1-C_6$-Alkyl [welches gegebenenfalls durch Fluor, Chlor, Cyano, $C_1-C_4$-Alkoxy, $C_1-C_4$-Alkylthio, $C_1-C_4$-Alkylsulfinyl, $C_1-C_4$-Alkylsulfonyl, $C_1-C_4$-Alkyl-carbonyl, $C_1-C_4$-Alkoxy-carbonyl, $C_1-C_4$-Alkylamino-carbonyl oder Di-($C_1-C_4$-alkyl)-amino-carbonyl substituiert ist], für $C_3-C_6$-Alkenyl [welches gegebenenfalls durch Fluor, Chlor oder Brom substituiert ist), $C_3-C_6$-Alkinyl, $C_3-C_6$-Cycloalkyl, $C_3-C_6$-Cycloalkyl-$C_1-C_2$-alkyl, Phenyl-$C_1-C_2$-alkyl [welches gegebenenfalls durch Fluor, Chlor, Nitro, Cyano, $C_1-C_4$-Alkyl, $C_1-C_4$-Alkoxy oder $C_1-C_4$-Alkoxy-carbonyl substituiert ist], für Benzhydryl oder für Phenyl [welches gegebenenfalls durch Fluor, Chlor, Nitro, Cyano, $C_1-C_4$-Alkyl, Trifluormethyl, $C_1-C_4$-Alkoxy, $C_1-C_2$-Fluoralkoxy, $C_1-C_4$-Alkylthio, Trifluormethylthio oder $C_1-C_4$-Alkoxy-carbonyl substituiert ist] steht,

$R^7$ und/oder $R^8$ weiterhin für Phenyl oder Phenoxy, für $C_1-C_4$-Alkylcarbonylamino, $C_1-C_4$-Alkoxy-carbonylamino, $C_1-C_4$-Alkylamino-carbonyl-amino, Di-($C_1-C_4$-alkyl)-amino-carbonylamino, oder für den Rest $-CO-R^{11}$ stehen, wobei

$R^{11}$ für $C_1-C_6$-Alkyl, $C_1-C_6$-Alkoxy, $C_3-C_6$-Cycloalkoxy, $C_3-C_6$-Alkenyloxy, $C_1-C_4$-Alkylthio, $C_1-C_4$-Alkylamino, $C_1-C_4$-Alkoxyamino, $C_1-C_4$-Alkoxy-$C_1-C_4$-alkyl-amino oder Di-($C_1-C_4$-alkyl)-amino steht (welche gegebenenfalls durch Fluor und/oder Chlor substituiert sind],

$R^7$ und/oder $R^8$ weiterhin für $C_1-C_4$-Alkylsulfonyloxy, Di-($C_1-C_4$-alkyl)-aminosulfonylamino oder für den Rest $-CH=N-R^{12}$ stehen, wobei

$R^{12}$ für gegebenenfalls durch Fluor, Chlor, Cyano, Carboxy, $C_1-C_4$-Alkoxy, $C_1-C_4$-Alkylthio, $C_1-C_4$-Alkylsulfinyl oder $C_1-C_4$-Alkylsulfonyl substituiertes $C_1-C_6$-Alkyl, für gegebenenfalls durch Fluor oder Chlor substituiertes Benzyl, für gegebenenfalls durch Fluor oder Chlor substituiertes $C_3-C_6$-Alkenyl oder $C_3-C_6$-Alkinyl, für gegebenenfalls durch Fluor, Chlor, Brom, $C_1-C_4$-Alkyl, $C_1-C_4$-Alkoxy, Trifluormethyl, Trifluormethoxy oder Trifluormethylthio substituiertes Phenyl, für gegebenenfalls durch Fluor und/ oder Chlor substituiertes $C_1-C_6$-Alkoxy, $C_3-C_6$-Alkenoxy, $C_3-C_6$-Alkinoxy oder Benzyloxy für Amino, $C_1-C_4$-Alkylamino, Di-($C_1-C_4$-alkyl)-amino, Phenylamino, $C_1-C_4$-Alkyl-carbonyl-amino, $C_1-C_4$-Alkoxy-carbonyl-amino, $C_1-C_4$-Alkyl-sulfonylamino oder für gegebenenfalls durch Fluor, Chlor, Brom oder Methyl substituiertes Phenylsulfonylamino steht,

worin weiter

$R^1$ für den Rest

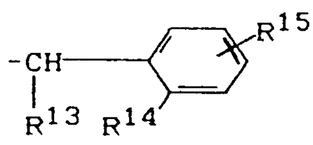

steht, worin

$R^{13}$ für Wasserstoff oder $C_1-C_4$-Alkyl steht,

$R^{14}$ und $R^{15}$ gleich oder verschieden sind und für Wasserstoff, Fluor, Chlor, Brom, Nitro, Cyano,

$C_1-C_4$-Alkyl [welches gegebenenfalls durch Fluor und/oder Chlor substituiert ist], $C_1-C_4$-Alkoxy [welches gegebenenfalls durch Fluor und/oder Chlor substituiert ist], Carboxy, $C_1-C_4$-Alkoxy-carbonyl, $C_1-C_4$-Alkylsulfonyl oder Di-($C_1-C_4$-alkyl)-aminosulfonyl stehen; worin weiter

$R^1$ für den Rest

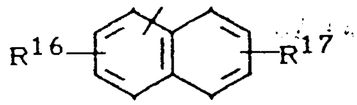

steht, worin

$R^{16}$ und $R^{17}$ gleich oder verschieden sind und für Wasserstoff, Fluor, Chlor, Brom, Nitro, Cyano, $C_1-C_4$-Alkyl [welches gegebenenfalls durch Fluor und/oder Chlor substituiert ist] oder $C_1-C_4$-Alkoxy [welches gegebenenfalls durch Fluor und/oder Chlor substituiert ist], stehen; worin weiter

$R^1$ für den Rest

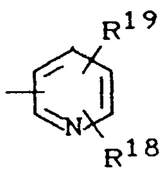

steht, worin

$R^{18}$ und $R^{19}$ gleich oder verschieden sind und für Wasserstoff, Fluor, Chlor, Brom, Nitro, Cyano, $C_1-C_4$-Alkyl [welches gegebenenfalls durch Fluor und/oder Chlor substituiert ist], $C_2-C_4$-Alkenyl [welches gegebenenfalls durch Fluor und/oder Chlor substituiert ist], $C_1-C_4$-Alkoxy [welches gegebenenfalls durch Fluor und/ oder Chlor substituiert ist], für $C_1$ $C_4$-Alkylthio, $C_1-C_4$-Alkylsulfinyl oder $C_1-C_4$-Alkylsulfonyl [welche gegebenenfalls durch Fluor und/oder Chlor substituiert sind], sowie für Di-($C_1-C_4$-alkyl)-aminosulfonyl, $C_1-C_4$-Alkoxy-carbonyl, Dimethylamino-carbonyl oder Dioxolanyl stehen; worin weiter

$R^1$ für den Rest

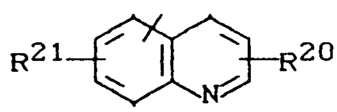

steht, worin

$R^{20}$ und $R^{21}$ gleich oder verschieden sind und für Wasserstoff, Fluor, Chlor, Brom, $C_1-C_4$-Alkyl [welches gegebenenfalls durch Fluor und/oder Brom substituiert ist], $C_1-C_4$-Alkoxy [welches gegebenenfalls durch Fluor und/oder Chlor substituiert ist], für $C_1-C_4$-Alkylthio, $C_1-C_4$-Alkylsulfinyl oder $C_1-C_4$-Alkylsulfonyl [welche gegebenenfalls durch Fluor und/oder Chlor substituiert sind], oder für Di-($C_1-C_4$-alkyl)-aminosulfonyl stehen; worin weiter

$R^1$ für den Rest

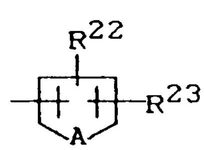

steht, worin

$R^{22}$ und $R^{23}$ gleich oder verschieden sind und für Wasserstoff, Fluor, Chlor, Brom, Cyano, Nitro, $C_1-C_4$-Alkyl [welches gegebenenfalls durch Fluor, Chlor, $C_1-C_4$-Alkoxy und/oder $C_1-C_4$-Halogenalkoxy substituiert ist], $C_1-C_4$-Alkoxy [welches gegebenenfalls durch Fluor und/oder Chlor substituiert ist], $C_1-C_4$-Alkylthio, $C_1-C_4$-Alkylsulfinyl oder $C_1-C_4$-Alkylsulfonyl [welches gegebenenfalls durch Fluor und/oder Chlor substituiert ist], Di-($C_1-C_4$-alkyl)-amino-sulfonyl oder $C_1-C_4$-Alkoxy-carbonyl stehen, und

A für Sauerstoff, Schwefel oder die Gruppierung $N-Z^1$ steht, wobei

$Z^1$ für Wasserstoff, $C_1-C_4$-Alkyl [welches gegebenenfalls durch Fluor, Chlor, Brom oder Cyano substituiert ist], $C_3-C_6$-Cycloalkyl, Benzyl, Phenyl [welches gegebenenfalls durch Fluor, Chlor, Brom oder Nitro substituiert ist], $C_1-C_4$-Alkylcarbonyl, $C_1-C_4$-Alkoxy-carbonyl oder Di-($C_1-C_4$-alkyl)-aminocarbonyl steht; worin weiter

$R^1$ für den Rest

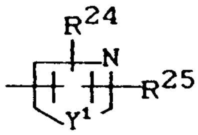

steht, worin

$R^{24}$ und $R^{25}$ gleich oder verschieden sind und für Wasserstoff, $C_1-C_4$-Alkyl, Halogen, $C_1-C_4$-Alkoxycarbonyl, $C_1-C_4$-Alkoxy oder $C_1-C_4$-Halogenalkoxy stehen,

$Y^1$ für Schwefel oder die Gruppierung $N-R^{26}$ steht, wobei

$R^{26}$ für Wasserstoff oder $C_1-C_4$-Alkyl steht; worin weiter

$R^1$ für den Rest

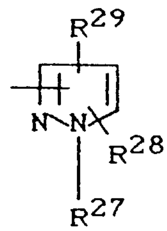

steht, worin

$R^{27}$ für Wasserstoff, $C_1-C_4$-Alkyl, Benzyl, (Iso)Chinolinyl oder Phenyl steht,

$R^{28}$ für Wasserstoff, Halogen, Cyano, Nitro, $C_1-C_4$-Alkyl [welches gegebenenfalls durch Fluor und/oder Chlor substituiert ist], $C_1-C_4$-Alkoxy [welches gegebenenfalls durch Fluor und/oder Chlor substituiert ist], Dioxolanyl oder $C_1-C_4$-Alkoxy-carbonyl steht und

$R^{29}$ für Wasserstoff, Halogen oder $C_1-C_4$-Alkyl steht; worin weiter

$R^1$ für den Rest

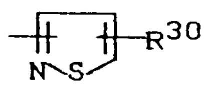

steht, worin

$R^{30}$ für Wasserstoff, Halogen, $C_1-C_4$-Alkyl, $C_1-C_4$-Halogenalkyl, $C_1-C_4$-Alkoxy, $C_1-C_4$-Halogenalkoxy oder $C_1-C_4$-Alkoxy-carbonyl steht;
worin weiter

$R^1$ für den Rest

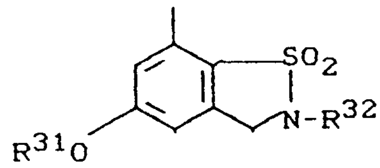

steht, worin

$R^{31}$ für $C_1-C_4$-Alkyl steht und

$R^{32}$ für $C_1-C_4$-Alkyl steht; worin weiter

$R^1$ für den Rest

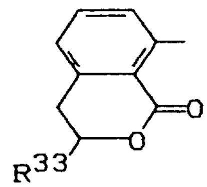

steht, worin

$R^{33}$ für Wasserstoff oder Methyl steht

in welcher weiter

$R^2$ für Wasserstoff oder die Gruppierung $-SO_2-R^1$ steht, worin

$R^1$ die oben vorzugsweise angegebene Bedeutung hat;

in welcher weiter

$R^3$ für Wasserstoff, Fluor, Chlor, Brom, Iod, Hydroxy, Mercapto, Amino oder einen gegebenenfalls durch Fluor und/oder Chlor substituierten Rest aus der Reihe $C_1-C_4$-Alkyl, $C_3-C_6$-Cycloalkyl, Benzyl, Phenyl, $C_1-C_4$-Alkoxy, $C_3-C_4$-Alkenyloxy, $C_3-C_4$-Alkinyloxy, $C_1-C_4$-Alkylthio, $C_1-C_4$-Alkylsulfinyl, $C_1-C_4$-Alkylsulfonyl, $C_3-C_4$-Alkenylthio, $C_3-C_4$-Alkinylthio, Benzyloxy, Benzylthio, $C_1-C_4$-Alkylamino und Di-($C_1-C_4$-alkyl)-amino steht,

$R^4$ für Wasserstoff, Fluor, Chlor, Brom, Hydroxy, Amino, $C_1-C_4$-Alkyl, $C_1-C_4$-Halogenalkyl, $C_1-C_2$-Alkoxy-$C_1-C_2$-alkyl, $C_1-C_4$-Alkoxy, $C_1-C_4$-Halogenalkoxy, $C_1-C_2$-Alkoxy-$C_1-C_2$-alkoxy, $C_1-C_4$-Alkylthio, $C_1-C_4$-Halogenalkylthio, $C_1-C_4$-Alkylsulfinyl, $C_1-C_4$-Alkylsulfonyl, $C_1-C_4$-Alkylamino oder Di-($C_1-C_2$-alkyl)-amino steht,

X für Stickstoff oder eine CH-Gruppierung steht,

Y für Stickstoff oder eine $CR^5$-Gruppierung steht, worin

$R^5$ für Wasserstoff, Fluor, Chlor, Brom, Cyano, Methyl, Formyl, Acetyl, Methoxycarbonyl oder Ethoxycarbonyl steht und

Z für Stickstoff oder eine $CR^6$-Gruppierung steht, worin

$R^6$ für Wasserstoff, Fluor, Chlor, Brom, $C_1-C_4$-Alkyl, $C_1-C_4$-Alkoxy, $C_1-C_4$-Halogenalkoxy, $C_1-C_4$-Alkylthio, $C_1-C_4$-Alkylsulfinyl, $C_1-C_4$-Alkylsulfonyl, $C_1-C_4$-Alkylamino, Dimethylamino oder Diethylamino steht,

sowie Salze von Verbindungen der Formel (I).

**2.** Substituierte Sulfonylaminotriazolinone der allgemeinen Formel (I) gemäß Anspruch 1, dadurch gekennzeichnet, daß darin

$R^1$ für den Rest

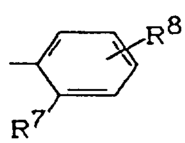

steht worin

$R^7$ für Fluor, Chlor, Brom, Methyl, Trifluormethyl, Methoxy, Difluormethoxy, Trifluormethoxy,

$C_1-C_3$-Alkylthio, $C_1-C_3$-Alkylsulfinyl, $C_1-C_3$-Alkylsulfonyl, Dimethylaminosulfonyl, N-Methoxy-N-methylaminosulfonyl, Phenyl, Phenoxy oder $C_1-C_3$-Alkoxy-carbonyl steht und

$R^8$ für Wasserstoff, Fluor oder Chlor steht; worin weiter

$R^1$ für den Rest

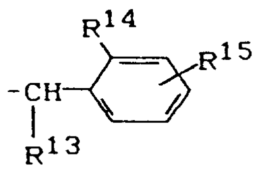

steht, worin

$R^{13}$ für Wasserstoff steht,

$R^{14}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{15}$ für Wasserstoff steht; worin weiter

$R^1$ für den Rest

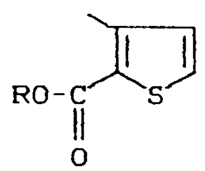

steht, worin

R für $C_1-C_4$-Alkyl steht, oder

$R^1$ für den Rest

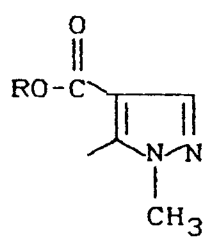

steht, worin

R für $C_1-C_4$-Alkyl steht, oder

$R^1$ für den Rest

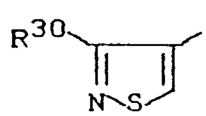

steht, worin

$R^{30}$ für Wasserstoff, Chlor, Methyl, Ethyl, Methoxycarbonyl oder Ethoxycarbonyl steht;

in welcher weiter

$R^2$ für Wasserstoff oder für die Gruppierung $-SO_2-R^1$ steht, worin

$R^1$ die oben als insbesondere bevorzugt angegebene Bedeutung hat,

$R^3$ für Wasserstoff, Methyl, Ethyl, Propyl, Isopropyl, Trifluormethyl, Cyclopropyl, Benzyl, Phenyl, t-Butyl, s-Butyl, i-Butyl, n-Butyl, Methoxy oder Methylthio steht,

$R^4$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff oder eine CH-Gruppierung steht,
Y für Stickstoff oder eine $CR^5$-Gruppierung steht, worin
$R^5$ für Wasserstoff, Fluor, Chlor oder Methyl steht, und
Z für Stickstoff oder eine $CR^6$-Gruppierung steht, worin
$R^6$ für Wasserstoff, Fluor, Chlor, Brom, Methyl, Ethyl, Methoxy, Ethoxy, Propoxy, Isopropoxy, Difluormethoxy, Methylthio, Ethylthio, Methylamino, Ethylamino, Dimethylamino oder Diethylamino steht,

sowie Salze von Verbindungen der Formel (I).

**3.** Verfahren zur Herstellung von substituierten Sulfonylaminotriazolinonen der allgemeinen Formel (I) gemäß Anspruch 1 und deren Salzen, dadurch gekennzeichnet, daß man

(a) substituierte Aminotriazolinone der allgemeinen Formel (II)

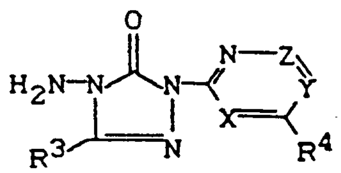

(II)

in welcher
$R^3$, $R^4$, X, Y und Z die in Anspruch 1 angegebene Bedeutung haben,
mit Sulfonsäurehalogeniden oder Sulfonsäureanhydriden der allgemeinen Formel (III)

$R^1-SO_2-Q$ (III)

in welcher
$R^1$ die in Anspruch 1 angegebene Bedeutung hat und
Q für Fluor, Chlor, Brom oder die Gruppierung $-O-SO_2-R^1$ steht, worin
$R^1$ die oben angegebene Bedeutung hat,

gegebenenfalls in Gegenwart eines Säureakzeptors und gegebenenfalls in Gegenwart eines Verdünnungsmittels umsetzt,
gegebenenfalls die so erhältlichen Verbindungen der Formel (I), in welcher
$R^2$ für die Gruppierung $-SO_2-R^1$ steht,
mit Desulfonylierungsmitteln,
gegebenenfalls in Gegenwart von Verdünnungsmitteln, zu Verbindungen der Formel (I), in welcher
$R^2$ für Wasserstoff steht, umsetzt, oder

(b) Sulfonylaminotriazolinone der allgemeinen Formel (IV)

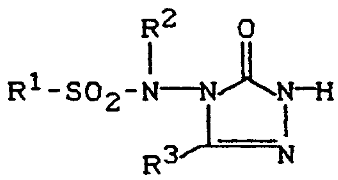

(IV)

in welcher
$R^1$, $R^2$ und $R^3$ die in Anspruch 1 angegebenen Bedeutungen haben,
mit Azinen der allgemeinen Formel (V)

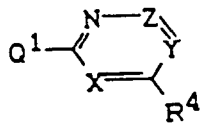

(V)

in welcher

$R^4$, X, Y und Z die in Anspruch 1 angegebenen Bedeutungen haben und

$Q^1$ für Halogen, Benzylsulfonyl oder Alkylsulfonyl steht,

gegebenenfalls in Gegenwart eines Säureakzeptors und gegebenenfalls in Gegenwart eines Verdünnungsmittels umsetzt und gegebenenfalls aus den so erhaltenen Verbindungen der Formel (I) nach üblichen Methoden Salze erzeugt.

**4.** Herbizide Mittel, gekennzeichnet durch einen Gehalt an mindestens einem substituierten Sulfonylaminotriazolinon der Formel (I) gemäß Anspruch 1.

**5.** Verfahren zur Bekämpfung von Unkräutern, dadurch gekennzeichnet, daß man substituierte Sulfonylaminotriazolinone der Formel (I) gemäß Anspruch 1 auf die Unkräuter und/oder ihren Lebensraum einwirken läßt.

**6.** Verwendung von substituierten Sulfonylaminotriazolinonen der Formel (I) gemäß Anspruch 1 zur Bekämpfung von Unkräutern.

**7.** Verfahren zur Herstellung von herbiziden Mitteln, dadurch gekennzeichnet, daß man substituierte Sulfonylaminotriazolinone der Formel (I) gemäß Anspruch 1 mit Streckmitteln und/oder oberflächenaktiven Mitteln vermischt.

## Patentansprüche für folgenden Vertragsstaat : ES

**1.** Verfahren zur Herstellung von substituierten Sulfonylaminotriazolinonen der allgemeinen Formel (I),

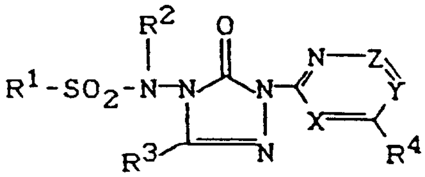

(I)

in welcher

$R^1$ für den Rest

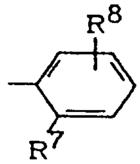

steht, worin

$R^7$ und $R^8$ gleich oder verschieden sind und für Wasserstoff, Fluor, Chlor, Brom, Iod, Cyano, Nitro, $C_1-C_6$-Alkyl (welches gegebenenfalls durch Fluor, Chlor, Brom, Cyano, Carboxy, $C_1-C_4$-Alkoxycarbonyl, $C_1-C_4$-Alkylamino-carbonyl, Di-($C_1-C_4$-alkyl)amino-carbonyl, Hydroxy, $C_1-C_4$-Alkoxy, Formyloxy, $C_1-C_4$-Alkyl-carbonyloxy, $C_1-C_4$-Alkoxy-carbonyloxy, $C_1-C_4$-Alkylamino-carbonyloxy, $C_1-C_4$-Alkylthio, $C_1-C_4$-Alkylsulfinyl, $C_1-C_4$-Alkylsulfonyl,

Di−($C_1$−$C_4$−alkyl)−aminosulfonyl, $C_3$−$C_6$−Cycloalkyl oder Phenyl substituiert ist], für $C_2$−$C_6$−Alkenyl [welches gegebenenfalls durch Fluor, Chlor, Brom, Cyano, $C_1$−$C_4$−Alkoxy−carbonyl, Carboxy oder Phenyl substituiert ist], für $C_2$−$C_6$−Alkinyl [welches gegebenenfalls durch Fluor, Chlor, Brom, Cyano, $C_1$−$C_4$−Alkoxy−carbonyl, Carboxy oder Phenyl substituiert ist], für $C_1$−$C_4$−Alkoxy [welches gegebenenfalls durch Fluor, Chlor, Brom, Cyano, Carboxy, $C_1$−$C_4$−Alkoxy−carbonyl, $C_1$−$C_4$−Alkoxy, $C_1$−$C_4$−Alkylthio, $C_1$−$C_4$−Alkylsulfinyl oder $C_1$−$C_4$−Alkylsulfonyl substituiert ist], für $C_1$−$C_4$−Alkylthio [welches gegebenenfalls durch Fluor, Chlor, Brom, Cyano, Carboxy, $C_1$−$C_4$−Alkoxy−carbonyl, $C_1$−$C_4$−Alkylthio, $C_1$−$C_4$−Alkylsulfinyl oder $C_1$−$C_4$−Alkylsulfonyl substituiert ist], für $C_3$−$C_6$−Alkenyloxy [welches gegebenenfalls durch Fluor, Chlor, Brom, Cyano oder $C_1$−$C_4$−Alkoxy−carbonyl substituiert ist], für $C_2$−$C_6$−Alkenylthio [welches gegebenenfalls durch Fluor, Chlor, Brom, Cyano, Nitro, $C_1$−$C_3$−Alkylthio oder $C_1$−$C_4$−Alkoxy−carbonyl substituiert ist], $C_3$−$C_6$−Alkinyloxy, $C_3$−$C_6$−Alkinylthio oder für den Rest $-S(O)_p-R^9$ stehen, wobei

p für die Zahlen 1 oder 2 steht und

$R^9$ für Fluor, $C_1$−$C_4$−Alkyl [welches gegebenenfalls durch Fluor, Chlor, Brom, Cyano oder $C_1$−$C_4$−Alkoxy−carbonyl substituiert ist], $C_3$−$C_6$−Alkenyl, $C_3$−$C_6$−Alkinyl, $C_1$−$C_4$−Alkoxy, $C_1$−$C_4$−Alkoxy−$C_1$−$C_4$−alkylamino, $C_1$−$C_4$−Alkylamino, Di−($C_1$−$C_4$−alkyl)−amino oder für den Rest $-NHOR^{10}$ steht, wobei

$R^{10}$ für $C_1$−$C_6$−Alkyl [welches gegebenenfalls durch Fluor, Chlor, Cyano, $C_1$−$C_4$−Alkoxy, $C_1$−$C_4$−Alkylthio, $C_1$−$C_4$−Alkylsulfinyl, $C_1$−$C_4$−Alkylsulfonyl, $C_1$−$C_4$−Alkyl−carbonyl, $C_1$−$C_4$−Alkoxy−carbonyl, $C_1$−$C_4$−Alkylamino−carbonyl oder Di−($C_1$−$C_4$−alkyl)−amino−carbonyl substituiert ist], für $C_3$−$C_6$−Alkenyl [welches gegebenenfalls durch Fluor, Chlor oder Brom substituiert ist], $C_3$−$C_6$−Alkinyl, $C_3$−$C_6$−Cycloalkyl, $C_3$−$C_6$−Cycloalkyl−$C_1$−$C_2$−alkyl, Phenyl−$C_1$−$C_2$−alkyl [welches gegebenenfalls durch Fluor, Chlor, Nitro, Cyano, $C_1$−$C_4$−Alkyl, $C_1$−$C_4$−Alkoxy oder $C_1$−$C_4$−Alkoxy−carbonyl substituiert ist], für Benzhydryl oder für Phenyl [welches gegebenenfalls durch Fluor, Chlor, Nitro, Cyano, $C_1$−$C_4$−Alkyl, Trifluormethyl, $C_1$−$C_4$−Alkoxy, $C_1$−$C_2$−Fluoralkoxy, $C_1$−$C_4$−Alkylthio, Trifluormethylthio oder $C_1$−$C_4$−Alkoxy−carbonyl substituiert ist] steht,

$R^7$ und/oder $R^8$ weiterhin für Phenyl oder Phenoxy, für $C_1$−$C_4$−Alkylcarbonylamino, $C_1$−$C_4$−Alkoxy−carbonylamino, $C_1$−$C_4$−Alkylamino−carbonyl−amino, Di−($C_1$−$C_4$−alkyl)−amino−carbonylamino, oder für den Rest $-CO-R^{11}$ stehen, wobei

$R^{11}$ für $C_1$−$C_6$−Alkyl, $C_1$−$C_6$−Alkoxy, $C_3$−$C_6$−Cycloalkoxy, $C_3$−$C_6$−Alkenyloxy, $C_1$−$C_4$−Alkylthio, $C_1$−$C_4$−Alkylamino, $C_1$−$C_4$−Alkoxyamino, $C_1$−$C_4$−Alkoxy−$C_1$−$C_4$−alkyl−amino oder Di−($C_1$−$C_4$−alkyl)−amino steht [welche gegebenenfalls durch Fluor und/oder Chlor substituiert sind],

$R^7$ und/oder $R^8$ weiterhin für $C_1$−$C_4$−Alkylsulfonyloxy, Di−($C_1$−$C_4$−alkyl)−aminosulfonylamino oder für den Rest $-CH=N-R^{12}$ stehen, wobei

$R^{12}$ für gegebenenfalls durch Fluor, Chlor, Cyano, Carboxy, $C_1$−$C_4$−Alkoxy, $C_1$−$C_4$−Alkylthio, $C_1$−$C_4$−Alkylsulfinyl oder $C_1$−$C_4$−Alkylsulfonyl substituiertes $C_1$−$C_6$−Alkyl, für gegebenenfalls durch Fluor oder Chlor substituiertes Benzyl, für gegebenenfalls durch Fluor oder Chlor substituiertes $C_3$−$C_6$−Alkenyl oder $C_3$−$C_6$−Alkinyl, für gegebenenfalls durch Fluor, Chlor, Brom, $C_1$−$C_4$−Alkyl, $C_1$−$C_4$−Alkoxy, Trifluormethyl, Trifluormethoxy oder Trifluormethylthio substituiertes Phenyl, für gegebenenfalls durch Fluor und/ oder Chlor substituiertes $C_1$−$C_6$−Alkoxy, $C_3$−$C_6$−Alkenoxy, $C_3$−$C_6$−Alkinoxy oder Benzyloxy für Amino, $C_1$−$C_4$−Alkylamino, Di−($C_1$−$C_4$−alkyl)−amino, Phenylamino, $C_1$−$C_4$−Alkyl−carbonyl−amino, $C_1$−$C_4$−Alkoxy−carbonyl−amino, $C_1$−$C_4$−Alkyl−sulfonylamino oder für gegebenenfalls durch Fluor, Chlor, Brom oder Methyl substituiertes Phenylsulfonylamino steht,

worin weiter

$R^1$ für den Rest

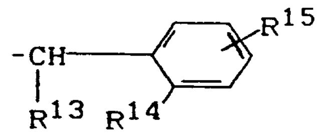

steht, worin

$R^{13}$ für Wasserstoff oder $C_1-C_4$-Alkyl steht,

$R^{14}$ und $R^{15}$ gleich oder verschieden sind und für Wasserstoff, Fluor, Chlor, Brom, Nitro, Cyano, $C_1-C_4$-Alkyl [welches gegebenenfalls durch Fluor und/oder Chlor substituiert ist], $C_1-C_4$-Alkoxy [welches gegebenenfalls durch Fluor und/oder Chlor substituiert ist], Carboxy, $C_1-C_4$-Alkoxy-carbonyl, $C_1-C_4$-Alkylsulfonyl oder Di-($C_1-C_4$-alkyl)-aminosulfonyl stehen; worin weiter

$R^1$ für den Rest

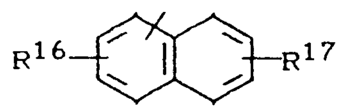

steht, worin

$R^{16}$ und $R^{17}$ gleich oder verschieden sind und für Wasserstoff, Fluor, Chlor, Brom,- Nitro, Cyano, $C_1-C_4$-Alkyl [welches gegebenenfalls durch Fluor und/oder Chlor substituiert ist] oder $C_1-C_4$-Alkoxy [welches gegebenenfalls durch Fluor und/oder Chlor substituiert ist], stehen; worin weiter

$R^1$ für den Rest

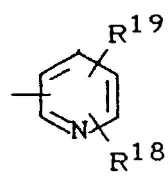

steht, worin

$R^{18}$ und $R^{19}$ gleich oder verschieden sind und für Wasserstoff, Fluor, Chlor, Brom, Nitro, Cyano, $C_1-C_4$-Alkyl [welches gegebenenfalls durch Fluor und/oder Chlor substituiert ist], $C_2-C_4$-Alkenyl [welches gegebenenfalls durch Fluor und/oder Chlor substituiert ist], $C_1-C_4$-Alkoxy [welches gegebenenfalls durch Fluor und/ oder Chlor substituiert ist], für $C_1$ $C_4$-Alkylthio, $C_1-C_4$-Alkylsulfinyl oder $C_1-C_4$-Alkylsulfonyl [welche gegebenenfalls durch Fluor und/oder Chlor substituiert sind], sowie für Di-($C_1-C_4$-alkyl)-aminosulfonyl, $C_1-C_4$-Alkoxy-carbonyl, Dimethylamino-carbonyl oder Dioxolanyl stehen; worin weiter

$R^1$ für den Rest

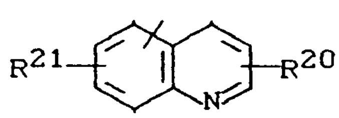

steht, worin

$R^{20}$ und $R^{21}$ gleich oder verschieden sind und für Wasserstoff, Fluor, Chlor, Brom, $C_1-C_4$-Alkyl [welches gegebenenfalls durch Fluor und/oder Brom substituiert ist], $C_1-C_4$-Alkoxy [welches gegebenenfalls durch Fluor und/oder Chlor substituiert ist], für $C_1-C_4$-Alkylthio, $C_1-C_4$-Alkylsulfinyl oder $C_1-C_4$-Alkylsulfonyl [welche gegebe-

nenfalls durch Fluor und/oder Chlor substituiert sind], oder für Di-($C_1$-$C_4$-alkyl)-aminosulfonyl stehen; worin weiter

$R^1$ für den Rest

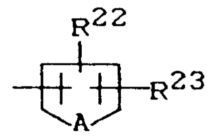

steht, worin

$R^{22}$ und $R^{23}$ gleich oder verschieden sind und für Wasserstoff, Fluor, Chlor, Brom, Cyano, Nitro, $C_1$-$C_4$-Alkyl [welches gegebenenfalls durch Fluor, Chlor, $C_1$-$C_4$-Alkoxy und/oder $C_1$-$C_4$-Halogenalkoxy substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-amino-sulfonyl oder $C_1$-$C_4$-Alkoxy-carbonyl stehen, und

A für Sauerstoff, Schwefel oder die Gruppierung N-$Z^1$ steht, wobei

$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; worin weiter

$R^1$ für den Rest

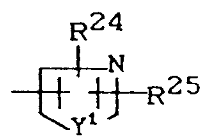

steht, worin

$R^{24}$ und $R^{25}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{26}$ steht, wobei

$R^{26}$ für Wasserstoff oder $C_1$-$C_4$-Alkyl steht; worin weiter

$R^1$ für den Rest

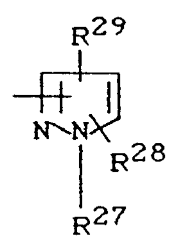

steht, worin

$R^{27}$ für Wasserstoff, $C_1$-$C_4$-Alkyl, Benzyl, (Iso)Chinolinyl oder Phenyl steht,

$R^{28}$ für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Dioxolanyl oder $C_1$-$C_4$-Alkoxy-carbonyl steht und

$R^{29}$ für Wasserstoff, Halogen oder $C_1$-$C_4$-Alkyl steht; worin weiter

$R^1$ für den Rest

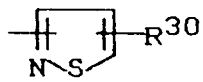

steht, worin

$R^{30}$ für Wasserstoff, Halogen, $C_1-C_4$-Alkyl, $C_1-C_4$-Halogenalkyl, $C_1-C_4$-Alkoxy, $C_1-C_4$-Halogenalkoxy oder $C_1-C_4$-Alkoxy-carbonyl steht; worin weiter

$R^1$ für den Rest

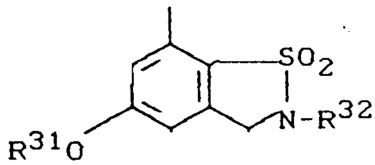

steht, worin

$R^{31}$ für $C_1-C_4$-Alkyl steht und

$R^{32}$ für $C_1-C_4$-Alkyl steht; worin weiter

$R^1$ für den Rest

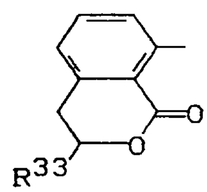

steht, worin

$R^{33}$ für Wasserstoff oder Methyl steht

in welcher weiter

$R^2$ für Wasserstoff oder die Gruppierung $-SO_2-R^1$ steht, worin

$R^1$ die oben vorzugsweise angegebene Bedeutung hat;

in welcher weiter

$R^3$ für Wasserstoff, Fluor, Chlor, Brom, Iod, Hydroxy, Mercapto, Amino oder einen gegebenenfalls durch Fluor und/oder Chlor substituierten Rest aus der Reihe $C_1-C_4$-Alkyl, $C_3-C_6$-Cycloalkyl, Benzyl, Phenyl, $C_1-C_4$-Alkoxy, $C_3-C_4$-Alkenyloxy, $C_3-C_4$-Alkinyloxy, $C_1-C_4$-Alkylthio, $C_1-C_4$-Alkylsulfinyl, $C_1-C_4$-Alkylsulfonyl, $C_3-C_4$-Alkenylthio, $C_3-C_4$-Alkinylthio, Benzyloxy, Benzylthio, $C_1-C_4$-Alkylamino und Di-($C_1-C_4$-alkyl)-amino steht,

$R^4$ für Wasserstoff, Fluor, Chlor, Brom, Hydroxy, Amino, $C_1-C_4$-Alkyl, $C_1-C_4$-Halogenalkyl, $C_1-C_2$-Alkoxy-$C_1-C_2$-alkyl, $C_1-C_4$-Alkoxy, $C_1-C_4$-Halogenalkoxy, $C_1-C_2$-Alkoxy-$C_1-C_2$-alkoxy, $C_1-C_4$-Alkylthio, $C_1-C_4$-Halogenalkylthio, $C_1-C_4$-Alkylsulfinyl, $C_1-C_4$-Alkylsulfonyl, $C_1-C_4$-Alkylamino oder Di-($C_1-C_2$-alkyl)-amino steht,

X für Stickstoff oder eine CH-Gruppierung steht,

Y für Stickstoff oder eine $CR^5$-Gruppierung steht, worin

$R^5$ für Wasserstoff, Fluor, Chlor, Brom, Cyano, Methyl, Formyl, Acetyl, Methoxycarbonyl oder Ethoxycarbonyl steht und

Z für Stickstoff oder eine $CR^6$-Gruppierung steht, worin

$R^6$ für Wasserstoff, Fluor, Chlor, Brom, $C_1-C_4$-Alkyl, $C_1-C_4$-Alkoxy, $C_1-C_4$-Halogenalkoxy, $C_1-C_4$-Alkylthio, $C_1-C_4$-Alkylsulfinyl, $C_1-C_4$-Alkylsulfonyl, $C_1-C_4$-Alkylamino, Dimethylamino oder Diethylamino steht,

sowie von Salzen der Verbindungen der Formel (I),

dadurch gekennzeichnet, daß man

(a) substituierte Aminotriazolinone der allgemeinen Formel (II)

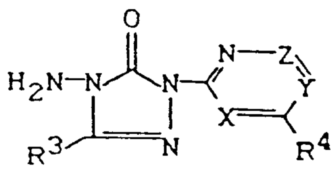

(II)

in welcher

$R^3$, $R^4$, X, Y und Z die oben (bei Formel I) angegebene Bedeutung haben,

mit Sulfonsäurehalogeniden oder Sulfonsäureanhydriden der allgemeinen Formel (III)

$R^1 - SO_2 - Q$ (III)

in welcher

$R^1$ die oben angegebene Bedeutung hat und

Q für Fluor, Chlor, Brom oder die Gruppierung $-O-SO_2-R^1$ steht, worin

$R^1$ die oben angegebene Bedeutung hat,

gegebenenfalls in Gegenwart eines Säureakzeptors und gegebenenfalls in Gegenwart eines Verdünnungsmittels umsetzt,

gegebenenfalls die so erhältlichen Verbindungen der Formel (I), in welcher

$R^2$ für die Gruppierung $-SO_2-R^1$ steht,

mit Desulfonylierungsmitteln,

gegebenenfalls in Gegenwart von Verdünnungsmitteln, zu Verbindungen der Formel (I), in welcher

$R^2$ für Wasserstoff steht, umsetzt, oder

(b) Sulfonylaminotriazolinone der allgemeinen Formel (IV)

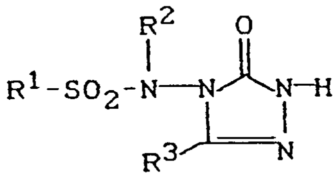

(IV)

in welcher

$R^1$, $R^2$ und $R^3$ die oben angegebenen Bedeutungen haben,

mit Azinen der allgemeinen Formel (V)

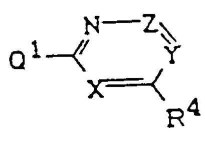

(V)

in welcher

$R^4$, X, Y und Z die oben angegebenen Bedeutungen haben und

$Q^1$ für Halogen, Benzylsulfonyl oder Alkylsulfonyl steht,

gegebenenfalls in Gegenwart eines Säureakzeptors und gegebenenfalls in Gegenwart eines Verdünnungsmittels umsetzt und gegebenenfalls aus den so erhaltenen Verbindungen der Formel (I) nach üblichen Methoden Salze erzeugt.

**2.** Verfahren zur Herstellung von substituierten Sulfonylamino triazolinonen der allgemeinen Formel (I) und von deren Salzen gemäß Anspruch 1, dadurch gekennzeichnet, daß in Formel (I)

$R^1$ für den Rest

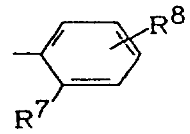

steht worin

$R^7$ für Fluor, Chlor, Brom, Methyl, Trifluormethyl, Methoxy, Difluormethoxy, Trifluormethoxy, $C_1-C_3$-Alkylthio, $C_1-C_3$-Alkylsulfinyl, $C_1-C_3$-Alkylsulfonyl, Dimethylaminosulfonyl, N-Methoxy-N-methylaminosulfonyl, Phenyl, Phenoxy oder $C_1-C_3$-Alkoxy-carbonyl steht und

$R^8$ für Wasserstoff, Fluor oder Chlor steht; worin weiter

$R^1$ für den Rest

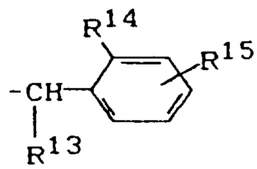

steht, worin

$R^{13}$ für Wasserstoff steht,

$R^{14}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{15}$ für Wasserstoff steht; worin weiter

$R^1$ für den Rest

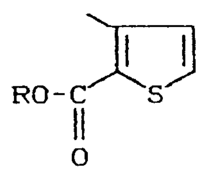

steht, worin

R für $C_1-C_4$-Alkyl steht, oder

$R^1$ für den Rest

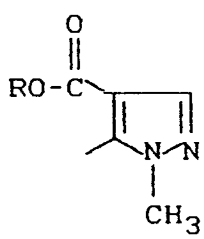

steht, worin

R für $C_1-C_4$-Alkyl steht, oder

$R^1$ für den Rest

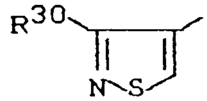

steht, worin

$R^{30}$ für Wasserstoff, Chlor, Methyl, Ethyl, Methoxycarbonyl oder Ethoxycarbonyl steht;

in welcher weiter

$R^2$ für Wasserstoff oder für die Gruppierung $-SO_2-R^1$ steht, worin

$R^1$ die oben als insbesondere bevorzugt angegebene Bedeutung hat,

$R^3$ für Wasserstoff, Methyl, Ethyl, Propyl, Isopropyl, Trifluormethyl, Cyclopropyl, Benzyl, Phenyl, t-Butyl, s-Butyl, i-Butyl, n-Butyl, Methoxy oder Methylthio steht,

$R^4$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine CH-Gruppierung steht,

Y für Stickstoff oder eine $CR^5$-Gruppierung steht, worin

$R^5$ für Wasserstoff, Fluor, Chlor oder Methyl steht, und

Z für Stickstoff oder eine $CR^6$-Gruppierung steht, worin

$R^6$ für Wasserstoff, Fluor, Chlor, Brom, Methyl, Ethyl, Methoxy, Ethoxy, Propoxy, Isopropoxy, Difluormethoxy, Methylthio, Ethylthio, Methylamino, Ethylamino, Dimethylamino oder Diethylamino steht.

3. Herbizide Mittel, gekennzeichnet durch einen Gehalt an mindestens einem substituierten Sulfonylamlnotriazolinon der Formel (I) gemäß Anspruch 1.

4. Verfahren zur Bekämpfung von Unkräutern, dadurch gekennzeichnet, daß man substituierte Sulfonylaminotriazolinone der Formel (I) gemäß Anspruch 1 auf die Unkräuter und/oder ihren Lebensraum einwirken läßt.

5. Verwendung von substituierten Sulfonylaminotriazolinonen der Formel (I) gemäß Anspruch 1 zur Bekämpfung von Unkräutern.

6. Verfahren zur Herstellung von herbiziden Mitteln, dadurch gekennzeichnet, daß man substituierte Sulfonylaminotriazolinone der Formel (I) gemäß Anspruch 1 mit Streckmitteln und/oder oberflächenaktiven Mitteln vermischt.

**Claims**

**Claims for the following Contracting States : BE, CH, DE, FR, GB, IT, LI, NL**

1. Substituted sulphonylaminotriazolinones of the general formula (I)

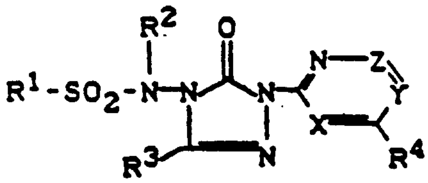

(I)

in which

$R^1$ represents the radical

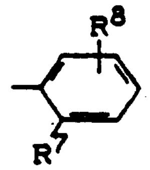

in which

$R^7$ and $R^8$ are identical or different and represent hydrogen, fluorine, chlorine, bromine, iodine, cyano, nitro, $C_1-C_6$-alkyl [which is optionally substituted by fluorine, chlorine, bromine, cyano, carboxyl, $C_1-C_4$-alkoxycarbonyl, $C_1-C_4$-alkylamino-carbonyl, di-($C_1-C_4$-alkyl)aminocarbonyl, hydroxyl, $C_1-C_4$-alkoxy, formyloxy, $C_1-C_4$-alkyl-carbonyloxy, $C_1-C_4$-alkoxy-carbonyloxy, $C_1-C_4$-alkylamino-carbonyloxy, $C_1-C_4$-alkylthio, $C_1-C_4$-alkylsulphinyl, $C_1-C_4$-alkylsulphonyl, di-($C_1-C_4$-alkyl)-aminosulphonyl, $C_3-C_6$-cycloalkyl or phenyl] or represent $C_2-C_6$-alkenyl [which is optionally substituted by fluorine, chlorine, bromine, cyano, $C_1-C_4$-alkoxy-carbonyl, carboxyl or phenyl], or represent $C_2-C_6$-alkinyl [which is optionally substituted by fluorine, chlorine, bromine, cyano, $C_1-C_4$-alkoxy-carbonyl, carboxyl or phenyl], or represent $C_1-C_4$-alkoxy [which is optionally substituted by fluorine, chlorine, bromine, cyano, carboxyl, $C_1-C_4$-alkoxy-carbonyl, $C_1-C_4$-alkoxy, $C_1-C_4$-alkylthio, $C_1-C_4$-alkylsulphinyl or $C_1-C_4$-alkylsulphonyl], or represent $C_1-C_4$-alkylthio [which is optionally substituted by fluorine, chlorine, bromine, cyano, carboxyl, $C_1-C_4$-alkoxy-carbonyl, C,$-C_4$-alkylthio, $C_1-C_4$-alkylsulphinyl or $C_1-C_4$-alkylsulphonyl], or represent $C_3-C_6$-alkenyloxy [which is optionally substituted by fluorine, chlorine, bromine, cyano or $C_1-C_4$-alkoxy-carbonyl], or represent $C_2-C_6$-alkenylthio [which is optionally substituted by fluorine, chlorine, bromine, cyano, nitro, $C_1-C_3$-alkylthio or $C_1-C_4$-alkoxy-carbonyl], $C_3-C_6$-alkinyloxy, $C_3-C_6$-alkinylthio or the radical $-S(O)_p-R^9$, in which

p represents the numbers 1 or 2 and

$R^9$ represents fluorine, $C_1-C_4$-alkyl [which is optionally substituted by fluorine, chlorine, bromine, cyano or $C_1-C_4$-alkoxy-carbonyl], $C_3-C_6$-alkenyl, $C_3-C_6$-alkinyl, $C_1-C_4$-alkoxy, $C_1-C_4$-alkoxy-$C_1-C_4$-alkylamino, $C_1-C_4$-alkylamino, di-($C_1-C_4$-alkyl)-amino or the radical $-NHOR^{10}$, in which

$R^{10}$ represents $C_1-C_6$-alkyl [which is optionally substituted by fluorine, chlorine, cyano, $C_1-C_4$-alkoxy, $C_1-C_4$-alkylthio, $C_1-C_4$-alkylsulphinyl, $C_1-C_4$-alkylsulphonyl, $C_1-C_4$-alkyl-carbonyl, $C_1-C_4$-alkoxy-carbonyl, $C_1-C_4$-alkylamino-carbonyl or di-($C_1-C_4$-alkyl)-amino-carbonyl], or represents $C_3-C_6$-alkenyl [which is optionally substituted by fluorine, chlorine or bromine], $C_3-C_6$-alkinyl, $C_3-C_6$-cycloalkyl, $C_3-C_6$-cycloalkyl-$C_1-C_2$-alkyl, phenyl-$C_1-C_2$-alkyl [which is optionally substituted by fluorine, chlorine, nitro, cyano, $C_1-C_4$-alkyl, $C_1-C_4$-alkoxy or $C_1-C_4$-alkoxy-carbonyl], or represents benzhydryl, or represents phenyl [which is optionally substituted by fluorine, chlorine, nitro, cyano, $C_1-C_4$-alkyl, trifluoromethyl, $C_1-C_4$-alkoxy, $C_1-C_2$-fluoroalkoxy, $C_1-C_4$-alkylthio, trifluoromethylthio or $C_1-C_4$-alkoxy-carbonyl],

$R^7$ and/or $R^8$ furthermore represent phenyl or phenoxy, or represent $C_1-C_4$-alkylcarbonylamino, $C_1-C_4$-alkoxy-carbonylamino, $C_1-C_4$-alkylaminocarbonyl-amino, di-($C_1-C_4$-alkyl)-aminocarbonylamino, or represent the radical $-CO-R^{11}$, in which

$R^{11}$ represents $C_1-C_6$-alkyl, $C_1-C_6$-alkoxy, $C_3-C_6$-cycloalkoxy, $C_3-C_6$-alkenyloxy, $C_1-C_4$-alkylthio, $C_1-C_4$-alkylamino, $C_1-C_4$-alkoxyamino, $C_1-C_4$-alkoxy-$C_1-C_4$-alkyl-amino or di-($C_1-C_4$-alkyl)-amino [each of which is optionally substituted by fluorine and/or chlorine],

$R^7$ and/or $R^8$ furthermore represent $C_1-C_4$-alkylsulphonyloxy, di-($C_1-C_4$-alkyl)-aminosulphonyl-amino or the radical $-CH=N-R^{12}$, in which

$R^{12}$ represents $C_1-C_6$-alkyl which is optionally substituted by fluorine, chlorine, cyano, carboxyl, $C_1-C_4$-alkoxy, $C_1-C_4$-alkylthio, $C_1-C_4$-alkylsulphinyl or C

$_1-C_4$-alkylsulphonyl, or represents benzyl which is optionally substituted by fluorine or chlorine, or represents $C_3-C_6$-alkenyl or $C_3-C_6$-alkinyl, each of which is optionally substituted by fluorine or chlorine, or represents phenyl which is optionally substituted by fluorine, chlorine, bromine, $C_1-C_4$-alkyl, $C_1-C_4$-alkoxy, trifluoromethyl, trifluoromethoxy or trifluoromethylthio, or represents $C_1-C_6$-alkoxy, $C_3-C_6$-alkenoxy, $C_3-C_6$-alkinoxy or benzyloxy, each of which is optionally substituted by fluorine and/or chlorine, or represents amino, $C_1-C_4$-alkylamino, di-($C_1-C_4$-alkyl)-amino, phenylamino, $C_1-C_4$-alkyl-carbonyl-amino, $C_1-C_4$-alkoxy-carbonylamino, $C_1-C_4$-alkylsulphonylamino, or represents phenylsulphonylamino which is optionally substituted by fluorine, chlorine, bromine or methyl,

in which furthermore

$R^1$ represents the radical

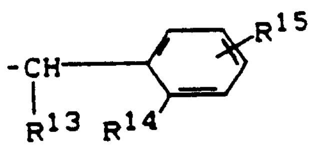

in which

$R^{13}$ represents hydrogen or $C_1-C_4$-alkyl,

$R^{14}$ and $R^{15}$ are identical or different and represent hydrogen, fluorine, chlorine, bromine, nitro, cyano, $C_1-C_4$-alkyl [which is optionally substituted by fluorine and/or chlorine], $C_1-C_4$-alkoxy [which is optionally substituted by fluorine and/or chlorine], carboxyl, $C_1-C_4$-alkoxy-carbonyl, $C_1-C_4$-alkylsulphonyl or di-($C_1-C_4$-alkyl)-aminosulphonyl; in which furthermore

$R^1$ represents the radical

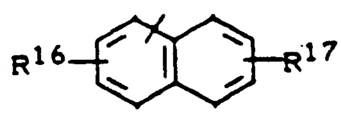

in which

$R^{16}$ and $R^{17}$ are identical or different and represent hydrogen, fluorine, chlorine, bromine, nitro, cyano, $C_1-C_4$-alkyl [which is optionally substituted by fluorine and/or chlorine] or $C_1-C_4$-alkoxy [which is optionally substituted by fluorine and/or chlorine]; in which furthermore

$R^1$ represents the radical

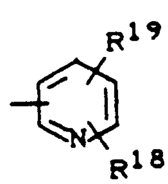

in which

$R^{18}$ and $R^{19}$ are identical or different and represent hydrogen, fluorine, chlorine, bromine, nitro, cyano, $C_1-C_4$-alkyl [which is optionally substituted by fluorine and/or chlorine], $C_2-C_4$-alkenyl [which is optionally substituted by fluorine and/or chlorine]; $C_1-C_4$-alkoxy [which is optionally substituted by fluorine and/or chlorine], or represent $C_1-C_4$-alkylthio, $C_1-C_4$-alkylsulphinyl or $C_1-C_4$-alkylsulphonyl [each of which is optionally substituted by fluorine and/or chlorine], or represent di-($C_1-C_4$-alkyl)-aminosulphonyl, $C_1-C_4$-alkoxy-carbonyl, dimethylamino-carbonyl or

dioxolanyl; in which furthermore

$R^1$ represents the radical

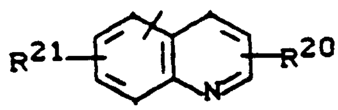

in which

$R^{20}$ and $R^{21}$ are identical or different and represent hydrogen, fluorine, chlorine, bromine, $C_1-C_4$-alkyl [which is optionally substituted by fluorine and/or bromine], $C_1-C_4$-alkoxy [which is optionally substituted by fluorine and/or chlorine], or represent $C_1-C_4$-alkylthio, $C_1-C_4$-alkylsulphinyl or $C_1-C_4$-alkylsulphonyl [each of which is optionally substituted by fluorine and/or chlorine], or represent di-($C_1-C_4$-alkyl)-aminosulphonyl, in which furthermore

$R^1$ represents the radical

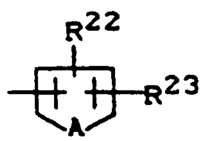

in which

$R^{22}$ and $R^{23}$ are identical or different and represent hydrogen, fluorine, chlorine, bromine, cyano, nitro, $C_1-C_4$-alkyl [which is optionally substituted by fluorine, chlorine, $C_1-C_4$-alkoxy and/or $C_1-C_4$-halogenoalkoxy], $C_1-C_4$-alkoxy [which is optionally substituted by fluorine and/or chlorine], $C_1-C_4$-alkylthio, $C_1-C_4$-alkylsulphinyl or $C_1-C_4$-alkylsulphonyl [which is optionally substituted by fluorine and/or chlorine], di-($C_1-C_4$-alkyl)-amino-sulphonyl or $C_1-C_4$-alkoxy-carbonyl, and

A represents oxygen, sulphur or the group $N-Z^1$, in which

$Z^1$ represents hydrogen, $C_1-C_4$-alkyl [which is optionally substituted by fluorine, chlorine, bromine or cyano], $C_3-C_6$-cycloalkyl, benzyl, phenyl [which is optionally substituted by fluorine, chlorine, bromine or nitro], $C_1-C_4$-alkylcarbonyl, $C_1-C_4$-alkoxy-carbonyl or di-($C_1-C_4$-alkyl)-amino-carbonyl; in which furthermore

$R^1$ represents the radical

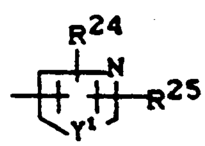

in which

$R^{24}$ and $R^{25}$ are identical or different and represent hydrogen, $C_1-C_4$-alkyl, halogen, $C_1-C_4$-alkoxycarbonyl, $C_1-C_4$-alkoxy or $C_1-C_4$-halogenoalkoxy,

$Y^1$ represents sulphur or the group $N-R^{26}$, in which

$R^{26}$ represents hydrogen or $C_1-C_4$-alkyl; in which furthermore

$R^1$ represents the radical

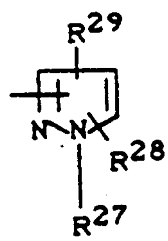

in which

$R^{27}$ represents hydrogen, $C_1-C_4$-alkyl, benzyl, (iso)-quinolinyl or phenyl,

$R^{28}$ represents hydrogen, halogen, cyano, nitro, $C_1-C_4$-alkyl [which is optionally substituted by fluorine and/or chlorine], $C_1-C_4$-alkoxy [which is optionally substituted by fluorine and/or chlorine], dioxolanyl or $C_1-C_4$-alkoxy-carbonyl and

$R^{29}$ represents hydrogen, halogen or $C_1-C_4$-alkyl; in which furthermore

$R^1$ represents the radical

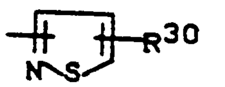

in which

$R^{30}$ represents hydrogen, halogen, $C_1-C_4$-alkyl, $C_1-C_4$-halogenoalkyl, $C_1-C_4$-alkoxy, $C_1-C_4$-halogenoalkoxy or $C_1-C_4$-alkoxy-carbonyl; in which furthermore

$R^1$ represents the radical

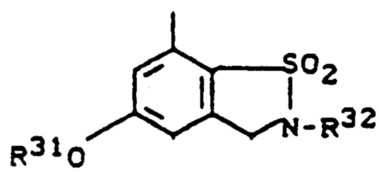

in which

$R^{31}$ represents $C_1-C_4$-alkyl and

$R^{32}$ represents $C_1-C_4$-alkyl; in which furthermore

$R^1$ represents the radical

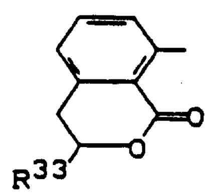

in which

$R^{33}$ represents hydrogen or methyl,

in which furthermore

$R^2$ represents hydrogen or the group $-SO_2-R^1$ in which

$R^1$ has the meaning mentioned above as being preferred;

in which furthermore

$R^3$ represents hydrogen, fluorine, chlorine, bromine, iodine, hydroxyl, mercapto, amino, or a radical which is selected from the series comprising $C_1-C_4$-alkyl, $C_3-C_6$-cycloalkyl, benzyl, phenyl, $C_1-C_4$-alkoxy, $C_3-C_4$-alkenyloxy, $C_3-C_4$-alkinyloxy, $C_1-C_4$-alkylthio,

$C_1-C_4$-alkylsulphinyl, $C_1-C_4$-alkylsulphonyl, $C_3-C_4$-alkenylthio, $C_3-C_4$-alkinylthio, benzyloxy, benzylthio, $C_1-C_4$-alkylamino and di-($C_1-C_4$-alkyl)-amino, and which is optionally substituted by fluorine and/or chlorine,

$R^4$ represents hydrogen, fluorine, chlorine, bromine, hydroxyl, amino, $C_1-C_4$-alkyl, $C_1-C_4$-halogenoalkyl, $C_1-C_2$-alkoxy-$C_1-C_2$-alkyl, $C_1-C_4$-alkoxy, $C_1-C_4$-halogenoalkoxy, $C_1-C_2$-alkoxy-$C_1-C_2$-alkoxy, $C_1-C_4$-alkylthio, $C_1-C_4$-halogenoalkylthio, $C_1-C_4$-alkylsulphinyl, $C_1-C_4$-alkylsulphonyl, $C_1-C_4$-alkylamino or di-($C_1-C_2$-alkyl)-amino,

X represents nitrogen or a CH group,

Y represents nitrogen or a $CR^5$ group in which

$R^5$ represents hydrogen, fluorine, chlorine, bromine, cyano, methyl, formyl, acetyl, methoxycarbonyl or ethoxycarbonyl and

Z represents nitrogen or a $CR^6$ group in which

$R^6$ represents hydrogen, fluorine, chlorine, bromine, $C_1-C_4$-alkyl, $C_1-C_4$-alkoxy, $C_1-C_4$-halogenoalkoxy, $C_1-C_4$-alkylthio, $C_1-C_4$-alkylsulphinyl, $C_1-C_4$-alkylsulphonyl, $C_1-C_4$-alkylamino, dimethylamino or diethylamino,

and salts of compounds of the formula (I).

**2.** Substituted sulphonylaminotriazolinones of the general formula (I) according to Claim 1, characterised in that, in this formula,

$R^1$ represents the radical

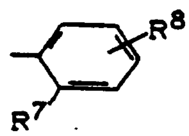

in which

$R^7$ represents fluorine, chlorine, bromine, methyl, trifluoromethyl, methoxy, difluoromethoxy, trifluoromethoxy, $C_1-C_3$-alkylthio, $C_1-C_3$-alkylsulphinyl, $C_1-C_3$-alkylsulphonyl, dimethylaminosulphonyl, N-methoxy-N-methylaminosulphonyl, phenyl, phenoxy or $C_1-C_3$-alkoxy-carbonyl and

$R^8$ represents hydrogen, fluorine or chlorine; in which furthermore

$R^1$ represents the radical

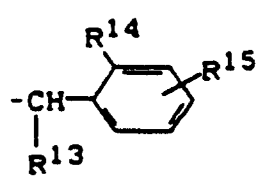

in which

$R^{13}$ represents hydrogen,

$R^{14}$ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and

$R^{15}$ represents hydrogen; in which furthermore

$R^1$ represents the radical

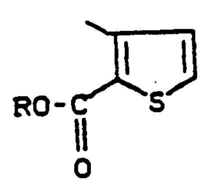

in which

R represents $C_1-C_4$-alkyl, or

$R^1$ represents the radical

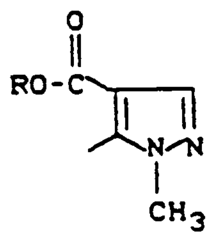

in which

R represents $C_1-C_4$-alkyl, or

$R^1$ represents the radical

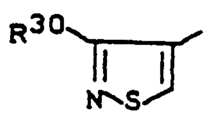

in which

$R^{30}$ represents hydrogen, chlorine, methyl, ethyl, methoxycarbonyl or ethoxycarbonyl;

in which furthermore

$R^2$ represents hydrogen or the group $-SO_2-R^1$ in which

$R^1$ has the meaning given above as being particularly preferred,

$R^3$ represents hydrogen, methyl, ethyl, propyl, isopropyl, trifluoromethyl, cyclopropyl, benzyl, phenyl, t-butyl, s-butyl, i-butyl, n-butyl, methoxy or methylthio,

$R^4$ represents hydrogen, fluorine, chlorine, bromine, methyl, trifluoromethyl, methoxy, ethoxy, difluoromethoxy, methylthio, ethylthio, amino, methylamino, ethylamino, dimethylamino or diethylamino,

X represents nitrogen or a CH group,

Y represents nitrogen or a $CR^5$ group in which

$R^5$ represents hydrogen, fluorine, chlorine or methyl, and

Z represents nitrogen or a $CR^6$ group in which

$R^6$ represents hydrogen, fluorine, chlorine, bromine, methyl, ethyl, methoxy, ethoxy, propoxy, isopropoxy, difluoromethoxy, methylthio, ethylthio, methylamino, ethylamino, dimethylamino or diethylamino,

and salts of compounds of the formula (I).

**3.** Process for the preparation of substituted sulphonylaminotriazolinones of the general formula (I) according to Claim 1 and their salts, characterised in that

(a) substituted aminotriazolinones of the general formula (II)

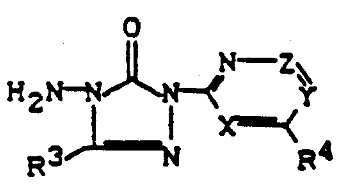

(II)

in which

$R^3$, $R^4$, X, Y and Z have the meaning given in Claim 1,

are reacted with sulphonyl halides or sulphonic anhydrides of the general formula (III)

$R^1 - SO_2 - Q$ (III)

in which

$R^1$ has the meaning given in Claim 1 and

Q represents fluorine, chlorine, bromine or the group $-O-SO_2-R^1$ in which

$R^1$ has the abovementioned meaning,

if appropriate in the presence of an acid acceptor and if appropriate in the presence of a diluent, the resulting compounds of the formula (I) in which

$R^2$ represents the group $-SO_2-R^1$,

are optionally reacted with desulphonylating agents,

if appropriate in the presence of diluents, to give compounds of the formula (I) in which

$R^2$ represents hydrogen, or

(b) sulphonylaminotriazolinones of the general formula (IV)

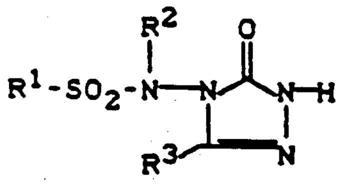

(IV)

in which

$R^1$, $R^2$ and $R^3$ have the meanings given in Claim 1,

are reacted with azines of the general formula (V)

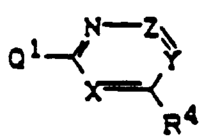

(V)

in which

$R^4$, X, Y and Z have the meanings given in Claim 1 and

$Q^1$ represents halogen, benzylsulphonyl or alkylsulphonyl,

if appropriate in the presence of an acid acceptor and if appropriate in the presence of a diluent, and salts are optionally produced by customary methods from the resulting compounds of the formula (I).

**4.** Herbicidal agents, characterised in that they contain at least one substituted sulphonylaminotriazolinone of the formula (I) according to Claim 1.

**5.** Method of combating weeds, characterised in that substituted sulphonylaminotriazolinones of the formula (I) according to Claim 1 are allowed to act on the weeds and/or their environment.

**6.** Use of substituted sulphonylaminotriazolinones of the formula (I) according to Claim 1 for combating weeds.

**7.** Process for the preparation of herbicidal agents, characterised in that substituted sulphonylaminotriazolinones of the formula (I) according to Claim 1 are mixed with extenders and/or surface-active agents.

**Claims for the following Contracting State : ES**

1. Process for the preparation of substituted sulphonylaminotriazolinones of the general formula (I)

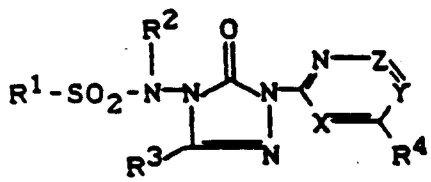

(I)

in which

$R^1$ represents the radical

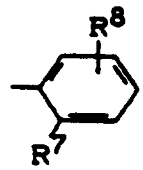

in which

$R^7$ and $R^8$ are identical or different and represent hydrogen, fluorine, chlorine, bromine, iodine, cyano, nitro, $C_1-C_6$-alkyl [which is optionally substituted by fluorine, chlorine, bromine, cyano, carboxyl, $C_1-C_4$-alkoxycarbonyl, $C_1-C_4$-alkylamino-carbonyl, di-($C_1-C_4$-alkyl)aminocarbonyl, hydroxyl, $C_1-C_4$-alkoxy, formyloxy, $C_1-C_4$-alkyl-carbonyloxy, $C_1-C_4$-alkoxy-carbonyloxy, $C_1-C_4$-alkylamino-carbonyloxy, $C_1-C_4$-alkylthio, $C_1-C_4$-alkylsulphinyl, $C_1-C_4$-alkylsulphonyl, di-($C_1-C_4$-alkyl)-aminosulphonyl, $C_3-C_6$-cycloalkyl or phenyl] or represent $C_2-C_6$-alkenyl [which is optionally substituted by fluorine, chlorine, bromine, cyano, $C_1-C_4$-alkoxy-carbonyl, carboxyl or phenyl], or represent $C_2-C_6$-alkinyl [which is optionally substituted by fluorine, chlorine, bromine, cyano, $C_1-C_4$-alkoxy-carbonyl, carboxyl or phenyl], or represent $C_1-C_4$-alkoxy [which is optionally substituted by fluorine, chlorine, bromine, cyano, carboxyl, $C_1-C_4$-alkoxy-carbonyl, $C_1-C_4$-alkoxy, $C_1-C_4$-alkylthio, $C_1-C_4$-alkylsulphinyl or $C_1-C_4$-alkylsulphonyl], or represent $C_1-C_4$-alkylthio [which is optionally substituted by fluorine, chlorine, bromine, cyano, carboxyl, $C_1-C_4$-alkoxy-carbonyl, $C_1-C_4$-alkylthio, $C_1-C_4$-alkylsulphinyl or $C_1-C_4$-alkylsulphonyl], or represent $C_3-C_6$-alkenyloxy [which is optionally substituted by fluorine, chlorine, bromine, cyano or $C_1-C_4$-alkoxy-carbonyl], or represent $C_2-C_6$-alkenylthio [which is optionally substituted by fluorine, chlorine, bromine, cyano, nitro, $C_1-C_3$-alkylthio or $C_1-C_4$-alkoxy-carbonyl], $C_3-C_6$-alkinyloxy, $C_3-C_6$-alkinylthio or the radical $-S(O)_pR^9$, in which

p represents the numbers 1 or 2 and

$R^9$ represents fluorine, $C_1-C_4$-alkyl [which is optionally substituted by fluorine, chlorine, bromine, cyano or $C_1-C_4$-alkoxy-carbonyl], $C_3-C_6$-alkenyl, $C_3-C_6$-alkinyl, $C_1-C_4$-alkoxy, $C_1-C_4$-alkoxy-$C_1-C_4$-alkylamino, $C_1-C_4$-alkylamino, di-($C_1-C_4$-alkyl)-amino or the radical $-NHOR^{10}$, in which

$R^{10}$ represents $C_1-C_6$-alkyl [which is optionally substituted by fluorine, chlorine, cyano, $C_1-C_4$-alkoxy, $C_1-C_4$-alkylthio, $C_1-C_4$-alkylsulphinyl, $C_1-C_4$-alkylsulphonyl, $C_1-C_4$-alkyl-carbonyl, $C_1-C_4$-alkoxy-carbonyl, $C_1-C_4$-alkylamino-carbonyl or di-($C_1-C_4$-alkyl)-amino-carbonyl], or represents $C_3-C_6$-alkenyl [which is optionally substituted by fluorine, chlorine or bromine], $C_3-C_6$-alkinyl, $C_3-C_6$-cycloalkyl, $C_3-C_6$-cycloalkyl-$C_1-C_2$-alkyl, phenyl-$C_1-C_2$-alkyl [which is optionally substituted by fluorine, chlorine, nitro, cyano, $C_1-C_4$-alkyl, $C_1-C_4$-alkoxy or $C_1-C_4$-alkoxy-carbonyl], or represents ben-

zhydryl, or represents phenyl [which is optionally substituted by fluorine, chlorine, nitro, cyano, $C_1-C_4$-alkyl, trifluoromethyl, $C_1-C_4$-alkoxy, $C_1-C_2$-fluoroalkoxy, $C_1-C_4$-alkylthio, trifluoromethylthio or $C_1-C_4$-alkoxy-carbonyl],

$R^7$ and/or $R^8$ furthermore represent phenyl or phenoxy, or represent $C_1-C_4$-alkylcarbonylamino, $C_1-C_4$-alkoxy-carbonylamino, $C_1-C_4$-alkyl-aminocarbonyl-amino, di-($C_1-C_4$-alkyl)-aminocarbonylamino, or represent the radical $-CO-R^{11}$, in which

$R^{11}$ represents $C_1-C_6$-alkyl, $C_1-C_6$-alkoxy, $C_3-C_6$-cycloalkoxy, $C_3-C_6$-alkenyloxy, $C_1-C_4$-alkylthio, $C_1-C_4$-alkylamino, $C_1-C_4$-alkoxyamino, $C_1-C_4$-alkoxy-$C_1-C_4$-alkyl-amino or di-($C_1-C_4$-alkyl)-amino [each of which is optionally substituted by fluorine and/or chlorine],

$R^7$ and/or $R^8$ furthermore represent $C_1-C_4$-alkylsulphonyloxy, di-($C_1-C_4$-alkyl)-aminosulphonylamino or the radical $-CH=N-R^{12}$, in which

$R^{12}$ represents $C_1-C_6$-alkyl which is optionally substituted by fluorine, chlorine, cyano, carboxyl, $C_1-C_4$-alkoxy, $C_1-C_4$-alkylthio, $C_1-C_4$-alkylsulphinyl or $C_1-C_4$-alkylsulphonyl, or represents benzyl which is optionally substituted by fluorine or chlorine, or represents $C_3-C_6$-alkenyl or $C_3-C_6$-alkinyl, each of which is optionally substituted by fluorine or chlorine, or represents phenyl which is optionally substituted by fluorine, chlorine, bromine, $C_1-C_4$-alkyl, $C_1-C_4$-alkoxy, trifluoromethyl, trifluoromethoxy or trifluoromethylthio, or represents $C_1-C_6$-alkoxy, $C_3-C_6$-alkenoxy, $C_3-C_6$-alkinoxy or benzyloxy, each of which is optionally substituted by fluorine and/or chlorine, or represents amino, $C_1-C_4$-alkylamino, di-($C_1-C_4$-alkyl)-amino, phenylamino, $C_1-C_4$-alkyl-carbonyl-amino, $C_1-C_4$-alkoxy-carbonylamino, $C_1-C_4$-alkyl-sulphonylamino, or represents phenylsulphonylamino which is optionally substituted by fluorine, chlorine, bromine or methyl,

in which furthermore

$R^1$ represents the radical

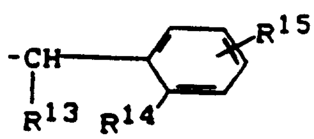

in which

$R^{13}$ represents hydrogen or $C_1-C_4$-alkyl,

$R^{14}$ and $R^{15}$ are identical or different and represent hydrogen, fluorine, chlorine, bromine, nitro, cyano, $C_1-C_4$-alkyl [which is optionally substituted by fluorine and/or chlorine], $C_1-C_4$-alkoxy [which is optionally substituted by fluorine and/or chlorine], carboxyl, $C_1-C_4$-alkoxy-carbonyl, $C_1-C_4$-alkylsulphonyl or di-($C_1-C_4$-alkyl)-aminosulphonyl; in which furthermore

$R^1$ represents the radical

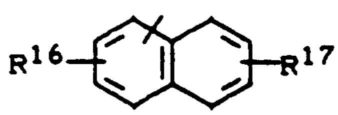

in which

$R^{16}$ and $R^{17}$ are identical or different and represent hydrogen, fluorine, chlorine, bromine, nitro, cyano, $C_1-C_4$-alkyl [which is optionally substituted by fluorine and/or chlorine] or $C_1-C_4$-alkoxy [which is optionally substituted by fluorine and/or chlorine]; in which furthermore

$R^1$ represents the radical

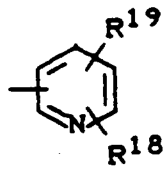

in which

$R^{18}$ and $R^{19}$ are identical or different and represent hydrogen, fluorine, chlorine, bromine, nitro, cyano, $C_1-C_4$-alkyl [which is optionally substituted by fluorine and/or chlorine], $C_2-C_4$-alkenyl [which is optionally substituted by fluorine and/or chlorine]; $C_1-C_4$-alkoxy [which is optionally substituted by fluorine and/or chlorine], or represent $C_1-C_4$-alkylthio, $C_1-C_4$-alkylsulphinyl or $C_1-C_4$-alkylsulphonyl [each of which is optionally substituted by fluorine and/or chlorine], or represent di-($C_1-C_4$-alkyl)-aminosulphonyl, $C_1-C_4$-alkoxy-carbonyl, dimethylamino-carbonyl or dioxolanyl; in which furthermore

$R^1$ represents the radical

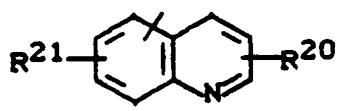

in which

$R^{20}$ and $R^{21}$ are identical or different and represent hydrogen, fluorine, chlorine, bromine, $C_1-C_4$-alkyl [which is optionally substituted by fluorine and/or bromine], $C_1-C_4$-alkoxy [which is optionally substituted by fluorine and/or chlorine], or represent $C_1-C_4$-alkylthio, $C_1-C_4$-alkylsulphinyl or $C_1-C_4$-alkylsulphonyl [each of which is optionally substituted by fluorine and/or chlorine], or represent di-($C_1-C_4$-alkyl)-aminosulphonyl, in which furthermore

$R^1$ represents the radical

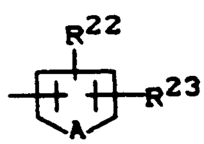

in which

$R^{22}$ and $R^{23}$ are identical or different and represent hydrogen, fluorine, chlorine, bromine, cyano, nitro, $C_1-C_4$-alkyl [which is optionally substituted by fluorine, chlorine, $C_1-C_4$-alkoxy and/or $C_1-C_4$-halogenoalkoxy], $C_1-C_4$-alkoxy [which is optionally substituted by fluorine and/or chlorine], $C_1-C_4$-alkylthio, $C_1-C_4$-alkylsulphinyl or $C_1-C_4$-alkylsulphonyl [which is optionally substituted by fluorine and/or chlorine], di-($C_1-C_4$-alkyl)-aminosulphonyl or $C_1-C_4$-alkoxy-carbonyl, and

A represents oxygen, sulphur or the group $N-Z^1$, in which

$Z^1$ represents hydrogen, $C_1-C_4$-alkyl [which is optionally substituted by fluorine, chlorine, bromine or cyano], $C_3-C_6$-cycloalkyl, benzyl, phenyl [which is optionally substituted by fluorine, chlorine, bromine or nitro], $C_1-C_4$-alkylcarbonyl, $C_1-C_4$-alkoxy-carbonyl or di-($C_1-C_4$-alkyl)-amino-carbonyl; in which furthermore

$R^1$ represents the radical

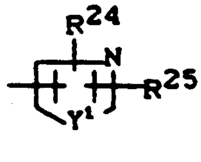

in which

$R^{24}$ and $R^{25}$ are identical or different and represent hydrogen, $C_1-C_4$-alkyl, halogen, $C_1-C_4$-alkoxycarbonyl, $C_1-C_4$-alkoxy or $C_1-C_4$-halogenoalkoxy,

$Y^1$ represents sulphur or the group $N-R^{26}$, in which

$R^{26}$ represents hydrogen or $C_1-C_4$-alkyl; in which furthermore

$R^1$ represents the radical

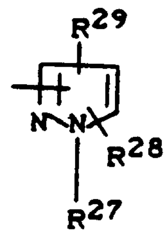

in which

$R^{27}$ represents hydrogen, $C_1-C_4$-alkyl, benzyl, (iso)-quinolinyl or phenyl,

$R^{28}$ represents hydrogen, halogen, cyano, nitro, $C_1-C_4$-alkyl [which is optionally substituted by fluorine and/or chlorine], $C_1-C_4$-alkoxy [which is optionally substituted by fluorine and/or chlorine], dioxolanyl or $C_1-C_4$-alkoxy-carbonyl and

$R^{29}$ represents hydrogen, halogen or $C_1-C_4$-alkyl; in which furthermore

$R^1$ represents the radical

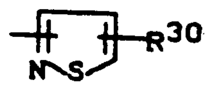

in which

$R^{30}$ represents hydrogen, halogen, $C_1-C_4$-alkyl, $C_1-C_4$-halogenoalkyl, $C_1-C_4$-alkoxy, $C_1-C_4$-halogenoalkoxy or $C_1-C_4$-alkoxy-carbonyl; in which furthermore

$R^1$ represents the radical

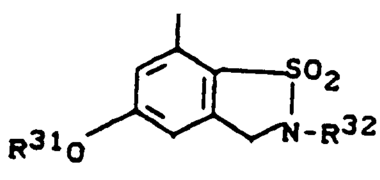

in which

$R^{31}$ represents $C_1-C_4$-alkyl and

$R^{32}$ represents $C_1-C_4$-alkyl; in which furthermore

$R^1$ represents the radical

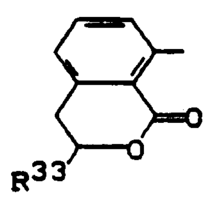

in which

$R^{33}$ represents hydrogen or methyl,

in which furthermore

$R^2$ represents hydrogen or the group $-SO_2-R^1$ in which

$R^1$ has the meaning mentioned above as being preferred;

in which furthermore

$R^3$ represents hydrogen, fluorine, chlorine, bromine, iodine, hydroxyl, mercapto, amino, or a radical which is selected from the series comprising $C_1-C_4$-alkyl, $C_3-C_6$-cycloalkyl, benzyl, phenyl, $C_1-C_4$-alkoxy, $C_3-C_4$-alkenyloxy, $C_3-C_4$-alkinyloxy, $C_1-C_4$-alkylthio, $C_1-C_4$-alkylsulphinyl, $C_1-C_4$-alkylsulphonyl, $C_3-C_4$-alkenylthio, $C_3-C_4$-alkinylthio, benzyloxy, benzylthio, $C_1-C_4$-alkylamino and di-($C_1-C_4$-alkyl)-amino, and which is optionally substituted by fluorine and/or chlorine,

$R^4$ represents hydrogen, fluorine, chlorine, bromine, hydroxyl, amino, $C_1-C_4$-alkyl, $C_1-C_4$-halogenoalkyl, $C_1-C_2$-alkoxy-$C_1-C_2$-alkyl, C,-$C_4$-alkoxy, $C_1-C_4$-halogenoalkoxy, $C_1-C_2$-alkoxy-$C_1-C_2$-alkoxy, $C_1-C_4$-alkylthio, $C_1-C_4$-halogenoalkylthio, $C_1-C_4$-alkylsulphinyl, $C_1-C_4$-alkylsulphonyl, $C_1-C_4$-alkylamino or di-($C_1-C_2$-alkyl)-amino,

X represents nitrogen or a CH group,

Y represents nitrogen or a $CR^5$ group in which

$R^5$ represents hydrogen, fluorine, chlorine, bromine, cyano, methyl, formyl, acetyl, methoxycarbonyl or ethoxycarbonyl and

Z represents nitrogen or a $CR^6$ group in which

$R^6$ represents hydrogen, fluorine, chlorine, bromine, $C_1-C_4$-alkyl, $C_1-C_4$-alkoxy, $C_1-C_4$-halogenoalkoxy, $C_1-C_4$-alkylthio, $C_1-C_4$-alkylsulphinyl, $C_1-C_4$-alkylsulphonyl, $C_1-C_4$-alkylamino, dimethylamino or diethylamino,

and of salts of the compounds of the formula (I),
characterised in that

(a) substituted aminotriazolinones of the general formula (II)

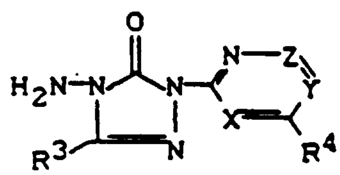

(II)

in which

$R^3$, $R^4$, X, Y and Z have the meaning given above in formula (I)

are reacted with sulphonyl halides or sulphonic anhydrides of the general formula (III)

$R^1 - SO_2 - Q$ (III)

in which

$R^1$ has the meaning given above and

Q represents fluorine, chlorine, bromine or the group $-O-SO_2-R^1$ in which

$R^1$ has the abovementioned meaning,

if appropriate in the presence of an acid acceptor and if appropriate in the presence of a diluent, the resulting compounds of the formula (I) in which

$R^2$ represents the group $-SO_2-R^1$,

are optionally reacted with desulphonylating agents,
if appropriate in the presence of diluents, to give compounds of the formula (I) in which

$R^2$ represents hydrogen, or

(b) sulphonylaminotriazolinones of the general formula (IV)

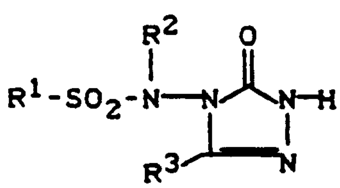

(IV)

in which

$R^1$, $R^2$ and $R^3$ have the meanings given above,

are reacted with azines of the general formula (V)

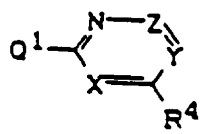

(V)

in which

$R^4$, X, Y and Z have the meanings given above and

$Q^1$ represents halogen, benzylsulphonyl or alkylsulphonyl,

if appropriate in the presence of an acid acceptor and if appropriate in the presence of a diluent, and salts are optionally produced by customary methods from the resulting compounds of the formula (I).

**2.** Process for the preparation of substituted sulphonylaminotriazolinones of the general formula (I) and of their salts according to Claim 1, characterised in that, in formula (I),

$R^1$ represents the radical

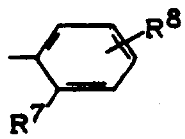

in which

$R^7$ represents fluorine, chlorine, bromine, methyl, trifluoromethyl, methoxy, difluoromethoxy, trifluoromethoxy, $C_1-C_3$-alkylthio, $C_1-C_3$-alkylsulphinyl, $C_1-C_3$-alkylsulphonyl, dimethylaminosulphonyl, N-methoxy-N-methylaminosulphonyl, phenyl, phenoxy or $C_1-C_3$-alkoxy-carbonyl and

$R^8$ represents hydrogen, fluorine or chlorine; in which furthermore

$R^1$ represents the radical

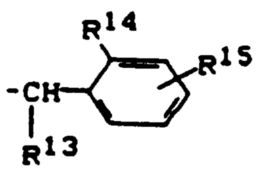

in which

$R^{13}$ represents hydrogen,

$R^{14}$ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and

$R^{15}$ represents hydrogen; in which furthermore

$R^1$ represents the radical

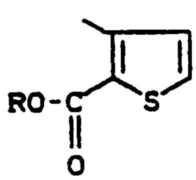

in which

R represents $C_1-C_4$-alkyl, or

$R^1$ represents the radical

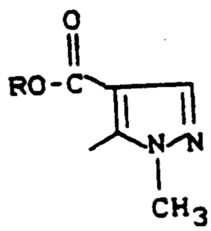

in which

R represents $C_1-C_4$-alkyl, or

$R^1$ represents the radical

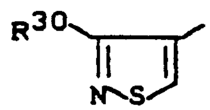

in which

$R^{30}$ represents hydrogen, chlorine, methyl, ethyl, methoxycarbonyl or ethoxycarbonyl;

in which furthermore

$R^2$ represents hydrogen or the group $-SO_2-R^1$ in which

$R^1$ has the meaning given above as being particularly preferred,

$R^3$ represents hydrogen, methyl, ethyl, propyl, isopropyl, trifluoromethyl, cyclopropyl, benzyl, phenyl, t-butyl, s-butyl, i-butyl, n-butyl, methoxy or methylthio,

$R^4$ represents hydrogen, fluorine, chlorine, bromine, methyl, trifluoromethyl, methoxy, ethoxy, difluoromethoxy, methylthio, ethylthio, amino, methylamino, ethylamino, dimethylamino or diethylamino,

X represents nitrogen or a CH group,

Y represents nitrogen or a $CR^5$ group in which

$R^5$ represents hydrogen, fluorine, chlorine or methyl, and

Z represents nitrogen or a $CR^6$ group in which

$R^6$ represents hydrogen, fluorine, chlorine, bromine, methyl, ethyl, methoxy, ethoxy, propoxy, isopropoxy, difluoromethoxy, methylthio, ethylthio, methylamino, ethylamino, dimethylamino or diethylamino.

**3.** Herbicidal agents, characterised in that they contain at least one substituted sulphonylaminotriazolinone of the formula (I) according to Claim 1.

**4.** Method of combating weeds, characterised in that substituted sulphonylaminotriazolinones of the formula (I) according to Claim 1 are allowed to act on the weeds and/or their environment.

**5.** Use of substituted sulphonylaminotriazolinones of the formula (I) according to Claim 1 for combating weeds.

**6.** Process for the preparation of herbicidal agents, characterised in that substituted sulphonylaminotriazolinones of the formula (I) according to Claim 1 are mixed with extenders and/or surface-active agents.

**Revendications**

**Revendications pour les Etats contractants suivants : BE, CH, DE, FR, GB, IT, LI, NL**

1. Sulfonylaminotriazolinones substituées de formule générale (I)

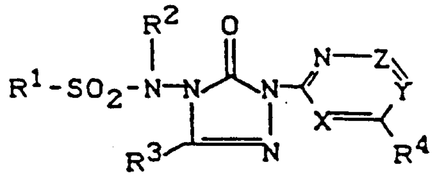

(I)

dans laquelle

$R^1$ représente le reste

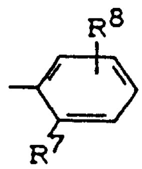

dans lequel

$R^7$ et $R^8$ sont identiques ou différents et représentent l'hydrogène, le fluor, le chlore, le brome, l'iode, un groupe cyano, nitro, alkyle en $C_1$ à $C_6$ [qui est éventuellement substitué par un radical fluoro, chloro, bromo, cyano, carboxy, (alkoxy en $C_1$ à $C_4$)-carbonyle, (alkylamino en $C_1$ à $C_4$)carbonyle, di(alkyle en $C_1$ à $C_4$)aminocarbonyle, hydroxy, alkoxy en $C_1$ à $C_4$, formyloxy, (alkyle en $C_1$ à $C_4$)carbonyloxy, (alkoxy en $C_1$ à $C_4$)carbonyloxy, (alkylamino en $C_1$ à $C_4$)carbonyloxy, alkylthio en $C_1$ à $C_4$, alkylsulfinyle en $C_1$ à $C_4$, alkylsulfonyle en $C_1$ à $C_4$, di(alkyle en $C_1$ à $C_4$)-aminosulfonyle, cycloalkyle en $C_3$ à $C_6$ ou phényle], un groupe alcényle en $C_2$ à $C_6$ [qui est substitué le cas échéant par du fluor, du chlore, du brome, un radical cyano, (alkoxy en $C_1$ à $C_4$)carbonyle, carboxy ou phényle], un groupe alcynyle en $C_2$ à $C_6$ [qui est substitué le cas échéant par du fluor, du chlore, du brome, un radical cyano, (alkoxy en $C_1$ à $C_4$)carbonyle, carboxy ou phényle], un groupe alkoxy en $C_1$ à $C_4$ - [qui est substitué le cas échéant par du fluor, du chlore, du brome, un radical cyano, carboxy, (alkoxy en $C_1$ à $C_4$)carbonyle, alkoxy en $C_1$ à $C_4$, alkylthio en $C_1$ à $C_4$, alkylsulfinyle en $C_1$ à $C_4$ ou alkylsulfonyle en $C_1$ à $C_4$], un groupe alkylthio en $C_1$ à $C_4$ [qui est substitué le cas échéant par du fluor, du chlore, du brome, un radical cyano, carboxy, (alkoxy en $C_1$ à $C_4$)carbonyle, alkylthio en $C_1$ à $C_4$, alkylsulfinyle en $C_1$ à $C_4$ ou alkylsulfonyle en $C_1$ à $C_4$], un groupe alcényloxy en $C_3$ à $C_6$ [qui est substitué le cas échéant par du fluor, du chlore, du brome, un radical cyano ou (alkoxy en $C_1$ à $C_4$)carbonyle], un groupe alcénylthio en $C_2$ à $C_6$ [qui est substitué le cas échéant par du fluor, du chlore, du brome, un radical cyano, nitro, alkylthio en $C_1$ à $C_3$ ou (alkoxy en $C_1$ à $C_4$)carbonyle], un groupe alcynyloxy en $C_3$ à $C_6$, alcynylthio en $C_3$ à $C_6$ ou le reste $-S(O)_p-R^9$, dans lequel

p représente les nombres 1 ou 2 et

$R^9$ représente le fluor, un groupe alkyle en $C_1$ à $C_4$ [qui est substitué le cas échéant par du fluor, du chlore, du brome, un radical cyano ou (alkoxy en $C_1$ à $C_4$) carbonyle], un groupe alcényle en $C_3$ à $C_6$, alcynyle en $C_3$ à $C_6$, alkoxy en $C_1$ à $C_4$, (alkoxy en $C_1$ à $C_4$)-(alkylamino en $C_1$ à $C_4$), alkylamino en $C_1$ à $C_4$, di(alkyle en $C_1$ à $C_4$)amino ou le reste $-NHOR^{10}$, dans lequel

$R^{10}$ est un radical alkyle en $C_1$ à $C_4$ [qui est substitué le cas échéant par du fluor, du chlore, un radical cyano, alkoxy en $C_1$ à $C_4$, alkylthio en $C_1$ à $C_4$, alkylsulfinyle en $C_1$ à $C_4$, alkylsulfonyle en $C_1$ à $C_4$, (alkyle en $C_1$ à $C_4$)carbonyle, (alkoxy en $C_1$ à $C_4$)carbonyle, (alkylamino en $C_1$ à $C_4$)carbonyle ou di(alkyle en $C_1$ à $C_4$)-aminocarbonyle], un groupe alcényle en $C_3$ à $C_6$ [qui est substitué le cas échéant

par du fluor, du chlore ou du brome], un groupe alcynyle en $C_3$ à $C_6$, cycloalkyle en $C_3$ à $C_6$, (cycloalkyle en $C_3$ à $C_6$)-(alkyle en $C_1$ ou $C_2$), phényl-(alkyle en $C_1$ ou $C_2$ [qui est substitué le cas échéant par un radical fluoro, chloro, nitro, cyano, alkyle en $C_1$ à $C_4$, alkoxy en $C_1$ à $C_4$ ou (alkoxy en $C_1$ à $C_4$)carbonyle], un groupe benzhydryle ou un groupe phényle [qui est substitué le cas échéant par un radical fluoro, chloro, nitro, cyano, alkyle en $C_1$ à $C_4$, trifluorométhyle, alkoxy en $C_1$ à $C_4$, fluoralkoxy en $C_1$ ou $C_2$, alkylthio en $C_1$ à $C_4$, trifluorométhylthio ou (alkoxy en $C_1$ à $C_4$)carbonyle],

$R^7$ et/ou $R^8$ représentent en outre un groupe phényle ou phénoxy, un groupe (alkyle en $C_1$ à $C_4$)carbonylamino, (alkoxy en $C_1$ à $C_4$)carbonylamino, (alkylamino en $C_1$ à $C_4$)-carbonylamino, di(alkyle en $C_1$ à $C_4$)amino-carbonylamino ou le reste $-CO-R^{11}$, dans lequel

$R^{11}$ est un groupe alkyle en $C_1$ à $C_6$, alkoxy en $C_1$ à $C_6$, cycloalkoxy en $C_3$ à $C_6$, alcényloxy en $C_3$ à $C_6$, alkylthio en $C_1$ à $C_4$, alkylamino en $C_1$ à $C_4$, alkoxyamino en $C_1$ à $C_4$, (alkoxy en $C_1$ à $C_4$)-(alkylamino en $C_1$ à $C_4$) ou di(alkyle en $C_1$ à $C_4$)-amino [chacun pouvant éventuellement être substitué par du fluor et/ou du chlore],

$R^7$ et/ou $R^8$ représentent en outre un groupe (alkyle en $C_1$ à $C_4$)sulfonyloxy, di(alkyle en $C_1$ à $C_4$)aminosulfonylamino ou le reste $-CH=N-R^{12}$, où

$R^{12}$ est un groupe alkyle en $C_1$ à $C_6$ éventuellement substitué par du fluor, du chlore, un radical cyano, carboxy, alkoxy en $C_1$ à $C_4$, alkylthio en $C_1$ à $C_4$, alkylsulfinyle en $C_1$ à $C_4$ ou alkylsulfonyle en $C_1$ à $C_4$, un groupe benzyle éventuellement substitué par du fluor ou du chlore, un groupe alcényle en $C_3$ à $C_6$ ou alcynyle en $C_3$ à $C_6$ éventuellement substitué par du fluor ou du chlore, un groupe phényle éventuellement substitué par du fluor, du chlore, du brome, un radical alkyle en $C_1$ à $C_4$, alkoxy en $C_1$ à $C_4$, trifluorométhyle, trifluorométhoxy ou trifluorométhylthio, un groupe alkoxy en $C_1$ à $C_6$, alcénoxy en $C_3$ à $C_6$, alcynoxy en $C_3$ à $C_6$ ou benzyloxy, éventuellement substitué par du fluor et/ou du chlore, un groupe amino, (alkyle en $C_1$ à $C_4$)amino, di(alkyle en $C_1$ à $C_4$)amino, phénylamino, (alkyle en $C_1$ à $C_4$)-carbonylamino, (alkoxy en $C_1$ à $C_4$)carbonylamino, alkylsulfonylamino en $C_1$ à $C_4$ ou un groupe phénylsulfonylamino éventuellement substitué par du fluor, du chlore, du brome ou un radical méthyle,

en outre

$R^1$ représente le reste

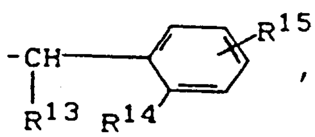

dans lequel

$R^{13}$ est l'hydrogène ou un radical alkyle en $C_1$ à $C_4$,

$R^{14}$ et $R^{15}$ sont identiques ou différents et représentent l'hydrogène, le fluor, le chlore, le brome, un groupe nitro, cyano, alkyle en $C_1$ à $C_4$ [qui est éventuellement substitué par du fluor et/ou par du chlore], un groupe alkoxy en $C_1$ à $C_4$ [qui est éventuellement substitué par du fluor et/ou par du chlore], un groupe carboxy, (alkoxy en $C_1$ à $C_4$)-carbonyle, alkylsulfonyle en $C_1$ à $C_4$ ou di(alkyle en $C_1$ à $C_4$)aminosulfonyle ;

en outre

$R^1$ représente le reste

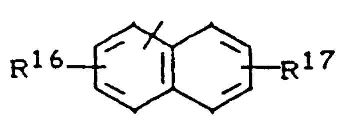

dans lequel

$R^{16}$ et $R^{17}$ sont identiques ou différents et représentent l'hydrogène, le fluor, le chlore, le brome,

un groupe nitro, cyano, alkyle en $C_1$ à $C_4$ [qui est éventuellement substitué par du fluor et/ou du chlore] ou un groupe alkoxy en $C_1$ à $C_4$ [qui est éventuellement substitué par du fluor et/ou du chlore] ; en outre

$R^1$ représente le reste

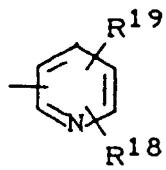

dans lequel

$R^{18}$ et $R^{19}$ sont identiques ou différents et représentent l'hydrogène, le fluor, le chlore, le brome, un groupe nitro, cyano, alkyle en $C_1$ à $C_4$ [qui est éventuellement substitué par du fluor et/ou du chlore], un groupe alcényle en $C_2$ à $C_4$ [qui est substitué le cas échéant par du fluor et/ou du chlore], un groupe alkoxy en $C_1$ à $C_4$ [qui est substitué le cas échéant par du fluor et/ou du chlore], un groupe alkylthio en $C_1$ à $C_4$, alkylsulfinyle en $C_1$ à $C_4$ ou alkylsulfonyle en $C_1$ à $C_4$ [qui sont substitués le cas échéant par du fluor et/ou du chlore] ainsi qu'un groupe di(alkyle en $C_1$ à $C_4$)aminosulfonyle, (alkoxy en $C_1$ à $C_4$)carbonyle, diméthylaminocarbonyle ou dioxolanyle ; en outre

$R^1$ représente le reste

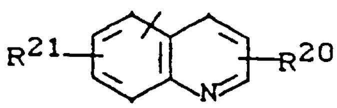

dans lequel

$R^{20}$ et $R^{21}$ sont identiques ou différents et représentent l'hydrogène, le fluor, le chlore, le brome, un groupe alkyle en $C_1$ à $C_4$ [qui est substitué le cas échéant par du fluor et/ou du brome], un groupe alkoxy en $C_1$ à $C_4$ [qui est substitué le cas échéant par du fluor et/ou du chlore], un groupe alkylthio en $C_1$ à $C_4$, alkylsulfinyle en $C_1$ à $C_4$ ou alkylsulfonyle en $C_1$ à $C_4$ [qui sont substitués le cas échéant par du fluor et/ou du chlore] ou un groupe di(alkyle en $C_1$ à $C_4$)aminosulfonyle ; en outre

$R^1$ représente le reste

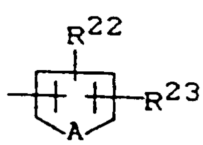

dans lequel

$R^{22}$ et $R^{23}$ sont identiques ou différents et représentent l'hydrogène, le fluor, le chlore, le brome, un groupe cyano, nitro, alkyle en $C_1$ à $C_4$ [qui est substitué le cas échéant par du fluor, du chlore, un radical alkoxy en $C_1$ à $C_4$ et/ou halogénalkoxy en $C_1$ à $C_4$], un groupe alkoxy en $C_1$ à $C_4$ [qui est substitué le cas échéant par du fluor et/ou du chlore], un groupe alkylthio en $C_1$ à $C_4$, alkylsulfinyle en $C_1$ à $C_4$ ou alkylsulfonyle en $C_1$ à $C_4$ [qui est substitué le cas échéant par du fluor et/ou du chlore], un groupe di-(alkyle en $C_1$ à $C_4$)aminosulfonyle ou (alkoxy en $C_1$ à $C_4$)carbonyle, et

A représente l'oxygène, le soufre ou le groupement $N-Z^1$, dans lequel

$Z^1$ est l'hydrogène, un groupe alkyle en $C_1$ à $C_4$ [qui est substitué le cas échéant par du fluor, du chlore, du brome ou un radical cyano] un groupe cycloalkyle en $C_3$ à $C_6$, benzyle, phényle [qui est éventuellement substitué par du fluor, du chlore, du brome ou un radical nitro], un groupe (alkyle en $C_1$ à $C_4$)carbonyle, (alkoxy en $C_1$ à $C_4$)-

carbonyle ou di(alkyle en $C_1$ à $C_4$)aminocarbonyle ; en outre

$R^1$ représente le reste

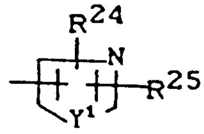

dans lequel

$R^{24}$ et $R^{25}$ sont identiques ou différents et représentent l'hydrogène, un groupe alkyle en $C_1$ à $C_4$, un halogène, un groupe (alkoxy en $C_1$ à $C_4$)carbonyle, alkoxy en $C_1$ à $C_4$ ou halogénalkoxy en $C_1$ à $C_4$,

$Y^1$ est le soufre ou représente le groupement $N-R^{26}$ dans lequel

$R^{26}$ est l'hydrogène ou un groupe alkyle en $C_1$ à $C_4$ ; en outre

$R^1$ représente le reste

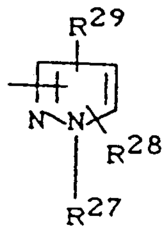

dans lequel

$R^{27}$ est l'hydrogène, un groupe alkyle en $C_1$ à $C_4$, benzyle, (iso)quinolinyle ou phényle,

$R^{28}$ est l'hydrogène, un halogène, un groupe cyano, nitro, alkyle en $C_1$ à $C_4$ [qui est substitué le cas échéant par du fluor et/ou du chlore], un groupe alkoxy en $C_1$ à $C_4$ – [qui est substitué le cas échéant par du fluor et/ou du chlore], un groupe dioxolanyle ou (alkoxy en $C_1$ à $C_4$)carbonyle et

$R^{29}$ représente l'hydrogène, un halogène ou un groupe alkyle en $C_1$ à $C_4$ ; en outre

$R^1$ représente le reste

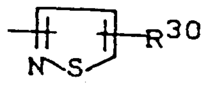

dans lequel

$R^{30}$ est l'hydrogène, un halogène, un groupe alkyle en $C_1$ à $C_4$, halogénalkyle en $C_1$ à $C_4$, alkoxy en $C_1$ à $C_4$, halogénalkoxy en $C_1$ à $C_4$ ou (alkoxy en $C_1$ à $C_4$)carbonyle ; en outre

$R^1$ représente le reste

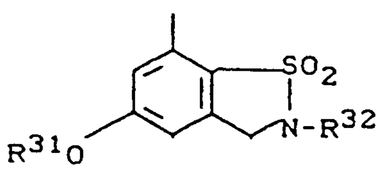

dans lequel

$R^{31}$ est un groupe alkyle en $C_1$ à $C_4$ et

$R^{32}$ est un groupe alkyle en $C_1$ à $C_4$ ; en outre

$R^1$ représente le reste

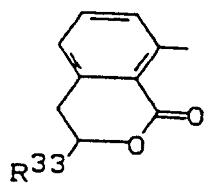

dans lequel

$R^{33}$ est l'hydrogène ou le groupe méthyle

où en outre

$R^2$ est l'hydrogène ou le groupement $-SO_2-R^1$ dans lequel

$R^1$ a la définition préférentielle indiquée ci-dessus ;

où en outre

$R^3$ est l'hydrogène, le fluor, le chlore, le brome, l'iode, un groupe hydroxy, mercapto, amino ou un reste éventuellement substitué par du fluor et/ou du chlore, de la série des restes alkyle en $C_1$ à $C_4$, cycloalkyle en $C_3$ à $C_6$, benzyle, phényle, alkoxy en $C_1$ à $C_4$, alcényloxy en $C_3$ ou $C_4$, alcynyloxy en $C_3$ ou $C_4$, alkylthio en $C_1$ à $C_4$, alkylsulfinyle en $C_1$ à $C_4$, alkylsulfonyle en $C_1$ à $C_4$, alcénylthio en $C_3$ ou $C_4$, alcynylthio en $C_3$ ou $C_4$, benzyloxy, benzylthio, (alkyle en $C_1$ à $C_4$)amino et di(alkyle en $C_1$ à $C_4$)amino,

$R^4$ est l'hydrogène, le fluor, le chlore, le brome, un groupe hydroxy, amino, alkyle en $C_1$ à $C_4$, halogénalkyle en $C_1$ à $C_4$, (alkoxy en $C_1$ ou $C_2$)-(alkyle en $C_1$ ou $C_2$), alkoxy en $C_1$ à $C_4$, halogénalkoxy en $C_1$ à $C_4$, (alkoxy en $C_1$ ou $C_2$)-(alkoxy en $C_1$ ou $C_2$), alkylthio en $C_1$ à $C_4$, halogénalkylthio en $C_1$ à $C_4$, alkylsulfinyle en $C_1$ à $C_4$, alkylsulfonyle en $C_1$ à $C_4$, alkylamino en $C_1$ à $C_4$ ou di(alkyle en $C_1$ ou $C_2$)amino,

X représente l'azote ou un groupement CH,

Y est l'azote ou un groupement $CR^5$, dans lequel

$R^5$ est l'hydrogène, le fluor, le chlore, le brome, un groupe cyano, méthyle, formyle, acétyle, méthoxycarbonyle ou éthoxycarbonyle et

Z est l'azote ou un groupement $CR^6$, dans lequel

$R^6$ est l'hydrogène, le flour, le chlore, le brome, un groupe alkyle en $C_1$ à $C_4$, alkoxy en $C_1$ à $C_4$, halogénalkoxy en $C_1$ à $C_4$, alkylthio en $C_1$ à $C_4$, alkylsulfinyle en $C_1$ à $C_4$, alkylsulfonyle en $C_1$ à $C_4$, alkylamino en $C_1$ à $C_4$, diméthylamino ou diéthylamino,

ainsi que des sels de composés de formule (I).

**2.** Sulfonylaminotriazolinones substituées de formule générale (I) suivant la revendication 1, caractérisées en ce que dans cette formule,

$R^1$ représente le reste

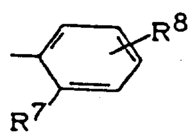

dans lequel

$R^7$ est le fluor, le chlore, le brome, un groupe méthyle, trifluorométhyle, méthoxy, difluorométhoxy, trifluorométhoxy, alkylthio en $C_1$ à $C_3$, alkylsulfinyle en $C_1$ à $C_3$, alkylsulfonyle en $C_1$ à $C_3$, diméthylaminosulfonyle, N-méthoxy-N-méthylaminosulfonyle, phényle, phénoxy ou (alkoxy en $C_1$ à $C_3$)carbonyle et

$R^8$ est l'hydrogène, le fluor ou le chlore ; dans laquelle en outre

$R^1$ représente le reste

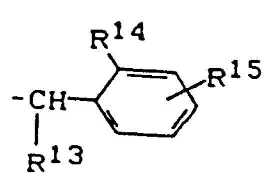

dans lequel

$R^{13}$ est l'hydrogène,

$R^{14}$ est le fluor, le chlore, le brome, un groupe méthyle, méthoxy, difluorométhoxy, trifluorométhoxy, éthoxy, méthoxycarbonyle, éthoxycarbonyle, méthylsulfonyle ou diméthylaminosulfonyle et

$R^{15}$ représente l'hydrogène ; dans laquelle en outre

$R^1$ représente le reste

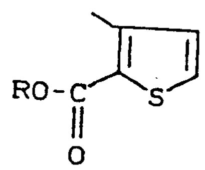

où

R est un radical alkyle en $C_1$ à $C_4$, ou bien

$R^1$ représente le reste

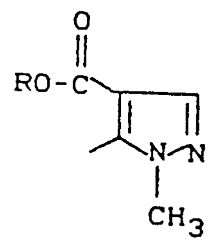

dans lequel

R est un groupe alkyle en $C_1$ à $C_4$, ou bien

$R^1$ représente le reste

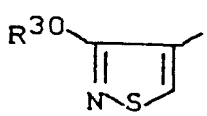

dans lequel

$R^{30}$ est l'hydrogène, le chlore, un radical méthyle, éthyle, méthoxycarbonyle ou éthoxycarbonyle ;

dans laquelle en outre

$R^2$ est l'hydrogène ou le groupement $-SO_2-R^1$ dans lequel

$R^1$ a la définition particulièrement appréciée indiquée ci-dessus

$R^3$ est l'hydrogène, un groupe méthyle, éthyle, propyle, isopropyle, trifluorométhyle, cyclopropyle, benzyle, phényle, tertiobutyle, sec.-butyle, isobutyle, n-butyle, méthoxy ou méthylthio,

$R^4$ est l'hydrogène, le fluor, le chlore, le brome, un groupe méthyle, trifluorométhyle, méthoxy, éthoxy, difluorométhoxy, méthylthio, éthylthio, amino, méthylamino, éthyalmino, diméthylamino ou diéthylamino,

X est l'azote ou un groupement CH,

Y est l'azote ou un groupement $CR^5$ dans lequel

$R^5$ est l'hydrogène, le fluor, le chlore ou un groupe méthyle, et

Z est l'azote ou un groupement $CR^6$,

dans lequel

$R^6$ est l'hydrogène, le fluor, le chlore, le brome, un groupe méthyle, éthyle, méthoxy, éthoxy,

propoxy, isopropoxy, difluorométhoxy, méthylthio, éthylthio, méthylamino, éthylamino, diméthylamino ou diéthylamino,

ainsi que des sels de composés de formule (I).

3. Procédé de production de sulfonylaminotriazolinones substituées de formule générale (I) suivant la revendication 1 et de leurs sels, caractérisé en ce que

(a) on fait réagir des aminotriazolinones substituées de formule générale (II)

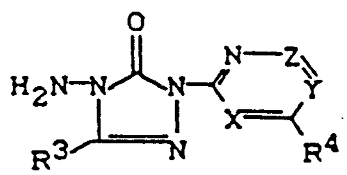

(II)

dans laquelle

$R^3$, $R^4$, X, Y et Z ont la définition indiquée dans la revendication 1,

avec des halogénures d'acides sulfoniques ou des anhydrides d'acides sulfoniques de formule générale (III)

$R^1 - SO_2 - Q$ (III)

où

$R^1$ a la définition indiquée dans la revendication 1 et

Q représente le fluor, le chlore, le brome ou le groupement $-O-SO_2-R^1$ dans lequel

$R^1$ a la définition indiquée ci-dessus,

le cas échéant en présence d'un accepteur d'acide et en la présence éventuelle d'un diluant,

on fait réagir éventuellement les composés ainsi obtenus de formule (I), dans laquelle

$R^2$ représente le groupement $-SO_2-R^1$,

avec des agents de désulfonylation,

éventuellement en présence de diluants pour former des composés de formule (I), dans laquelle

$R^2$ représente l'hydrogène, ou bien

(b) on fait réagir des sulfonylaminotriazolinones de formule générale (IV)

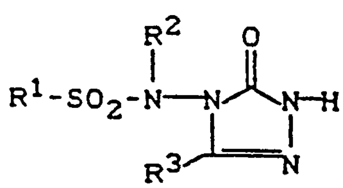

(IV)

dans laquelle

$R^1$, $R^2$ et $R^3$ ont la définition indiquée dans la revendication 1

avec des azines de formule générale (V)

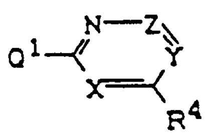

(V)

dans laquelle

$R^4$, X, Y et Z ont les définitions indiquées dans la revendication 1 et

$Q^1$ est un halogène, un groupe benzylsulfonyle ou alkylsulfonyle,

le cas échéant en présence d'un accepteur d'acide et en la présence éventuelle d'un diluant et on produit éventuellement des sels par des opérations classiques à partir des composés de formule (I)

ainsi obtenus.

4. Compositions herbicides, caractérisées par une teneur en au moins une sulfonylaminotriazolinone substituée de formule (I) suivant la revendication 1.

5. Procédé pour combattre des mauvaises herbes, caractérisé en ce qu'on fait agir des sulfonylamino-triazolinones substituées de formule (I) suivant la revendication 1 sur les mauvaises herbes et/ou sur leur milieu.

6. Utilisation de sulfonylaminotriazolinones substituées de formule (I) suivant la revendication 1 pour combattre des mauvaises herbes.

7. Procédé de préparation de compositions herbicides, caractérisé en ce qu'on mélange des sulfonylaminotriazolinones substituées de formule (I) suivant la revendication 1 avec des diluants et/ou des agents tensio-actifs.

**Revendications pour l'Etat contractant suivant : ES**

1. Procédé de production de sulfonylaminotriazolinones substituées de formule générale (I)

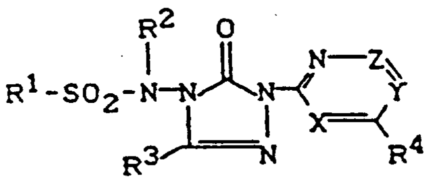

(I)

dans laquelle

$R^1$ représente le reste

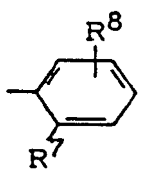

dans lequel

$R^7$ et $R^8$ sont identiques ou différents et représentent l'hydrogène, le fluor, le chlore, le brome, l'iode, un groupe cyano, nitro, alkyle en $C_1$ à $C_6$ [qui est éventuellement substitué par un radical fluoro, chloro, bromo, cyano, carboxy, (alkoxy en $C_1$ à $C_4$)-carbonyle, (alkylamino en $C_1$ à $C_4$)carbonyle, di(alkyle en $C_1$ à $C_4$)aminocarbonyle, hydroxy, alkoxy en $C_1$ à $C_4$, formyloxy, (alkyle en $C_1$ à $C_4$)carbonyloxy, (alkoxy en $C_1$ à $C_4$)carbonyloxy, (alkylamino en $C_1$ à $C_4$)carbonyloxy, alkylthio en $C_1$ à $C_4$, alkylsulfinyle en $C_1$ à $C_4$, alkylsulfonyle en $C_1$ à $C_4$, di(alkyle en $C_1$ à $C_4$)-aminosulfonyle, cycloalkyle en $C_3$ à $C_6$ ou phényle], un groupe alcényle en $C_2$ à $C_6$ [qui est substitué le cas échéant par du fluor, du chlore, du brome, un radical cyano, (alkoxy en $C_1$ à $C_4$)carbonyle, carboxy ou phényle], un groupe alcynyle en $C_2$ à $C_6$ [qui est substitué le cas échéant par du fluor, du chlore, du brome, un radical cyano, (alkoxy en $C_1$ à $C_4$)carbonyle, carboxy ou phényle], un groupe alkoxy en $C_1$ à $C_4$ - [qui est substitué le cas échéant par du fluor, du chlore, du brome, un radical cyano, carboxy, (alkoxy en $C_1$ à $C_4$)carbonyle, alkoxy en $C_1$ à $C_4$, alkylthio en $C_1$ à $C_4$, alkylsulfinyle en $C_1$ à $C_4$ ou alkylsulfonyle en $C_1$ à $C_4$], un groupe alkylthio en $C_1$ à $C_4$ [qui est substitué le cas échéant par du fluor, du chlore, du brome, un radical cyano, carboxy, (alkoxy en $C_1$ à $C_4$)carbonyle, alkylthio en $C_1$ à $C_4$, alkylsulfinyle en $C_1$ à $C_4$ ou alkylsulfonyle en $C_1$ à $C_4$], un groupe alcényloxy en $C_3$ à $C_6$ [qui est substitué le cas échéant par du fluor, du chlore, du brome, un radical cyano ou

(alkoxy en $C_1$ à $C_4$)carbonyle], un groupe alcénylthio en $C_2$ à $C_6$ [qui est substitué le cas échéant par du fluor, du chlore, du brome, un radical cyano, nitro, alkylthio en $C_1$ à $C_3$ ou (alkoxy en $C_1$ à $C_4$)carbonyle], un groupe alcynyloxy en $C_3$ à $C_6$, alcynylthio en $C_3$ à $C_6$ ou le reste $-S(O)_p-R^9$, dans lequel

p représente les nombres 1 ou 2 et

$R^9$ représente le fluor, un groupe alkyle en $C_1$ à $C_4$ [qui est substitué le cas échéant par du fluor, du chlore, du brome, un radical cyano ou (alkoxy en $C_1$ à $C_4$)-carbonyle], un groupe alcényle en $C_3$ à $C_6$, alcynyle en $C_3$ à $C_6$, alkoxy en $C_1$ à $C_4$, (alkoxy en $C_1$ à $C_4$)-(alkylamino en $C_1$ à $C_4$), alkylamino en $C_1$ à $C_4$, di(alkyle en $C_1$ à $C_4$)amino ou le reste $-NHOR^{10}$, dans lequel

$R^{10}$ est un radical alkyle en $C_1$ à $C_4$ [qui est substitué le cas échéant par du fluor, du chlore, un radical cyano, alkoxy en $C_1$ à $C_4$, alkylthio en $C_1$ à $C_4$, alkylsulfinyle en $C_1$ à $C_4$, alkylsulfonyle en $C_1$ à $C_4$, (alkyle en $C_1$ à $C_4$)carbonyle, (alkoxy en $C_1$ à $C_4$)carbonyle, (alkylamino en $C_1$ à $C_4$)carbonyle ou di(alkyle en $C_1$ à $C_4$)-aminocarbonyle], un groupe alcényle en $C_3$ à $C_6$ [qui est substitué le cas échéant par du fluor, du chlore ou du brome], un groupe alcynyle en $C_3$ à $C_6$, cycloalkyle en $C_3$ à $C_6$, (cycloalkyle en $C_3$ à $C_6$)-(alkyle en $C_1$ ou $C_2$), phényl(alkyle en $C_1$ ou $C_2$ [qui est substitué le cas échéant par un radical fluoro, chloro, nitro, cyano, alkyle en $C_1$ à $C_4$, alkoxy en $C_1$ à $C_4$ ou (alkoxy en $C_1$ à $C_4$)carbonyle], un groupe benzhydryle ou un groupe phényle [qui est substitué le cas échéant par un radical fluoro, chloro, nitro, cyano, alkyle en $C_1$ à $C_4$, trifluorométhyle, alkoxy en $C_1$ à $C_4$, fluoralkoxy en $C_1$ ou $C_2$, alkylthio en $C_1$ à $C_4$, trifluorométhylthio ou (alkoxy en $C_1$ à $C_4$)carbonyle],

$R^7$ et/ou $R^8$ représentent en outre un groupe phényle ou phénoxy, un groupe (alkyle en $C_1$ à $C_4$)carbonylamino, (alkoxy en $C_1$ à $C_4$)carbonylamino, (alkylamino en $C_1$ à $C_4$)-carbonylamino, di(alkyle en $C_1$ à $C_4$)amino-carbonylamino ou le reste $-CO-R^{11}$, dans lequel

$R^{11}$ est un groupe alkyle en $C_1$ à $C_6$, alkoxy en $C_1$ à $C_6$, cycloalkoxy en $C_3$ à $C_6$, alcényloxy en $C_3$ à $C_6$, alkylthio en $C_1$ à $C_4$, alkylamino en $C_1$ à $C_4$, alkoxyamino en $C_1$ à $C_4$, (alkoxy en $C_1$ à $C_4$)-(alkylamino en $C_1$ à $C_4$) ou di(alkyle en $C_1$ à $C_4$)-amino [chacun pouvant éventuellement être substitué par du fluor et/ou du chlore],

$R^7$ et/ou $R^8$ représentent en outre un groupe (alkyle en $C_1$ à $C_4$)sulfonyloxy, di(alkyle en $C_1$ à $C_4$)aminosulfonylamino ou le reste $-CH=N-R^{12}$, où

$R^{12}$ est un groupe alkyle en $C_1$ à $C_6$ éventuellement substitué par du fluor, du chlore, un radical cyano, carboxy, alkoxy en $C_1$ à $C_4$, alkylthio en $C_1$ à $C_4$, alkylsulfinyle en $C_1$ à $C_4$ ou alkylsulfonyle en $C_1$ à $C_4$, un groupe benzyle éventuellement substitué par du fluor ou du chlore, un groupe alcényle en $C_3$ à $C_6$ ou alcynyle en $C_3$ à $C_6$ éventuellement substitué par du fluor ou du chlore, un groupe phényle éventuellement substitué par du fluor, du chlore, du brome, un radical alkyle en $C_1$ à $C_4$, alkoxy en $C_1$ à $C_4$, trifluorométhyle, trifluorométhoxy ou trifluorométhylthio, un groupe alkoxy en $C_1$ à $C_6$, alcénoxy en $C_3$ à $C_6$, alcynoxy en $C_3$ à $C_6$ ou benzyloxy, éventuellement substitué par du fluor et/ou du chlore, un groupe amino, (alkyle en $C_1$ à $C_4$)amino, di(alkyle en $C_1$ à $C_4$)amino, phénylamino, (alkyle en $C_1$ à $C_4$)-carbonylamino, (alkoxy en $C_1$ à $C_4$)carbonylamino, alkylsulfonylamino en $C_1$ à $C_4$ ou un groupe phénylsulfonylamino éventuellement substitué par du fluor, du chlore, du brome ou un radical méthyle,

en outre

$R^1$ représente le reste

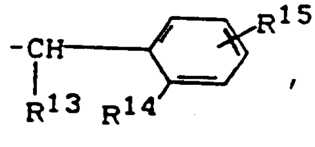,

dans lequel

$R^{13}$ est l'hydrogène ou un radical alkyle en $C_1$ à $C_4$,

$R^{14}$ et $R^{15}$ sont identiques ou différents et représentent l'hydrogène, le fluor, le chlore, le brome,

un groupe nitro, cyano, alkyle en $C_1$ à $C_4$ [qui est éventuellement substitué par du fluor et/ou par du chlore], un groupe alkoxy en $C_1$ à $C_4$ [qui est éventuellement substitué par du fluor et/ou par du chlore], un groupe carboxy, (alkoxy en Cl à $C_4$)-carbonyle, alkylsulfonyle en $C_1$ à $C_4$ ou di(alkyle en $C_1$ à $C_4$)aminosulfonyle ;

en outre

$R^1$ représente le reste

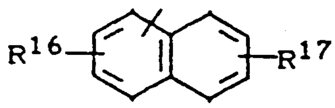

dans lequel

$R^{16}$ et $R^{17}$ sont identiques ou différents et représentent l'hydrogène, le fluor, le chlore, le brome, un groupe nitro, cyano, alkyle en $C_1$ à $C_4$ [qui est éventuellement substitué par du fluor et/ou du chlore] ou un groupe alkoxy en $C_1$ à $C_4$ [qui est éventuellement substitué par du fluor et/ou du chlore] ; en outre

$R^1$ représente le reste

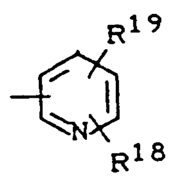

dans lequel

$R^{18}$ et $R^{19}$ sont identiques ou différents et représentent l'hydrogène, le fluor, le chlore, le brome, un groupe nitro, cyano, alkyle en $C_1$ à $C_4$ [qui est éventuellement substitué par du fluor et/ou du chlore], un groupe alcényle en $C_2$ à $C_4$ [qui est substitué le cas échéant par du fluor et/ou du chlore], un groupe alkoxy en $C_1$ à $C_4$ [qui est substitué le cas échéant par du fluor et/ou du chlore], un groupe alkylthio en $C_1$ à $C_4$, alkylsulfinyle en $C_1$ à $C_4$ ou alkylsulfonyle en $C_1$ à $C_4$ [qui sont substitués le cas échéant par du fluor et/ou du chlore] ainsi qu'un groupe di(alkyle en $C_1$ à $C_4$)aminosulfonyle, (alkoxy en $C_1$ à $C_4$)carbonyle, diméthylaminocarbonyle ou dioxolanyle ; en outre

$R^1$ représente le reste

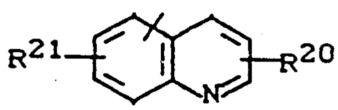

dans lequel

$R^{20}$ et $R^{21}$ sont identiques ou différents et représentent l'hydrogène, le fluor, le chlore, le brome, un groupe alkyle en $C_1$ à $C_4$ [qui est substitué le cas échéant par du fluor et/ou du brome], un groupe alkoxy en $C_1$ à $C_4$ [qui est substitué le cas échéant par du fluor et/ou du chlore], un groupe alkylthio en $C_1$ à $C_4$, alkylsulfinyle en $C_1$ à $C_4$ ou alkylsulfonyle en $C_1$ à $C_4$ [qui sont substitués le cas échéant par du fluor et/ou du chlore] ou un groupe di(alkyle en $C_1$ à $C_4$)aminosulfonyle ; en outre

$R^1$ représente le reste

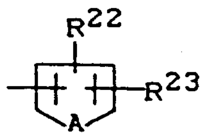

dans lequel

$R^{22}$ et $R^{23}$ sont identiques ou différents et représentent l'hydrogène, le fluor, le chlore, le brome, un groupe cyano, nitro, alkyle en $C_1$ à $C_4$ [qui est substitué le cas échéant par du fluor, du chlore, un radical alkoxy en $C_1$ à $C_4$ et/ou halogénalkoxy en $C_1$ à $C_4$], un groupe alkoxy en $C_1$ à $C_4$ [qui est substitué le cas échéant par du fluor et/ou du chlore], un groupe alkylthio en $C_1$ à $C_4$, alkylsulfinyle en $C_1$ à $C_4$ ou alkylsulfonyle en $C_1$ à $C_4$ [qui est substitué le cas échéant par du fluor et/ou du chlore], un groupe di-(alkyle en $C_1$ à $C_4$)aminosulfonyle ou (alkoxy en $C_1$ à $C_4$)carbonyle, et

A représente l'oxygène, le soufre ou le groupement $N-Z^1$, dans lequel

$Z^1$ est l'hydrogène, un groupe alkyle en $C_1$ à $C_4$ [qui est substitué le cas échéant par du fluor, du chlore, du brome ou un radical cyano], un groupe cycloalkyle en $C_3$ à $C_6$, benzyle, phényle [qui est éventuellement substitué par du fluor, du chlore, du brome ou un radical nitro], un groupe (alkyle en $C_1$ à $C_4$)carbonyle, (alkoxy en $C_1$ à $C_4$)-carbonyle ou di(alkyle en $C_1$ à $C_4$)aminocarbonyle ; en outre

$R^1$ représente le reste

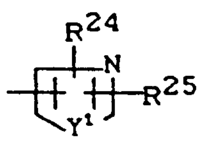

dans lequel

$R^{24}$ et $R^{25}$ sont identiques ou différents et représentent l'hydrogène, un groupe alkyle en $C_1$ à $C_4$, un halogène, un groupe (alkoxy en $C_1$ à $C_4$)carbonyle, alkoxy en $C_1$ à $C_4$ ou halogénalkoxy en $C_1$ à $C_4$,

$Y^1$ est le soufre ou représente le groupement $N-R^{26}$ dans lequel

$R^{26}$ est l'hydrogène ou un groupe alkyle en $C_1$ à $C_4$ ; en outre

$R^1$ représente le reste

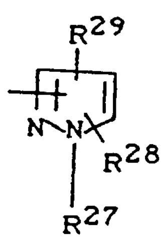

dans lequel

$R^{27}$ est l'hydrogène, un groupe alkyle en $C_1$ à $C_4$, benzyle, (iso)quinolinyle ou phényle,

$R^{28}$ est l'hydrogène, un halogène, un groupe cyano, nitro, alkyle en $C_1$ à $C_4$ [qui est substitué le cas échéant par du fluor et/ou du chlore], un groupe alkoxy en $C_1$ à $C_4$ - [qui est substitué le cas échéant par du fluor et/ou du chlore], un groupe dioxolanyle ou (alkoxy en $C_1$ à $C_4$)carbonyle et

$R^{29}$ représente l'hydrogène, un halogène ou un groupe alkyle en $C_1$ à $C_4$ ; en outre

$R^1$ représente le reste

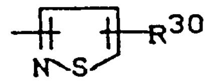

dans lequel

$R^{30}$ est l'hydrogène, un halogène, un groupe alkyle en $C_1$ à $C_4$, halogénalkyle en $C_1$ à $C_4$, alkoxy en $C_1$ à $C_4$, halogénalkoxy en $C_1$ à $C_4$ ou (alkoxy en $C_1$ à $C_4$)carbonyle ; en outre

$R^1$ représente le reste

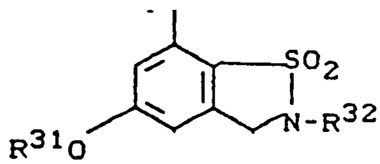

dans lequel

$R^{31}$ est un groupe alkyle en $C_1$ à $C_4$ et

$R^{32}$ est un groupe alkyle en $C_1$ à $C_4$ ; en outre

$R^1$ représente le reste

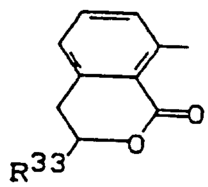

dans lequel

$R^{33}$ est l'hydrogène ou le groupe méthyle

où en outre

$R^2$ est l'hydrogène ou le groupement $-SO_2-R^1$ dans lequel

$R^1$ a la définition préférentielle indiquée ci-dessus ;

où en outre

$R^3$ est l'hydrogène, le fluor, le chlore, le brome, l'iode, un groupe hydroxy, mercapto, amino ou un reste éventuellement substitué par du fluor et/ou du chlore, de la série des restes alkyle en $C_1$ à $C_4$, cycloalkyle en $C_3$ à $C_6$, benzyle, phényle, alkoxy en $C_1$ à $C_4$, alcényloxy en $C_3$ ou $C_4$, alcynyloxy en $C_3$ ou $C_4$, alkylthio en $C_1$ à $C_4$, alkylsulfinyle en $C_1$ à $C_4$, alkylsulfonyle en $C_1$ à $C_4$, alcénylthio en $C_3$ ou $C_4$, alcynylthio en $C_3$ ou $C_4$, benzyloxy, benzylthio, (alkyle en $C_1$ à $C_4$)amino et di(alkyle en $C_1$ à $C_4$)amino,

$R^4$ est l'hydrogène, le fluor, le chlore, le brome, un groupe hydroxy, amino, alkyle en $C_1$ à $C_4$, halogénalkyle en $C_1$ à $C_4$, (alkoxy en $C_1$ ou $C_2$)-(alkyle en $C_1$ ou $C_2$), alkoxy en $C_1$ à $C_4$, halogénalkoxy en $C_1$ à $C_4$, (alkoxy en $C_1$ ou $C_2$)-(alkoxy en $C_1$ ou $C_2$), alkylthio en $C_1$ à $C_4$, halogénalkylthio en $C_1$ à $C_4$, alkylsulfinyle en $C_1$ à $C_4$, alkylsulfonyle en $C_1$ à $C_4$, alkylamino en $C_1$ à $C_4$ ou di(alkyle en $C_1$ ou $C_2$)amino,

X représente l'azote ou un groupement CH,

Y est l'azote ou un groupement $CR^5$ dans lequel,

$R^5$ est l'hydrogène, le fluor, le chlore, le brome, un groupe cyano, méthyle, formyle, acétyle, méthoxycarbonyle ou éthoxycarbonyle et

Z est l'azote ou un groupement $CR^6$, dans lequel

$R^6$ est l'hydrogène, le fluor, le chlore, le brome, un groupe alkyle en $C_1$ à $C_4$, alkoxy en $C_1$ à $C_4$, halogénalkoxy en $C_1$ à $C_4$, alkylthio en $C_1$ à $C_4$, alkylsulfinyle en $C_1$ à $C_4$, alkylsulfonyle en $C_1$ à $C_4$, alkylamino en $C_1$ à $C_4$, diméthylamino ou diéthylamino,

ainsi que de sels de composés de formule (I),

caractérisé en ce que

(a) on fait réagir des aminotriazolinones substituées de formule générale (II)

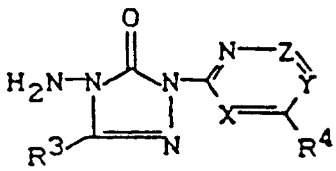

(II)

dans laquelle

$R^3$, $R^4$, X, Y et Z ont la définition indiquée dans la revendication 1,

avec des halogénures d'acides sulfoniques ou des anhydrides d'acides sulfoniques de formule générale (III)

$R^1 - SO_2 - Q$ (III)

où

$R^1$ a la définition indiquée dans la revendication 1 et

Q représente le fluor, le chlore, le brome ou le groupement $-O-SO_2-R^1$ dans lequel

$R^1$ a la définition indiquée ci-dessus,

le cas échéant en présence d'un accepteur d'acide et en la présence éventuelle d'un diluant,

on fait réagir éventuellement les composés ainsi obtenus de formule (I), dans laquelle

$R^2$ représente le groupement $-SO_2-R^1$,

avec des agents de désulfonylation, éventuellement en présence de diluants pour former des composés de formule (I), dans laquelle

$R^2$ représente l'hydrogène, ou bien

(b) on fait réagir des sulfonylaminotriazolinones de formule générale (IV)

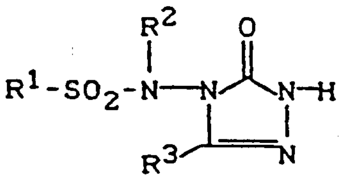

(IV)

dans laquelle

$R^1$, $R^2$ et $R^3$ ont la définition indiquée ci-dessus

avec des azines de formule générale (V)

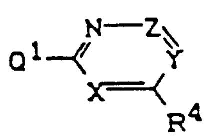

(V)

dans laquelle

$R^4$, X, Y et Z ont les définitions indiquées ci-dessus et

$Q^1$ est un halogène, un groupe benzylsulfonyle ou alkylsulfonyle,

le cas échéant en présence d'un accepteur d'acide et en la présence éventuelle d'un diluant et on produit éventuellement des sels par des opérations classiques à partir des composés de formule (I) ainsi obtenus.

**2.** Procédé de production de sulfonylaminotriazolinones substituées de formule générale (I) et de leurs sels suivant la revendication 1, caractérisé en ce que dans la formule (I)

$R^1$ représente le reste

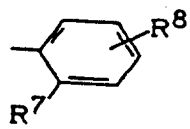

où

$R^7$ est le fluor, le chlore, le brome, un groupe méthyle, trifluorométhyle, méthoxy, difluorométhoxy, trifluorométhoxy, alkylthio en $C_1$ à $C_3$, alkylsulfynyle en $C_1$ à $C_3$, alkylsulfonyle en $C_1$ à $C_3$, diméthylaminosulfonyle, N-méthoxy-N-méthylaminosulfonyle, phényle, phénoxy ou (alkoxy en $C_1$ à $C_3$)carbonyle et

$R^8$ est l'hydrogène, le fluor ou le chlore ; dans laquelle en outre

$R^1$ représente le reste

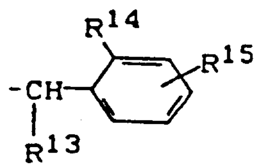

où

$R^{13}$ est l'hydrogène,

$R^{14}$ est le fluor, le chlore, le brome, un groupe méthyle, méthoxy, difluorométhoxy, trifluorométhoxy, éthoxy, méthoxycarbonyle, éthoxycarbonyle, méthylsulfonyle ou diméthylaminosulfonyle et

$R^{15}$ est l'hydrogène ; dans laquelle en outre

$R^1$ représente le reste

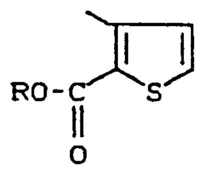 ,

où

R est un groupe alkyle en $C_1$ à $C_4$, ou bien

$R^1$ représente le reste

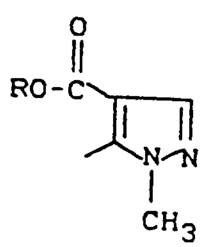

dans lequel

R est un radical alkyle en $C_1$ à $C_4$, ou bien

$R^1$ représente le reste

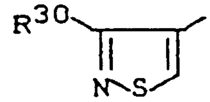

dans lequel

$R^{30}$ est l'hydrogène, le chlore, un groupe méthyle, éthyle, méthoxycarbonyle ou éthoxycarbonyle ;

dans laquelle en outre

$R^2$ est l'hydrogène ou le groupement $-SO_2-R^1$, où

$R^1$ a la définition particulièrement appréciée indiquée ci-dessus

$R^3$ est l'hydrogène, un groupe méthyle, éthyle, propyle, isopropyle, trifluorométhyle, cyclopropyle, benzyle, phényle, tertiobutyle, sec-butyle, isobutyle, n-butyle, méthoxy ou méthylthio,

$R^4$ est l,hydrogène, le fluor, le chlore, le brome, un groupe méthyle, trifluorométhyle, méthoxy, éthoxy, difluorométhoxy, méthylthio, éthylthio, amino, méthylamino, éthylamino, diméthylamino ou diéthylamino,

X représente l'azote ou un groupement CH,

Y représente l'azote ou un groupement $CR^5$, dans lequel

$R^5$ est l'hydrogène, le fluor, le chlore ou un groupe méthyle, et

Z est l'azote ou un groupement $CR^6$, dans lequel

$R^6$ est l'hydrogène, le fluor, le chlore, le brome, un groupe méthyle, éthyle, méthoxy, éthoxy, propoxy, isopropoxy, difluorométhoxy, méthylthio, éthylthio, méthylamino, éthylamino, diméthylamino ou diéthylamino.

**3.** Compositions herbicides, caractérisées par une teneur en au moins une sulfonylaminotriazolinone substituée de formule (I) suivant la revendication 1.

**4.** Procédé pour combattre des mauvaises herbes, caractérisé en ce qu'on fait agir des sulfonylaminotriazolinones substituées de formule (I) suivant la revendication 1 sur les mauvaises herbes et/ou sur leur milieu.

**5.** Utilisation de sulfonylaminotriazolinones substituées de formule (I) suivant la revendication 1 pour combattre des mauvaises herbes.

**6.** Procédé de préparation de compositions herbicides, caractérisé en ce qu'on mélange des sulfonylaminotriazolinones substituées de formule (I) suivant la revendication 1 avec des diluants et/ou des agents tensio-actifs.